(19) Europäisches Patentamt
European Patent Office
Office européen des brevets

(11) EP 4 138 941 B1

(12) EUROPEAN PATENT SPECIFICATION

(45) Date of publication and mention of the grant of the patent:
**29.07.2026 Bulletin 2026/31**

(21) Application number: **21720580.6**

(22) Date of filing: **20.04.2021**

(51) International Patent Classification (IPC):
***A61L 27/22** (2006.01)* ***A61L 27/18** (2006.01)*
***B33Y 70/00** (2020.01)* ***C08J 3/24** (2006.01)*
***C08G 67/00** (2006.01)* ***G03C 1/043** (2006.01)*

(52) Cooperative Patent Classification (CPC):
(C-Sets available)
**A61L 27/222; A61L 27/18; B33Y 70/00; C08J 3/075**
(Cont.)

(86) International application number:
**PCT/IB2021/053245**

(87) International publication number:
**WO 2021/214652 (28.10.2021 Gazette 2021/43)**

(54) **NEW COUMARIN-POLYMER CONJUGATES AND USES THEREOF**

NEUE CUMARIN-POLYMER-KONJUGATE UND VERWENDUNGEN DAVON

NOUVEAUX CONJUGUÉS DE COUMARINE-POLYMÈRE ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.04.2020 IT 202000008779**

(43) Date of publication of application:
**01.03.2023 Bulletin 2023/09**

(73) Proprietor: **Onyel Biotech S.r.l.**
**35129 Padova (IT)**

(72) Inventors:
- **ELVASSORE, Nicola**
**35129 Padova (IT)**
- **POLI, Ilaria**
**35129 Padova (IT)**
- **URCIUOLO, Anna**
**35129 Padova (IT)**

(74) Representative: **Predazzi, Valentina et al**
**Società Italiana Brevetti S.p.A.**
**Piazza di Pietra, 39**
**00186 Roma (IT)**

(56) References cited:
**WO-A1-2021/186335**

- **ASHAMMAKHI NUREDDIN ET AL: "In situthree-dimensional printing for reparative and regenerative therapy", BIOMED MICRODEVICES, KLUWER DORDRECHT, NL, vol. 21, no. 2, 6 April 2019 (2019-04-06), pages 1 - 6, XP036869425, ISSN: 1387-2176, [retrieved on 20190406], DOI: 10.1007/S10544-019-0372-2**
- **MALAR A. AZAGARSAMY ET AL: "Coumarin-Based Photodegradable Hydrogel: Design, Synthesis, Gelation, and Degradation Kinetics", ACS MACRO LETTERS, vol. 3, no. 6, 17 June 2014 (2014-06-17), pages 515 - 519, XP055681286, ISSN: 2161-1653, DOI: 10.1021/mz500230p**
- **CHRISTOPHER P. KABB ET AL: "Photoreversible Covalent Hydrogels for Soft-Matter Additive Manufacturing", ACS APPLIED MATERIALS & INTERFACES, vol. 10, no. 19, 4 May 2018 (2018-05-04), US, pages 16793 - 16801, XP055760788, ISSN: 1944-8244, DOI: 10.1021/acsami.8b02441**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 138 941 B1

(52) Cooperative Patent Classification (CPC): (Cont.)

C-Sets
**A61L 27/18, C08L 71/02**

## Description

The present invention provides new coumarin-polymer conjugates that, upon irradiation with IR light, undergo an intermolecular [2+2] cycloaddition reaction through coumarin units through the coumarin components of different units of said coumarin-polymer conjugate thereby forming a crosslinked product, compositions/mixtures comprising said conjugates, products comprising the crosslinked product, and uses thereof. The new coumarin-polymer conjugates of the invention are particularly useful for 3D bioprinting.

STATE OF THE ART

3D printing is an additive process in which successive layers of materials are structured to generate 3D shapes. Such technology is being applied to enable rapid prototyping and manufacturing in industry, as well as production of personalized consumer products. Advanced systems have recently implemented layer-by-layer precise positioning of biomaterials, biochemicals and living cells into complex 3D functional tissues moving from 3D printing to 3D bioprinting (L. Moroni et al., Biofabrication strategies for 3D in vitro models and regenerative medicine. Nat. Rev. Mater. 3, 21-37 (2018); S. V. Murphy, A. Atala, 3D bioprinting of tissues and organs. Nat. Biotechnol. 32, 773-785 (2014); C. S. Ong et al., 3D bioprinting using stem cells. Pediatr. Res., 1-23 (2017)).

3D bioprinting aims mainly at replicating the composition and the 3D architecture of biological tissues/organs. There are several 3D bioprinting approaches, which range from the manufacture of constructs that contain relevant cellular and extracellular components of a tissue or organ, to the fabrication of instructing scaffolds, in which stem cells can develop a tissue by their own autonomous organization and patterning. The guiding idea behind the development of these strategies is the realization of 3D functional living human constructs with biological and mechanical properties suitable for in vitro tissue modeling or the restoration of tissue/organ function to be then applied in clinic.

For in vitro application, 3D bioprinting allow the fabrication of constructs made by cells and biomaterial in a 3D architecture that can better mimic the histological organization of a specific tissue. Moreover, hydrogels can be also used as a non-organized 3D environment (bulk biomaterial) in which the cells can self-organize to generate complex systems that resemble tissues, including organoids or spheroids. Fabrication of constructs requires an open access to the bioprinting material and to the bioprinting set-up for allowing free XYZ spatial movements and fabrication. Therefore, the possibility to generate an organized architecture within pre-existing 3D solid environment (bulk biomaterial), without altering the overall integrity of such environment, is strongly limited by the state of the art of the bioprinting technologies. The possibility to perform 3D printing inside 3D bulk biomaterials, will open novel opportunity to generate defined 3D objects that can allow the control of cell cultured into conventional 3D environment.

As for their application in clinic, 3D functional living constructs are either incubated in vitro for maturation before implantation, or 3D bioprinted and implanted in vivo (N. Hong, G.-H. Yang, J. Lee, G. Kim, 3D bioprinting and its in vivo applications. J. Biomed. Mater. Res. Part B Appl. Biomater., 444-459 (2017); M. Wang et al., The trend towards in vivo bioprinting. Int. J. Bioprinting (2015), doi:10.18063/IJB.2015.01.001). However, in both cases, open surgery and invasive procedures for exposing the areas, where the printed object must be positioned, are required. Furthermore, in only few examples, the 3D bioprinted constructs are directly fabricated in situ in live animals, and such strategies have been limited to external accessible loci such as skin (A. Skardal et al., Bioprinted Amniotic Fluid-Derived Stem Cells Accelerate Healing of Large Skin Wounds. Stem Cells Transl. Med. 1, 792-802 (2012); K. W. Binder et al., In situ bioprinting of the skin for burns. J. Am. Coll. Surg. 211, S76 (2010)) or sites that have been exposed by invasive surgery procedure, such as bone (V. Keriquel et al., In situ printing of mesenchymal stromal cells, by laser-assisted bioprinting, for in vivo bone regeneration applications. Sci. Rep. 7, 1-10 (2017)) and cartilage (C. Di Bella et al., In situ handheld three-dimensional bioprinting for cartilage regeneration. J. Tissue Eng. Regen. Med., 611-621 (2017)). For in vivo bioprinting in regenerative medicine,also see ASHAMMAKHI et al, BIOMED MICRODEV. v.21(2) p.1-6 (2019). Thus, so far. the *in situ* 3D bioprinting for in vivo application requires an invasive open surgery to allow direct access of bioprinter head onto the tissues into the organism. In general, the conventional bioprinting approaches require direct access to printing sites and free XYZ spatial movements of the bioprinting head, precluding the internal modifications of pre-existing 3D structures, without affecting the integrity of the structures of interest.

On the other hand, injectable materials used as delivery vehicles hold great promise to improve the therapeutic efficacy for functional recovery of diseased or injured tissues and organs, including stem cell-based therapies (Wang, X et al. Advanced Functional Biomaterials for Stem Cell Delivery in Regenerative Engineering and Medicine. Advanced Functional Materials 29, 1-31 (2019)). An injectable hydrogel is the preferred material form for stem cell transplantation due to its potential to mimic the native stem cell microenvironment (Zhang, Z., et al. 3D bioprinting of soft materials-based regenerative vascular structures and tissues. Compos. Part B Eng. 123, 279-291 (2017); Chin, S. Y. et al. Additive manufacturing of hydrogel-based materials for next-generation implantable medical devices. Sci. Robot. 2, eaah6451 (2017); Murphy, et al. Evaluation of hydrogels for bio-printing applications. J. Biomed. Mater. Res. - Part A (2013)). A variety of crosslinking strategies have been developed to form hydrogel structures in internal regions of tissues: *i)* physical

crosslinking, which include ionic crosslinking and stimuli-responsive crosslinking (such as temperature and pH change); *ii)* chemical crosslinking, which includes enzymatic, radical or click chemistry, and provides better control of the hydrogel mechanical strength and degradation, compared to physical crosslinking processes (Wang, X et al. Advanced Functional Biomaterials for Stem Cell Delivery in Regenerative Engineering and Medicine. Advanced Functional Materials 29, 1-31 (2019)). However, independently from the crosslinking strategy used, injectable hydrogels form an isotropic structure around the site of the injection which cannot be controlled in terms of 3D shape and spatial organization.

Coumarin has been used to develop coumarin-based photodegradable hydrogel, under aqueous conditions using copper-catalyzed click chemistry (Malar A. Azagarsamy et al. Coumarin-based Photodegradable Hydrogel: Design, Synthesis, Gelation, and Degradation Kinetics). However, both the gelation and degradation process therein disclosed, involve the light exposure at a wavelength of 365 nm, that is a wavelength which can not pass across tissues/organs in living organism, and can not avoid surgery.

The possibility to directly fabricate in situ 3D constructs across tissues/organs in living organisms avoiding surgery would substantially change the paradigm of 3D bioprinting for clinical use. To date, this concept, defined in the preset description as intravital 3D bioprinting (i3D bioprinting), has never been reported. Compared to conventional 3D bioprinting, i3D bioprinting involves additional complexities related to the *in vivo* application. These include *i)* fabrication across tissues without inducing tissue damage or interfering with the pre-existing 3D tissue anatomical structures; *ii)* fabrication efficiency should not be affected by physiological tissue displacements given by heart beating as well as breathing of the live animals; *iii)* accurate (XYZ) positioning and orientation of 3D bioprinted structures within tissues at site of interest to support cell function and/or tissue regeneration. Ideally, 3D bioprinting in a live animal would also require accurate pre-processing imaging for fine 3D mapping the anatomical area in which the printed object needs to be manufactured, and post-processing analysis to effectively evaluate the positioning, the orientation and the accuracy of the printed object.

SUMMARY OF THE INVENTION

The present description discloses a strategy to overcome the complexities of i3D illustrated above, by developing photosensitive biopolymers that can be used to fabricate hydrogels within and across solid matrices or tissues. These biopolymers can be crosslinked in vitro in presence of living cells or inside a pre-mixed gels for controlling cell, cell cluster or organoid shape and behavior in a 3D culture. Moreover, after a simple injection inside the biological site, photosensitive biopolymers can be photo-crosslinked in vivo, into a 3D object by using near-infrared (IR) laser light, also associated to intravital multiphoton microscopy. This was accomplished by modifying the terminal ends of biocompatible hydrophilic polymeric backbones with a hydrophobic photo-active crosslinking group, which efficiently undergoes [2+2] cycloaddition reaction when excited by single-photon absorption of UV light (300 nm < $\lambda$ < 400) or two-photon absorption of IR light (700 nm < $\lambda$ < 950 nm). In virtue of the selected wavelength range, femtosecond IR tightly-focused pulsed laser light can penetrate through soft tissues (K. König, Multiphoton microscopy in life sciences. J. Microsc. 200, 83-104 (2000)), allowing a 3D product photo-crosslinking across tissues and deep within tissues. The present description discloses that the high-resolution of intravital multiphoton microscopy allows 3D object fabrication *in vitro,* inside preexisting gel, and *in vivo,* inside tissues of living animal models, without tissue damage, and with accurate control of object architecture, position and orientation. This approach allows highly efficient and fast (single line writing speed up to 0.8 mm/ms) 3D bioprinting of hydrogel construct of millimeter scale volume, with micrometric resolution and tunable physiological stiffness.

The authors of the present invention, also demonstrate with the data provided in the present description, that complex 3D constructs of polyethylene glycol (PEG) and gelatin (Gel) hydrogels, obtained with the coumarin-polymer complexes of the invention, are accurately fabricated into organs of living mice such as skin across epidermis, skeletal muscle across epimysium or brain across meninges. The 3D hydrogels show high biocompatibility and incorporation of functional vascular network, which makes the i3D bioprinting a minimally invasive procedure, suitable for clinical translation.

The authors of the present invention, found that the coumarin component of specific coumarin-polymer conjugates, as described in detail in the present specification and defined in the claims, undergoes cycloaddition by 2P (two-photons) absorption at a wavelength that doesn't correspond to the maximum single-photon absorption at a wavelength (Abs $\lambda$, nm) of the coumarin compound. Specific coumarin compounds were selected by the inventors as photo-sensitive compounds to be applied for i3D bioprinting. Such molecules have proved to undergo [2+2] cycloaddition (with no byproduct release) when exposed to UV light (300 nm < $\lambda$ < 400) or IR light (700 nm < $\lambda$ < 950 nm) at laser wavelengths that allow deep tissue penetration.

An object of the present invention is, therefore,

1. a coumarin-polymer conjugate comprising a coumarin compound and a polymer, characterized in the coumarin compound has a maximum single-photon absorption at wavelength, Abs $\lambda$, from 310 to 450 nm, at least one electron-donor in positions 6 and/or 7 and/or at least an electron-acceptor in positions 3 and/or 4 wherein said coumarin

compound is selected from
7-Carboxymethoxy-4-methylcoumarin
7-Hydroxy-4-methylcoumarin
7-Amino-4-methyl-3-coumarinylacetic acid
7-Hydroxy-4-(trifluoromethyl)coumarin
7-Hydroxycoumarin-3-carboxylic acid
Ethyl 7-Hydroxycoumarin-3-carboxylate
7-Hydroxycoumarin-3-carboxylic acid N-succinimidyl ester.

A further object of the present invention is a mixture of coumarin-polymer conjugates as defined above and in the present description and claims.

Another object of the present invention is a product comprising one or more coumarin-polymer conjugates according to the present description and claims whereby the coumarin components of different units of said coumarin-polymer conjugates are cross-linked.

Another object of the invention is the use of coumarin-polymer conjugates as defined above and in the present description and claims for the generation of cross-linked hydrogels for in vitro cell culture, including cell seeding onto fabricated hydrogels, cell-laden bioprinting and bioprinting into pre-existing 3D materials (including solid hydrogels).

A further object of the invention is a medical device or a scaffold comprising the coumarin-polymer conjugate as defined in the description and claims or the product as defined in the description and claims.

Another object of the invention is a method for the preparation of the coumarin-polymer conjugate as defined in the description and claims as well as a method for the preparation of the mixture as defined above and in the claims.

Yet another object of the invention is a method for the preparation of the coumarin-polymer conjugate crosslinked product as defined in the description and claims.

A further object of the invention is the use of the coumarin-polymer conjugate as defined above and in the present description and claims as drug or cell delivery systems (including for *de novo* tissue formation and regenerative strategies).

Another object of the invention is the use of coumarin-polymer conjugates as defined in the description and in the claims for the generation of cross-linked hydrogels for in vitro cell culture, cell-laden bioprinting and bioprinting into pre-existing 3D materials as well as the use of coumarin-polymer conjugates as defined in the description and in the claims as drug or cell delivery systems.

Still a further embodiment of the invention is said conjugate, said mixture, said product, or of said scaffold, or of said medical device for use in medical treatments, as well as medical treatments wherein said conjugate, said mixture, said product, or of said scaffold, or of said medical device shaped into a patient in need thereof by i3D by IR light-mediated cycloaddition.

GLOSSARY

Three dimensional (3D) bioprinting is the utilization of 3D printing-like techniques to combine cells, growth factors, and biomaterials to fabricate biologically functional 3D constructs that maximally reproduce natural tissue characteristics. Generally, 3D bioprinting utilizes the layer-by-layer method to deposit materials known as bioinks to create tissue-like structures that are later used in medical and tissue engineering fields. Bioprinting covers a broad range of biomaterials.

Coumarins, according to the art and to the description, are organic heterocycles characterized as 1,2 benzopyrones (coumarin: 1,2-Benzopyrone, 1-Benzopyran-2-one, 2H-Chromen-2-one) that comprise a group of natural products found in a wide range of plants. They represent an important type of naturally occurring and synthetic oxygen-containing heterocycles with typical benzopyrone framework.

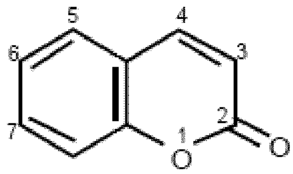

Coumarin

Several coumarin derivatives (i.e. compounds comprising the coumarin framework above and different residues in positions 1-8).

Coumarin compound according to the present invention is a compound comprising a coumarin framework and different residues in positions 1-8. Coumarin compounds are defined in the art also as coumarin derivatives.

A coumarin-polymer conjugate according to the present description is a compound wherein a coumarin or a coumarin compound is chemically bonded, by direct bond or through a chemical linker, to one polymer molecule.

Chemical linkers or linkers according to the art and to the present description are molecules providing a functional handle for the chemical bonding between molecules that would not be capable of linking one to another in a certain position, linkers are particularly useful for linking biomolecules to other molecules. Linkers can act by introducing new functional groups and/or, acting as spacer thereby improving

the spatial availability of functionalities of the molecules, or to modify the properties of a molecule, such as solubility and stability.

There are several linkers known in the art and available to the public, they can be also defined as bifunctional linkers, trifunctional linkers or crosslinkers.

Crosslinking reagents generally contain two or more reactive groups that are often 'activated' to react with certain functional groups such as amines and sulfhydryls, but also may be less specific in the case of photoreactive crosslinkers. The usefulness of crosslinking chemistry is realized in applications such as:

protein structure and/or function determination
protein or other biomolecule immobilization
general biomolecule-biomolecule conjugation
antibody-drug conjugates.

Crosslinking reagents (or crosslinkers) are molecules that contain two or more reactive ends capable or chemically attaching to specific functional groups (primary amines, sulfhydryls, etc.) on proteins or other molecules.

Therefore, according to the art, conventional linkers (crosslinkers, bifunctional or trifunctional) can contain diverse functional groups, such as primary amines, sulfhydryls, acids, alcohols and bromides.

Chemical linkers as defined above and in the art are commonly available to the skilled person.

The term electron-acceptor according to the present description has the meaning commonly used in the art, therefore an electron acceptor is a chemical entity that accepts electrons transferred to it from another compound. An electron-acceptor group is a chemical group that accepts electrons transferred to it from another compound (i.e. an oxidizing group).

The term electron-donor according to the present description has the meaning commonly used in the art, therefore an electron donor s a chemical entity that donates electrons to another compound. An electron-donor group is a chemical group that donates electrons to another compound (i.e. a reducing group).

Natural polymer according to the present description is a polymer existing in nature, therefore obtainable by extraction from natural compounds such as polypeptides, proteins and polysaccharides. Typically, natural polymers are biocompatible and enzymatically biodegradable, meaning that the polymer is readily recognized, accepted, and mechanically processed by the human body.

Synthetic polymer according to the present description is a polymer produced by chemical synthesis. Synthetic polymers are generally biologically inert, and they can be produced under controlled conditions. They have reproducible and tailored physical,

mechanical and chemical properties according to the requirements of specific applications. They can be biodegradable or nondegradable biomaterials.

A biomedical polymer according to the description is a polymer (natural or synthetic) that show biocompatibility and can be adapted for biomedical application to meet medical needs such as tissue-engineered constructs, implants, and cell- or drug-delivery.

A biocompatible polymer according to the description is a polymer (natural or synthetic) that by its physical, mechanical, and chemical properties, do not show potential cytotoxic, mutagenic, and allergenic effects, therefore not inducing significant injuries or toxic effects on the biological function of cells and individuals.

A hydrogel according to the description is a gel comprising of one or more polymers suspended in water or in an aqueous solution.

An organoid according to the description has the meaning commonly used in the art; an organoid is hence intended as a miniaturized and simplified version of an organ produced in vitro in three dimensions that shows realistic micro-anatomy. Organoids can be derived from one or a few cells from a tissue, embryonic stem cells or induced pluripotent stem cells, which can self-organize in three-dimensional culture owing to their self-renewal and differentiation capacities.

DETAILED DESCRIPTION OF THE FIGURES

Figure 1. Single-photon absorption spectra analysis of coumarin derivatives. The absorption spectra of 8 coumarin derivatives were investigated in PBS at pH 7.4 to reproduce the physiological conditions. 7-hydroxycoumarin-3-carboxylic acid (HCCA) has shown the higher absorption wavelength compared to the other derivatives, except for coumarins (dashed-lines spectra) bearing a carboxylic-derivate group able to promote a further red-shift ($\lambda$ > 400 nm). 7-Hydroxy-4-methylcoumarin (CMMC) was specifically selected to perform 1-photon UV mediated cycloaddition. The HCCA was converted to the 7-Hydroxycoumarin-3-carboxylic acid N-succinimidyl ester (HCCA-NSE) and the CMMC was converted to 7-(carboxymethoxy)-4-methylcoumarin N-succinimidyl ester (CMMC-NHS) for an easy conjugation with polymeric backbones through an amide group.

Figure 2. The ability of HCCA to undergo cycloaddition was confirmed by 1H-NMR spectroscopy and electrospray ionization mass spectrometry analysis of HCCA solutions.

Figure 3. Characterization of HCC- and CMMC-conjugates. HCC-PEG, HCC-4arm PEG, HCC-8arm PEG, CMMC-4arm PEG were characterized by using 1H NMR (A,D) and Fourier transform infrared (FT-IR) analysis (B). HCC-Gel and CMMC-Gel were characterized by size-exclusion chromatography (SEC; C,E).

Figure 4. Single photon absorption spectra analysis of HCC-polymers at pH 7.4 in PBS. Representative UV-Vis absorption spectra of HCC-PEG, HCC-4arm PEG, HCC-8arm PEG and HCC-Gel in the wavelength range of 250-450 nm.

Figure 5. (A) 8 different coumarins were selected for further characterization based on ring substituents that can impact their light-absorption properties and the chemistry for backbone conjugation. (B) the presence of a carboxylic group allows the conjugation of HCCA to polymers, such as linear PEG, branched PEG, and gelatin (Gel). (C) Single-photon absorption ($\lambda$ = 365 nm) was able to induce HCC-PEG intermolecular cycloaddition in solution, resulting in dimerization of HCC-PEG as showed by spectrophotometric analysis. (D) HCC underwent cycloaddition after 2P absorption at a wavelength of 850 nm using a multi-photon laser-scanning microscope, as showed by the rapid 2D HCC-PEG patterning (~10 sec per 850 $\mu m^2$) onto HCCA-functionalized glass slides with high spatial precision. (E) Multiple 3D HCC-Gel hydrogels can be fabricated according to the design structure, as showed by multiple 3D parallelepipeds rotated by 45° around mass centre and placed on the same Z plane.

Figure 6. Single 3D object fabrication at different wavelength and fixed laser power (0.7 mW). Representative phase contrast (higher panels) and autofluorescence (lower panels) images of HCC-4arm PEG hydrogel structures fabricated by near-IR excitation ($700<\lambda<850$nm) showing a cube-shaped Z-stack volume. Scale bar 200 $\mu$m.

Figure 7. Multiple 3D objects fabrication by 2P microscopy. (A) Representative phase contrast (PC) and fluorescent (Autofluorescence) images of three cubic-shaped HCC-4arm PEG structures with comparable dimension and fabricated at the same distance from each other. Scale bar 200 $\mu$m. (B) Representative phase contrast (PC) and fluorescent (Autofluorescence) images of parallelepiped-shaped HCC-8arm PEG structures with different dimensions and fabricated at defined XY position. Scale bar 50 $\mu$m. (C) Illustration and fluorescent images (Autofluorescence) of a readable 3D barcode-shaped "LIFE"-shaped HCC-4arm PEG 3D structures. Scale bar 100 $\mu$m. (D) Illustration and fluorescent images (Autofluorescence) of a "# LIFE"-shaped HCC-Gel 3D structures. Scale bar 100 $\mu$m. (E) Volume-3D reconstruction of a empty cube fabricated by sequential addition of multiple objects made of HCC-8arm PEG. Scale bars are reported in the red grid.

Figure 8. (A) Multiple hydrogels with different Z positions can be photo-crosslinked. B,C) The minimal linewidth (1.9 $\pm$0.2 $\mu$m) of fabricated linear objects and the sub-micrometric resolution of the bioprinting were achieved. (D) Using a computer drawing pad combined to a free line scan program of the multi-photon microscope, it was possible to fabricate linear object of the desired shape, a 3D single line flower-shaped hydrogel of HCC-Gel. (E) The maximum 3D object dimension that was possible to fabricate without changing printing parameters (scanning speed and laser power) was ~2 mm. (F) Minimal writing time per single line that allows 3D hydrogel production with structural integrity was defined as 1 ms/line. (G) The inventors were able to finely tune the stiffness of HCC-4arm PEG or HCC-Gel hydrogels over biological-matched ranges (1 kPa - 20 kPa) by modulating the laser power. (H) CMMC-4arm PEG and CMMC-Gel were crosslinked by using 1-photon UV mediated cycloaddition. The elastic modulus increases to asymptotic values by increasing the irradiation time, showing a sigmoidal temporal profile of elastic modulus that reached the polymer crosslinking saturation, independently from the polymeric backbone used. (I) The modulation of elastic modulus of hydrogels obtained with 1P and 2P photo-crosslinking (with CMMC-Gel and HCC-gel, respectively) by varying the polymer concentration showed remarkable similarity.

Figure 9. Transmission electron microscopy analysis of HCC-PEG conjugates. Nanoaggregates are only revealed in HCC-PEG samples and are absent in PEG samples; scale bar 500 nm. Insets show higher magnification; scale bar 100 nm.

Figure 10. A) HCC-Gel hydrogels supported adhesion and culture of HUVEC cells and B) human embryonic stem cell-derived neural stem cells (hES-derived NSCs). Three days after seeding, hES-derived NSCs showed expression of specific neural markers and absence of apoptosis when cultured on pre-assembled flat HCC-Gel hydrogels.

Figure 11. Fibroblasts (FBs) derived from green fluorescent protein (GFP)-expressing mice efficiently adhere to

parallelepiped-shaped HCC-Gel hydrogels characterized by different size (ΔZ up to 150 μm) and interspace (down to 10 μm) and change their morphology according to the 3D shape of fabricated hydrogels

Figure 12. (A) Three days after seeding, hES-derived NSCs showed expression of specific neural markers and absence of apoptosis when cultured on pre-assembled flat HCC-Gel hydrogels. (B) Biocompatibility of HCC-Gel solution and of the photo-crosslinking strategy was confirmed by using cell-laden HCC-Gel solution. Six hours after hydrogel crosslinking, FBs were viable and embedded within 3D hydrogel structures. (C) The elastic modulus of bioprinted structures was not strongly changed by the presence of cells (Young's modulus was reduced by about 6% in presence of cells, suggesting that the 2P-crosslinking and hydrogel fabrication were not substantially affected by cells suspended in the HCC-Gel solution. (D) Cell viability evaluated comparing cell-laden constructs obtained with conventional single photon hydrogel crosslinking (λ = 365 nm, single photon, CMMC-Gel), those fabricated with 2P bioprinting (λ = 850 nm, 2-photon, HCC-Gel), compared to irradiated-only (λ = 365 nm, Matrigel) or unirradiated (Matrigel) controls. Two days after 3D culture, cell viability was between 90-99% in all conditions. (E,F) Murine muscle-derived stem cells (MuSC) were used to evaluate instructive cues of hydrogel with elongated-shape or cuboid-shape. Quantification of cell directionality showed that single-nucleated or polynucleated cells within elongated-shaped HCC-Gel structures appeared oriented along major axis of the 3D structures, whereas cells located in cuboid-shaped HCC-Gel structures were randomly oriented. (G) Microscopic analysis demonstrated that parallelepiped-shaped HCC-Gel hydrogels support the formation of aligned multinucleated myotubes that showed spontaneous contraction.

Figure 13. (A) HCC-Gel 2P photo-crosslinking showed a bio-orthogonal reaction in pre-existing gel. (B) When fabricated into pre-existing 3D Matrigel, hydrogel structures remained in shape also after Matrigel dissociation and showed comparable elastic modulus of hydrogel fabricated in absence of Matrigel. (C) Hydrogels were fabricated as 3D parallelepipeds accurately positioned and oriented relative to selected hSIOs within the 3D gel culture. (D) HCC-Gel hydrogel cytocompatibility on hSIOs enclosed in the structures was demonstrated by live/dead assay performed after 2 days and 8 days of culture. (E) hSIOs confined inside an open square-box (formed by 4 orthogonal walls) grew normally and after few days of culture touched and deformed HCC-Gel hydrogel walls. (F) Between day 6 and day 8 of culture, hSIOs enclosed in HCC-Gel-based hydrogel showed marked morphology changes, which lead to well-developed columnar epithelium. (G-I) This morphology was characterized by thickening of the organoid wall, F-actin localization in apical position and nuclei constriction in the basal domain as quantified by the basal-apical distance ratio and the basal/apical axis. (J) HCC-8arm PEG/Matrigel allowed hSIO survival and growth in culture. (K,L) Differently from what observed with HCC-Gel, hSIOs enclosed in HCC-8arm PEG-based hydrogel did not show columnar epithelium after 8 days of culture, hydrogel deformation, actin apical localization nor nuclei constriction. Instead, hSIOs morphology reflects the 3D shape of the enclosed open-box made by HCC-8arm PEG-based hydrogel. (M) Feret's angle quantification demonstrated that only hSIOs enclosed by HCC-8arm PEG acquired a cuboidal cystic structure 6 days after culture, when compared to HCC-Gel enclosed hSIOs.

Figure 14. (A) HCC-polymer solution was exposed to focalized pulsed near-IR laser light (λ = 850 nm) using a 2P microscope. (B) The inventors succeeded in efficiently fabricating isolated 3D objects inside the dermis of living mice. In $GFP^+$ mice, by taking advantage of fluorescence imaging of host tissues, both the manufactured objects located inside dermis, and the integrity of the epidermis above the crosslinked hydrogel were easy to visualize. (C) The high compatibility of i3D bioprinting was further confirmed by the integrity of the skin after the entire procedure, which was comparable to untreated tissue. (D,E) Photo-sensitive solutions injected under the epimysium of skeletal muscle were photo-crosslinked to fabricate 3D hydrogels at the surface of muscle fibers without evident alteration of the overall muscle fiber morphology and connective tissue integrity. (F) 2P-crosslinked hydrogels were formed only in the area where the reaction was performed. (G) Safe i3D bioprinting was also possible in a particularly vulnerable tissue such as brain. Burr hole was performed into the skull of mice to allow HCC-polymeric solution injection across meninges, and subsequent hydrogel crosslinking was achieved by using 2P absorption through multi-photon microscope. Importantly, absence of bleeding foci and a correct maintenance of blood flow at the area of i3D bioprinting in the brain were found. (H) 2P crosslinked hydrogels surrounded and embedded the host vasculature network without damaging the vessels and preserved blood flow, as shown by red-dextran tracing.

Figure 15. i3D bioprinting into skin. (A) Representative images showing HCC-4arm PEG structures fabricated across epidermis in different anatomic positions of wild-type animals. Scale bar 200 μm. (B) Representative images showing HCC-8arm PEG structures fabricated across epidermis with the minimum line scan given by the instrumental setup. The inset shows higher magnification of the object. Scale bar 10 μm.

Figure 16. i3D bioprinting of multiple objects and hydrogel positioning into muscle. (A) Representative images showing multiple fabrication of HCC-4arm PEG structures across epimysium in GFP+ animals. Scale bar 50 μm. (B) Representative images showing multiple HCC-Gel structures fabricated across epimysium in wild-type animals. The three objects were placed at 20 or 70 μm from the first one. Scale bar 100 μm. (C) Representative images showing multiple HCC-Gel structures fabricated at 45° from each other across epimysium in wild-type animals. Scale bar 100 μm. (D) Representative images showing multiple HCC-8arm PEG structures fabricated across epimysium in wild-type animals. Hydrogels were fabricated in a parallel or perpendicular fashion, according with myofibers orientation,

and at 90° from each other. Scale bar 100 μm.

Figure 17. Free line scan i3D bioprinting into muscle. Representative 3D reconstruction of a HCC-Gel single line infinite shaped-hydrogel fabricated by using free line scan program. Scale bars are included in the red grid that shows 3D orientation.

Figure 18. Embedding of vasculature into the hydrogel and persistence of blood flow after i3D bioprinting. (A) Representative intravital imaging displaying HCC-8arm PEG structure (green, ΔZ = 200 μm) fabricated into brain meninges and the integrity of brain blood flow (red). Scale bar 50 μm. (B) Representative confocal intravital imaging showing blood flow persistence inside the photo-polymerized hydrogel. Scale bars 50 μm.

Figure 19. Identification of the optimal i3D bioprinting conditions after HCC-Gel injection under epimysium. (A) Representative stereomicroscope imaging showing a single stack irradiated area at 3mW laser power or HCC-Gel structures (green, ΔZ = 200 μm) fabricated across epimysium at different laser power (3, 2, and 1 mW). Compact hydrogels were observed only when 1 mW and 2 mW laser power were used. Scale bar 500 μm. (B) Representative confocal intravital imaging of irradiated region of interest (ROI) located parallel to myofibers. Left panel, intravital imaging of a single Z-plane of the ROI at increasing time (from 0 to 20 sec) of irradiation (3 mW). Portion of a single myofiber (green, longitudinal view) and non-muscular tissue (black) were clearly visible to 1 sec of irradiation. Myofiber contraction (shortening) was observed at 5 sec from irradiation. Heat-induced tissue damage was evident at 10 sec from irradiation. Right panel, intravital imaging during i3D bioprinting (ΔZ-plane) at the writing time required for each single plane of irradiation (from 0 to 0.3 sec) at different laser power (3, 2, and 1 mW). No myofiber contraction nor heat-induced tissue damage were evident when 1 mW and 2 mW laser power were used. Scale bar 250 μm. (C) Immunofluorescent analysis of skeletal muscle cross-section isolated 4 hours after treatment. Representative immunofluorescence images showing cleaved form of caspase-8 (caspase-8 p18, red), laminin (green), collagen I (magenta) and hydrogel (blue, autofluorescence). Hydrogels were located between the connective tissue (collagen I positive) and the myofibers (each myofiber surrounded by laminin). Accumulation of caspase-8 p18 was evident in tissues subject to single stack irradiation (3mW laser power). Scale bar 100 μm.

Figure 20. Cytocompatibility of HCC-PEG i3D printing. (A) Stereomicroscope image showing distinct structures of HCC-PEG in mice 4 days after injection under epimysium at the site of 2P cross-linking and according with the i3D design. Scale bar, 1mm. (B) Intravital 2-Photon image showing HCC-PEG hydrogel 4 days after 2-Photon intravital fabrication. Scale bar 100 μm. (C) Immunofluorescence analysis for macrophages (F4/80, red), PEG (green) and actin (Phalloidin, magenta). Nuclei were stained with Hoechst (blue) in skeletal muscle cross sections from wild-type mice 4 days after under epimysium injection of HCC-PEG at the site of 2P cross-linking or untreated animals (CTRL). The inset shows a big view of merge imaging showing presence of F4/80 positive cells (red) surrounded by PEG (green). Nuclei were stained with Hoechst (Blue). Scale bars 100 μm. (D) Masson Trichrome staining of skeletal muscle cross sections from wild-type mice 4 days after under epimysium injection of HCC-PEG at the site of 2P cross-linking. Scale bars 100 μm.

Figure 21. Cytocompatibility of HCC-Gel solution. (A) Haematoxylin-eosin staining of skin and underneath skeletal muscle cross-sections from wild-type mice 4, 7 and 21 days after subcutaneous injection of HCC-Gel or PBS (CTRL). The inset shows magnification of the area of interest. Scale bars 100 μm. (B) Masson Trichrome staining of skin and underneath skeletal muscle cross-sections from wild-type mice 4, 7 and 21 days after subcutaneous injection of HCC-Gel or PBS (CTRL). The inset shows magnification of the area of interest. Scale bars 100 μm. (C) TUNEL staining of skin and underneath skeletal muscle cross-sections from wild-type mice 4, 7 and 21 days after subcutaneous injection of HCC-Gel or PBS (CTRL). The inset shows magnification of the area of interest. Scale bars 50 μm. (D) Immunofluorescence analysis for macrophages (F4/80, green) and actin (Phalloidin, magenta). Nuclei were stained with Hoechst (blue). Scale bars 100 μm.

Figure 22. i3D bioprinting with mCherry Fbs. (A) Intravital 2-Photon image showing the margins of two distinct freshly fabricated HCC-Gel mCherry$^+$FBs laden constructs with cuboid or parallelepiped shapes. Scale bar 100 μm. (B) Fluorescence stereomicroscope image showing mCherry$^+$FBs 21 days after their injection under the epimysium (no i3D, left panel) and after fabrication of parallelepiped-shaped HCC-Gel mCherry$^+$FBs laden construct (i3D, right panel) into wild-type animals. Scale bars 1 mm. (C) Intravital 2-Photon image showing mCherry+FBs organization 21 days after their injection under the epimysium coupled with fabrication of parallelepiped-shaped HCC-Gel mCherry+FBs laden construct (i3D) into wild-type animals. Scale bar 100 μm. (D) 3D reconstruction of 2-Photon intravital imaging showing mCherry$^+$FBs 21 days after their injection under the epimysium and fabrication of parallelepiped-shaped HCC-Gel mCherry$^+$FBs laden construct (i3D) into wild-type animals. (E) Intravital imaging showing morphology of mCherry$^+$FBs 21 days after their injection under the epimysium and fabrication of parallelepiped-shaped HCC-Gel mCherry$^+$FBs laden construct (i3D) into wild-type animals. Scale bars 50 μm. (F) Intravital 2-Photon image showing mCherry$^+$FBs organization 21 days after their injection under the epimysium (no i3D). Scale bar 100 μm. (G) 3D reconstruction of 2-Photon intravital imaging showing mCherry$^+$FBs 21 days after their injection under the epimysium (No i3D) into wild-type animals. (H) Intravital imaging showing morphology of mCherry$^+$FBs 21 days after their injection under the epimysium (No i3D) into wild-type animals. Scale bars 50 μm. (I) Fluorescence stereo-

microscope image showing mCherry$^+$FBs 14 days after their sub-epidermal injection (No i3D) and fabrication of parallelepiped-shaped HCC-Gel mCherry$^+$FBs laden construct (i3D) into wild-type animals across epidermis. Scale bars 1 mm.

Figure 23. Cell-laden i3D bioprinting. (A) Representative stereomicroscope images of hindlimb muscle just after (day 0) or 7 days after i3D bioprinting across epimysium of parallelepiped-shaped GFP+MuSC/mCherry+FB HCC-Gel construct (green) into wild-type animals. Scale bar 1mm. (B) Intravital 2-Photon mosaic image showing GFP+MuSC-derived cells 7 days after injection under the epimysium (no i3D) or 7 days after injection and fabrication of parallelepiped-shaped GFP+MuSC(green)/mCherry+FB-laden HCC-Gel construct (i3D) into wild-type animals. Blood flow is shown in red. Scale bar 200 μm. (C) Quantification of cell directionality of GFP+ cells in i3D bioprinted muscles (gray) or in muscles injected with GFP+MuSC (black). Data are shown as mean ± s.d. of 3 independent replicates; multiple comparison one-way ANOVA was used; ****P< 0.0001. Specific bioprinting conditions are reported in Suppl. Table S6. (D) Mosaic images showing host and de novo tissue sections. Immunofluorescence performed for GFP (green) and laminin (gray) in wild-type animals 7 days after i3D bioprinting of GFP+MuSC/m-Cherry+FB-laden HCC-Gel. Dotted line shows the limit between host muscle (above) and de novo tissue (below); scale bar, 400 μm. Nuclei were stained with Hoechst (blue). (E) Confocal images of regenerating myofibers stained for GFP (green) and eMyHC (red). Scale bars, 30 μm. Nuclei were stained with Hoechst (blue). (F) Higher magnification of regenerating myofibers stained for eMyHC (red). Arrowheads point at striated organization of the cytoskeleton (left) and presence of centrally locate nuclei (right); scale bar, 15 μm. Nuclei were stained with Hoechst (blue). (G) Left panel, quantification of GFP-negative (GFP-) and GFP-positive (GFP+) myofibers that showed centrally located nuclei (CNF) or peripheral location of the nuclei (no CNF). Data are shown as mean ± s.d. of 3 independent replicates; multiple comparison one-way ANOVA was used; ****P< 0.0001. Right panel, representative confocal image showing single multinucleated GFP+ myofiber surrounded by laminin; centrally located nuclei are highlighted by arrowheads. Scale bar 10 μm. Nuclei were stained with Hoechst (blue).

Figure 24. Multiphoton-mediated crosslinking and decrosslinking of developed hydrogels. A. CMMC-4arm PEG was crosslinked using UV light and hydrogel decrosslinking was possible by irradiation of the hydrogel in the wavelength range of 700 nm < λ < 900 nm of multiphoton NIR light. Scale bar 100 μm. B. Bright field imaging showing microfiber entrance in the newly formed channel at the beginning (left panel) and at the complete introduction (right panel) into the cavity generated within CMMC-Gel hydrogels. Scale bar 100 μm. C. Autofluorescence of microbeads (green) moving through a U-shaped channel generated within HCC-Gel hydrogel. Scale bar 100 μm. D. FITC-dextran solution (green) entering in the decrosslinked multiple ROI generated within CMMC-Gel hydrogels (black). Scale bar 100 μm. E. Quantification of the Young's modulus by atomic force microscopy analysis of HCC-Gel hydrogels subjected to 1 or multiple cycles of irradiation with a multiphoton light in specific ROI. Data are shown as mean ± s.d. of 3 and 6 independent replicates.

## DETAILED DESCRIPTION

The present invention provides a new coumarin- polymer conjugate suitable for 3D bioprinting. The particular advantage of the conjugate of the invention is that it allows the construction of 3D biocompatible structures that can be directly and precisely fabricated into pre-existing 3D solid environments, and in particular in tissues, in organs, in cell and organoid cultures or in vivo by irradiation of the conjugate with IR laser associated to surgical lasers. The new 3D bioprinting disclosed in the present description is also herein defined as intravital 3D printing or i3D printing.

The advantage of the conjugates of the invention is that they can undergo cycloaddition when irradiated at wavelengths that can penetrate biological tissues, i.e. wavelengths between 700 nm and 1000 nm.

The new conjugates of the invention, upon cycloaddition are suitable for developing in vivo or in vitro 3D biocompatible structures that can be used for:

- drugs, cells, therapeutic DNA or RNA constructs, proteins and/or material delivery into the body;
- tissue or organs regeneration and/or reparation
- ex novo tissue fabrication
- medical devices (developed in vivo or suitable for implantation)
- research applications in vivo or in vitro
- 3D printing into/or pre-existing 3D structures, tissues, ECM
- surgery on animals or humans
- organoid development
- prosthesis for medical use

and the like.

The data provided in the examples below and in the figures, provide the first proof of concept that i3D bioprinting

can be performed across tissues in different target organs in living organisms with minimal invasiveness. The i3D bioprinting with the conjugates of the invention substantially changes the paradigm of 3D bioprinting for clinical use opening up the possibility of spatial-defined scaffold bioprinting, in situ drug and cell delivery for tissue repair according with regenerative medicine principles and/or de novo fabrication of functional replacement directly within human or animal body.

The invention discloses for the first time a coumarin-polymer conjugate comprising a coumarin compound and a polymer, characterized in that said coumarin compound has maximum single-photon absorption at wavelength, Abs λ, from 310 to 450 nm, at least one electron-donor in positions 6 and/or 7 and at least an electron-acceptor group in positions 3 and/or 4 wherein said coumarin compound is selected from

7-Carboxymethoxy-4-methylcoumarin
7-Hydroxy-4-methylcoumarin
7-Amino-4-methyl-3-coumarinylacetic acid
7-Hydroxy-4-(trifluoromethyl)coumarin
7-Hydroxycoumarin-3-carboxylic acid
Ethyl 7-Hydroxycoumarin-3-carboxylate
7-Hydroxycoumarin-3-carboxylic acid N-succinimidyl ester, said conjugate being further characterized in that, when irradiated with UV light (310 nm < λ < 450) or IR light (700 nm < λ < 1000 nm), the coumarin components of different units of said coumarin-polymer conjugate undergo a cycloaddition reaction thereby forming a crosslinked compound.
Commonly known coumarin compounds derivatives known in the art, normally show a maximum single-photon absorption at wavelength (Abs λ, nm) comprised between about 290 and 440 nm as reported in literature.

The coumarin compound selected by the authors, i.e. characterized in that it has maximum single-photon absorption at wavelength, Abs λ, from 310 to 450 nm and at least one electron-donor in positions 6 and/or 7 and at least an electron-acceptor group in positions 3 and/or 4 is capable of photocycloaddition upon irradiation at a completely different wavelength, i.e. a wavelength comprised between 360-450nm at 1P absorption or 700-1000nm at 2P absorption.

According to the invention, the irradiating wavelength can be with UV light can be from 310 nm to 450 nm, or from 375 nm to 440 nm, or from 400 nm to 425 nm or any combination of the extremes provided above or, the irradiating wavelength can be with IR light from 700 nm to 1000 nm, or from 800 nm to 1000 nm, or from 850 to 950 nm, or from 900 nm to 950 nm or any combination of the extremes provided above.

In a particular advantageous embodiment of the invention the coumarin compound of the invention and the coumarin-polymer conjugate of the invention undergoes photocycloaddition upon irradiation at a wavelength of 700 nm to 1000 nm; in particular at a wavelength of 800 nm to 1000 nm; more in particular at a wavelength of 850 nm to 950 nm, even more in particular at a wavelength of 900 nm to 950 nm, i.e. at a wavelength that penetrates biological tissues, thereby allowing an in-vivo or in-tissue or in-organoid photocycloaddition, thereby allowing the formation of a hydrogel comprising said coumarin-polymer conjugate and optionally one or more drug or one or more cell type.

In one embodiment, the coumarin compound of coumarin-polymer conjugate of the invention has an electron-donor group in each of positions 6 and 7 and an electron-acceptor group in each of position 3 and 4 wherein said coumarin compound is selected from

7-Carboxymethoxy-4-methylcoumarin
7-Hydroxy-4-methylcoumarin
7-Amino-4-methyl-3-coumarinylacetic acid
7-Hydroxy-4-(trifluoromethyl)coumarin
7-Hydroxycoumarin-3-carboxylic acid
Ethyl 7-Hydroxycoumarin-3-carboxylate
7-Hydroxycoumarin-3-carboxylic acid N-succinimidyl ester.

In a particular embodiment, applicable to all the previously described embodiments as well as to the embodiments described below, the coumarin compound of the conjugate of the invention, may be optionally substituted in one or more positions of the coumarin ring, with the provision that, when a substituent is in positions 6 and/or 7 at least one substituent has an electron-donor group and, when a substituent is in positions 3 and/or 4, at least one substituent has an electron-acceptor group and said substituted coumarin compound has maximum single-photon absorption at wavelength, Abs λ, from 310 to 450 nm.

Electron-donor groups and electron-accepting groups are known and already commonly used in the arts. The skilled person would hence know how to select suitable electron-donor groups and electron-accepting groups for carrying out the invention.

In a particular embodiment, the electron-donor group is selected from:

- The oxygen anion, $-O^-$
- Alcohol groups, -OH
- Amine groups, $-NH_2$ or $-NR_2$
- Ethers, -OR
- Alkyl groups,

And the electron-accepting group is selected from:

- Nitro groups ($-NO_2$)
- Aldehydes (-CHO)
- Ketones (-C=OR)
- Cyano groups (-CN)
- Carboxylic acid (-COOH)
- Esters (-COOR).

In particular, it has been demonstrated that the maximum bathochromic shift of the absorption spectra is observed when an electron-donating substituent (-OH,-OCH,-CH3, -NH2...) is introduced in the 6- and 7-positions of the cromophore (the effect of substitution in the 6-position is higher). The stronger is the electron-donating property of the substituent, the greater is the red-shift.

The further introduction of an electron-accepting substituent (trifluoromethyl, ethoxycarbonyl, aryl, heteroaryl) into the pyrone fragment (3 and 4-positions) of the molecule induces an even greater red-shift of the absorption maximum.

Both substitutions are responsible of an increasing intramolecular charge transfer (ICT) character resulting in a lowering of the gap between energy levels of the cromophore as compared to those of coumarin and so in a pronounced red-shift.

According to an aspect of the invention, the coumarin compound of the conjugate of the invention 7-amino-4-methyl coumarin, can be excluded from anyone of the embodiments herein disclosed or claimed. In other words, in one aspect of the invention, the coumarin compound of the conjugates of the invention can be any of the compounds falling in the definitions of the description or of the claims, provided that it is not 7-amino-4-methyl coumarin. In a further aspect, the coumarin compound of the conjugate of the invention, wherein the coumarin has$NH_2$ as an electron-donor group in position 7 and no electron donor groups in position 6, and a methyl group ($-CH_3$) as an electron-acceptor group in position 3 and no electron acceptor group in position 4, can also be excluded from anyone of the embodiment herein disclosed or claimed. In other words, in one aspect of the invention, the coumarin compound of the conjugates of the invention can be any of the compounds falling in the definitions of the description or of the claims, provided that it is not a coumarin having $NH_2$ as an electron-donor group in position 7 and no electron donor groups in position 6, and a methyl group ($-CH_3$) as an electron-acceptor group in position 3 and no electron acceptor group in position 4.

In a preferred embodiment of the invention, when the coumarin compound is the 7-amino-4-methyl coumarin, it is a coumarin with an additional electron-donor group in position 6 and/or an electron-acceptor group in position 4.

In an embodiment of the invention applicable to all the previously described embodiments as well as to the embodiments described below, in the coumarin-polymer conjugate as defined in the description and in the claims, the coumarin compound can be directly conjugated by a covalent bond to the polymer, or it can be conjugated to the polymer by a chemical linker molecule as defined in the present description.

As explained above, chemical linkers provide a functional handle for the chemical bonding of biomolecules to other molecules. Firstly, linkers can introduce new functional groups or, in addition, they can be necessary as spacer to improve the spatial availability of functionalities or to modify the properties of a molecule, like solubility and stability.

In a preferred embodiment, applicable to all the previously described embodiments as well as to the embodiments described below, the linker introduces amino groups into the polymer backbone for the further functionalization with coumarin derivatives and increases the hydrophilicity and the flexibility of the polymeric backbone.

Non-limiting examples of suitable linkers according to the present invention is represented by bifunctional linkers with appropriate chemical groups and provided with the flexible and hydrophilic backbone of triethylene (PEG3) or tetraethylene glycol (PEG4).

A list of possible PEG3 and PEG4 derivatives used as linkers is provided below:

- Triethylene glycol diamine (NH2-PEG3-NH2) and tetraethylene glycol diamine (NH2-PEG4-NH2)
- Amine-PEG3-carboxilic acid (NH2-PEG3-COOH) and amine-PEG4-carboxilic acid (NH2-PEG4-COOH)
- Hydroxy-PEG3-amine (OH-PEG3-NH2) and Hydroxy-PEG4-amine (OH-PEG4-NH2)
- Hydroxy-PEG3-carboxilic acid (OH-PEG3-COOH) and hydroxy-PEG4-carboxilic acid (OH-PEG4-COOH)
- Triethylene glycol (OH-PEG3-OH) and tetraethylene glycol (OH-PEG4-OH)5.

The coumarin-polymer conjugates according to any embodiment herein disclosed, is represented by conjugates according to the description and the claims wherein said coumarin compound is selected from

7-Carboxymethoxy-4-methylcoumarin
7-Hydroxy-4-methylcoumarin
7-Amino-4-methyl-3-coumarinylacetic acid
7-Hydroxy-4-(trifluoromethyl)coumarin
7-Hydroxycoumarin-3-carboxylic acid
Ethyl 7-Hydroxycoumarin-3-carboxylate
7-Hydroxycoumarin-3-carboxylic acid N-succinimidyl ester.

In particular, the coumarin-polymer conjugate of the invention, wherein the substituted coumarin compound is 7-(carboxymethoxy)-4-methylcoumarin or 7-hydroxycoumarin-3-carboxylic acid.

Table 1, below, reports the absorbance spectra analysis of selected coumarin derivatives dissolved in PBS at pH 7.4, the maximum single photon absorption wavelengths in specific solvents are indicated.

| Compound | Abs λ, nm | Solvent |
|---|---|---|
| 7-Carboxymethoxy-4-methylcoumarin | 321 | Ethanol |
| 7-Hydroxy-4-methylcoumarin | 322 | 95% Ethanol |
| 7-Amino-4-methyl-3-coumarinylacetic acid | 340 | Methanol |
| 7-Hydroxy-4-(trifluoromethyl)coumarin | 383 | Ethanol |
| 7-Hydroxycoumarin-3-carboxylic acid | 342, 385 | Ethanol |
| Ethyl 7-Hydroxycoumarin-3-carboxylate | 403 | Ethanol |
| 7-Hydroxycoumarin-3-carboxylic acid N-succinimidyl ester | 413 | |

According to the invention, any suitable polymer can be conjugated with the coumarin compound as defined in the present disclosure.

However, due to the intended use of the conjugate, polymers that are suitable for biomedical use or for biomedical research are preferred.

Natural or synthetic polymers are suitable for the realisation of the conjugate of the invention. Natural polymers are polymers that can be isolated from natural products, synthetic polymers are polymers obtained by chemical synthesis. As the skilled person is well aware, several polymeric materials have been investigated for various biomedical applications including controlled drug release, tissue engineering and regenerative medicine. According to their origin, they can be classified as natural and synthetic polymers.

In a preferred embodiment of the invention, applicable to all the embodiments disclosed herein, the conjugated polymer is a polymer is a biomedical and/or a biocompatible polymer.

Several biomedical and/or biocompatible polymers are known and already commonly used in the arts. The skilled person would hence know how to select suitable polymers for carrying out the invention. Preferably, the skilled person will consider polymers with one or more of certain optimal biological, chemical, and physical characteristics for selecting a polymeric material for biomedical uses:

- Polymer, as well as its metabolites, should not evoke any adverse inflammatory or toxic responses in vivo.
- Polymer should be easy to process and sterilized.
- Polymer should have an acceptable shelf life and degradation time matching the application.

- Polymer should have mechanical properties to match the required application.

Typically, natural polymers are biocompatible and enzymatically biodegradable, meaning that the material is readily recognized, accepted, and mechanically processed by the body. Natural polymers can be classified into 2 main classes:

i. Polypeptides and proteins
ii. Polysaccharides

Non-limiting examples of group i. and ii. are provided below.

- Collagen
- Gelatin
- Fibrin
- Elastin
- Silk
- Bulk decellularized extracellular matrix

ii. POLYSACCHARIDES

- Cellulose and derivatives
- Alginates
- Dextran
- Chitosan
- Hyaluronic acid
- Chondroitin sulphate

Any of the polymers indicated above can be used in the conjugate of the invention. In a particular embodiment, applicable to all the embodiments disclosed herein where natural polymers are used, the natural polymer can be selected from Collagen, Gelatin, Fibrin, Elastin, Silk, Bulk decellularized extracellular matrix, protein extract from decellularized extracellular matrix, Cellulose and derivatives, including methyl cellulose (MC), hydroxypropyl cellulose (HPC), hydroxypropylmethyl cellulose (HPMC), and carboxymethyl cellulose (CMC) thereof, Alginates, Dextran, Chitosan, Hyaluronic acid, Chondroitin sulfate.

According to the invention, cellulose derivative comprise including methyl cellulose (MC), hydroxypropyl cellulose (HPC), hydroxypropylmethyl cellulose (HPMC), and carboxymethyl cellulose (CMC).

However, due to the fact that natural polymers could present disadvantages as immunogenicity, variable and complex structure, inadequate mechanical properties and difficulty in controlling material degradability, synthetic polymers suitable for biomedical use as explained above, can be used in the conjugate of the invention.

Synthetic biomaterials are generally biologically inert, and they can be produced under controlled conditions. They have reproducible and tailored physical, mechanical and chemical properties according to the requirements of specific applications. They can be biodegradable or nondegradable biomaterials.

A non-limiting example of biodegradable synthetic polymers that can be used in the conjugate of the invention are polyesters, polyurethanes, polyanhydrides, polyphosphazenes, poly(propylenedumarates).

Specific, non-limiting examples of said biodegradable synthetic polymers is

i. Polyesters:
This class of synthetic polymers has been approved by FDA for various applications, resulting in a great variety of biomedical products currently on the market. The most employed are:

- Poly(lactic acid) (PLA)
- Poly(glycolic acid) (PGA)
- Copolymers of poly(lactic-co-glycolic acid) (PLGA)
- Poly($\varepsilon$-caprolactone) (PCL)
- polyhydroxybutyrate (PHB)
- poly(hydroxyvalerate) (PHV)
- copolimeri di PHB e 3-hydroxyvalerate (PHBHV)

ii. Polyurethanes

- Lysine diisocyanate (LDI)
- 1,4-diisocyanatobutane (BDI) ....

iii. Polyanhydrides: are FDA approved polymers for drug delivery application. Some polyanhydrides-based drug delivery vehicles are currently on the market for various applications. An example is:

- poly[(carboxy phenoxy propane)-(sebacic acid) P(CPP-SA)

iv. Polyphosphazenes: inorganic-organic hybrid polymers with a backbone of alternating phosphorus and nitrogen atoms with two organic side groups attached to each phosphorus atom. Some polyphosphazenes currently investigated for biomedical applications are:

- Poly[bis(ethyl glycinato)phosphazene]
- Poly[ethyl alanato)-(p-phenyl phenoxy) phosphazene]
- poly[di(carboxylatophenoxy)phosphazene] (PCPP)
- poly[di(methoxyethoxyethoxy)phosphazene] (MEEP)

v. Poly(Propylene Fumarate)

Also, in another embodiment of the invention, e.g. when a permanent structure is desired, nondegradable synthetic polymers can be used for carrying out the invention. This kind of polymers has been extensively used in medicine as drug delivery systems, fillings, orthopaedic implants, ocular lens, heart valves, bone cements, vascular grafts and tissue engineering scaffolds

Nondegradable polymers can also be indicated as biostable or biointegrable polymers as these offers enduring support over time, overtaking the problematic of premature degradation and formation of harmful degradation products. The main advantages are ease of processing, tissue/blood compatibility, biological stability, robustness and excellent mechanical properties.

As known in the art, nondegradable synthetic polymers for biomedical applications are mainly represented by :

i. poly(ethylene)
ii. poly(propylene)
iii. poly(methyl methacrylate) (PMMA): highly employed in medical applications as dental filling, intraocular lens, bone cement for osteoporotic bone and for implant fixation
iv. poly(dimethyl siloxane): a bio stable synthetic polymer used for biomedical applications such as drug delivery vehicles and blood-contacting biomaterial.
v. poly(ethylene oxide): water soluble polymer, it is approved by FDA for various medical applications. PEO has hydrogel-forming characteristics, which can be suitable for developing drug delivery vehicles and regenerating scaffolds.
vi. poly(propylene oxide)
vii. poly(vinyl alcohol) (pva)

In a preferred embodiment the following polymers for coumarin conjugation can be used:

Direct conjugation

- Collagen
- Gelatin
- Elastin
- Bulk decellularized extracellular matrix

Indirect conjugation through a linker (after introduction of an amine group in the backbone)

- Alginates
- Dextran
- Hyaluronic acid

- Water- soluble polyanhydrides: ex P(CPP-SA)
- Water-soluble polyphosphazenes: ex PCPP, MEEP
- Poly(ethylene oxide) (PEG)
- PVA

Conjugation according to the present invention can be carried out as described in the examples, and, more in general, as follows:
activation of the carboxylic group of 7-hydroxycumarin-3-carboxylic acid (HCCA) or 7-(carboxymethoxy)-4-methylcoumarin (CMMAC) is used a preliminary step before polymer conjugation. To do so, HCCA or CMMC were dissolved in anhydrous dimethyl sulfoxide (DMSO) and N-hydroxysuccinimide was added to the solution. After 10 min, N,N'-dicyclohexylcarbodiimide (DCC) was added. The reaction mixture was stirred overnight, then filtered, precipitated dropwise in diethyl ether, rinsed with diethyl ether and finally desiccated under vacuum. For gelatin conjugation, Gelatin (Gel) or PEG-polymers were dissolved in anhydrous DMSO, then triethylamine (Et3N) was added to the solution. Finally, activated HCCA-NHS or CMMC-NHS dissolved in anhydrous DMSO, was added to the mixture. After 12 h stirring, the reaction mixture was diluted with phosphate buffer, dialyzed with a Spectra/Por dialysis membrane.

According to the invention specific possible combination coumarin compound-(linker if needed)-polymer are disclosed in the table 2 below:

| COUMARIN COMPOUND | POLYMER | LINKER | CONJUGATION POSITION |
|---|---|---|---|
| 7-Carboxymethoxy-4-methylcoumarin (herein and conventionally also named "Coumarin A") | -Gelatin (1), Collagen (2), Elastin (3), decellularized extracellular matrix (4), branchedPEG-$NH_2$ (5).<br>-Alginates (6), dextran (7), hyaluronic acid (8), polyvinyl alcohol (9). | - Polymers 1, 2, 3, 4, 5 direct conjugation to coumarin<br>- Polymer 6, 7, 8, 9 indirect conjugation through a linker.<br><u>Alginates</u>: $NH_2$-$PEG_{3\ or\ 4}$-$NH_2$ and $NH_2$-$PEG_{3\ or\ 4}$-OH.<br><u>Dextran:</u><br>$NH_2$-$PEG_{3\ or\ 4}$-COOH.<br><u>Hyaluronic acid:</u><br>$NH_2$-$PEG_{3\ or\ 4}$-$NH_2$ and $NH_2$-$PEG_{3\ or\ 4}$-OH.<br><u>Polyvinyl alcohol</u>:<br>$NH_2$-$PEG_{3\ or\ 4}$-COOH | Conjugation to the carboxylic group of the substituent in $C_7$ of coumarin through amide bond |
| 7-Hydroxycoumarin-3-carboxylic acid (herein and conventionally also conventionally named "Coumarin B") | -Gelatin (1), Collagen (2), Elastin (3), decellularized extracellular matrix (4), branchedPEG-$NH_2$ (5).<br><br>-Alginates (6), dextran (7), hyaluronic acid (8), Polyvinyl alcohol (9). | - Polymers 1, 2, 3, 4, 5 direct conjugation to coumarin<br>- Polymer 6, 7, 8, 9 indirect conjugation through a linker.<br><br><u>Alginates:</u> $NH_2$-$PEG_{3\ or\ 4}$-$NH_2$ and $NH_2$-$PEG_{3\ or\ 4}$-OH.<br><u>Dextran:</u><br>$NH_2$-$PEG_{3\ or\ 4}$-COOH.<br><u>Hyaluronic acid:</u><br>$NH_2$-$PEG_{3\ or\ 4}$-$NH_2$ and $NH_2$-$PEG_{3\ or\ 4}$-OH.<br><u>Polyvinyl alcohol</u>:<br>$NH_2$-$PEG_{3\ or\ 4}$-COOH | Conjugation to the carboxylic group of $C_3$ of coumarin ring through amide bond |
| 7-Amino-4-methyl-3-coumarinylacetic acid | -Gelatin (1), Collagen (2), Elastin (3), decellularized extracellular matrix (4), branchedPEG-$NH_2$ (5).<br>-Alginates (6), dextran (7), hyaluronic acid (8), Polyvinyl alcohol (9). | - Polymers 1, 2, 3, 4, 5 direct conjugation to coumarin<br>- Polymer 6, 7, 8, 9 indirect conjugation through a linker.<br><u>Alginates:</u> $NH_2$-$PEG_{3\ or\ 4}$-$NH_2$ and $NH_2$-$PEG_{3\ or\ 4}$-OH.<br><u>Dextran:</u><br>$NH_2$-$PEG_{3\ or\ 4}$-COOH. | Conjugation to the carboxylic group of the substituent in $C_3$ of coumarin ring through amide bond |

(continued)

| COUMARIN COMPOUND | POLYMER | LINKER | CONJUGATION POSITION |
|---|---|---|---|
| | | Hyaluronic acid: $NH_2$-$PEG_{3\ or\ 4}$-$NH_2$ and $NH_2$-$PEG_{3\ or\ 4}$-OH. Polyvinyl alcohol: $NH_2$-$PEG_{3\ or\ 4}$-COOH | |
| Ethyl 7-Hydroxycoumarin-3-carboxylate | -Gelatin (1), Collagen (2), Elastin (3), decellularized extracellular matrix (4), branchedPEG-$NH_2$ (5). -Alginates (6), dextran (7), hyaluronic acid (8), Polyvinyl alcohol (9). | - Polymers 1, 2, 3, 4, 5 direct conjugation to coumarin - Polymer 6, 7, 8, 9 indirect conjugation through a linker. Alginates: $NH_2$-$PEG_{3\ or\ 4}$-$NH_2$ and $NH_2$-$PEG_{3\ or\ 4}$-OH. Dextran: $NH_2$-$PEG_{3\ or\ 4}$-COOH. Hyaluronic acid: $NH_2$-$PEG_{3\ or\ 4}$-$NH_2$ and $NH_2$-$PEG_{3\ or\ 4}$-OH. Polyvinyl alcohol: $NH_2$-$PEG_{3\ or\ 4}$-COOH | Conjugation to the ester group of the substituent in $C_3$ of coumarin ring through amide bond |
| 7-Aminocoumarin-3-carboxylic acid (herein and conventionally also conventionally named "Coumarin C") | -Gelatin (1), Collagen (2), Elastin (3), decellularized extracellular matrix (4), branchedPEG-$NH_2$ (5). -Alginates (6), dextran (7), hyaluronic acid (8), Polyvinyl alcohol (9). | - Polymers 1, 2, 3, 4, 5 direct conjugation to coumarin - Polymer 6, 7, 8, 9 indirect conjugation through a linker. Alginates: $NH_2$-$PEG_{3\ or\ 4}$-$NH_2$ and $NH_2$-$PEG_{3\ or\ 4}$-OH. Dextran: $NH_2$-$PEG_{3\ or\ 4}$-COOH. Hyaluronic acid: $NH_2$-$PEG_{3\ or\ 4}$-$NH_2$ and $NH_2$-$PEG_{3\ or\ 4}$-OH. Polyvinyl alcohol: $NH_2$-$PEG_{3\ or\ 4}$-COOH | Conjugation to the carboxylic group of the substituent in $C_3$ of coumarin ring through amide bond |
| 7-Hydroxy-4-methylcoumarin | -Gelatin (1), Collagen (2), Elastin (3), decellularized extracellular matrix (4), branchedPEG-OH (5), polyvinyl alcohol (9). -Alginates (6), dextran (7), hyaluronic acid (8). | - Polymers 1, 2, 3, 4, 5, 9 direct conjugation to coumarin - Polymer 6, 7, 8, 9 indirect conjugation through a linker. Alginates: $NH_2$-$PEG_{3\ or\ 4}$-OH. Dextran: OH-$PEG_{3\ or\ 4}$-COOH. Hyaluronic acid: $NH_2$-$PEG_{3\ or\ 4}$-OH and OH-$PEG_{3\ or\ 4}$-OH. | Conjugation to the hydroxylic group in $C_7$ of coumarin ring through ether bond |
| 7-Hydroxy-4-(trifluoromethyl)coumarin | -Gelatin (1), Collagen (2), Elastin (3), decellularized extracellular matrix (4), branchedPEG-OH (5), polyvinyl alcohol (9). | - Polymers 1, 2, 3, 4, 5, 9 direct conjugation to coumarin - Polymer 6, 7, 8, 9 indirect conjugation through a linker. Alginates: $NH_2$-$PEG_{3\ or\ 4}$-OH. | Conjugation to the hydroxylic group in $C_7$ of coumarin ring through ether bond |

(continued)

| COUMARIN COMPOUND | POLYMER | LINKER | CONJUGATION POSITION |
|---|---|---|---|
| | -Alginates (6), dextran (7), hyaluronic acid (8). | Dextran: OH-PEG$_{3\ or\ 4}$-COOH. Hyaluronic acid: $NH_2$-PEG$_{3\ or\ 4}$-OH and OH-PEG$_{3\ or\ 4}$-OH. | |

In an embodiment, applicable to all the embodiments disclosed in the present description, the coumarin-polymer conjugate of the invention can be provided in aqueous suspension (as in phosphate buffered saline) or in lyophilized form.

The conjugate of the invention can be prepared in combination with one or more drugs, including growth factors and/or synthetic drugs, and/or one or more cell type, including somatic differentiated cells, stem cells of human or another animal origin wherein said stem cells are preferably non embryonic stem cells.

As stated above, in all the embodiments of the invention, when the conjugate as defined in the description and in the claims (coumarin compound has maximum single-photon absorption at wavelength, Abs λ, from 310 to 450 nm, and a maximum two-photon absorption at wavelength Abs λ, from 700 to 1000 nm at least one electron-donor in positions 6 and/or 7 and/or at least an electron-acceptor group in positions 3 and/or 4 and embodiments thereof herein claimed and/or described) is irradiated with IR light (700 nm < λ < 1000 nm), the coumarin components of different units of said coumarin-polymer conjugate undergo a cycloaddition reaction thereby forming a crosslinked product.

According to the invention, the irradiating wavelength can be with IR light from 700 nm to 1000 nm, or from 800 nm to 1000 nm, or from 850 to 950 nm, or from 900 nm to 950 nm or any combination of the extremes provided above.

An object of the present invention is also a mixture of coumarin-polymer conjugates as defined in the description and in the claims. The conjugates in the mixture can be two or more of any conjugate as described herein, therefore the mixture can be a mixture comprising conjugates with the same coumarin compound and different polymers (either natural polymers, natural and synthetic polymers or synthetic polymers as defined above), or it can be a mixture comprising conjugates with different coumarin compounds conjugated with the same polymer (natural or synthetic), or a mixture comprising both possibilities.

In an embodiment of the invention, the mixture can comprise at least two of any of the conjugates depicted in table 2 above.

In an embodiment of the invention, the mixture may comprise cell-adhesive coumarin-polymer conjugates (including PEG-conjugates) and cell-non adhesive coumarin-polymer conjugates (including Gel-conjugates).

In an embodiment of the invention applicable to all the embodiments described herein, the mixture of the invention can be prepared in aqueous suspension (as in phosphate buffered saline) or in lyophilized form (solid).

In a further embodiment, applicable to all the embodiments described herein, the mixture of coumarin-polymer conjugates of the invention, can be provided in combination with one or more drugs, including growth factors and/or synthetic drugs, and/or one or more cell type, including somatic differentiated cells, stem cells of human or another animal origin.

As stated above, in all the embodiments of the invention, when the conjugates of the mixture as defined in the description and in the claims is irradiated with IR light (700 nm < λ < 1000 nm), the coumarin components of different units of said coumarin-polymer conjugate undergo a (photo)cycloaddition reaction thereby forming a crosslinked product. The (photo)cycloaddition can be performed by using light emitting diodes (LEDs) and 3D photo-bioprinters, or multiphoton laser associated to optical set-ups, including nanoscribe and microscopes.

The conjugates of the invention can be crosslinked as disclosed in the examples, and more generally as follows: For in vitro cell culture application, hydrogels can be fabricated by (photo)cycloaddition, developed and then cells of interest can be seeded on the top of the fabricated hydrogels in presence or absence of coatings; cells can be resuspended into liquid coumarin-polymer conjugate solution and then the suspension can be photo-crosslinked (cell-laden hydrogels), developed and cultured in accordance with the experimental needs; for hydrogel fabrication within pre-existing 3D environment, liquid coumarin-polymer conjugate can be combined with natural gels in presence or absence of cells (as mixture of a liquid solution or loaded on the top of solid gels) and cultured in accordance with the experimental needs. Hydrogel can be fabricated at the beginning of the culture (time 0 of culture) as well as during cell culture. In the last case, liquid coumarin-polymer conjugate can be added over the culture in absence of culture media and (photo)cycloaddition can be performed in absence or presence of cells.

For in vivo application, coumarin-polymer conjugates, in presence or absence of donor cells (autologous or heterologous), are injected in or expose on the anatomical site of interest in living animals subjected to general anesthesia. In case it is required, the anatomical site can be surgically exposed to allow hydrogel localization in the area of interest. In

case it will be required, after (photo)cycloaddition, sutures will be performed to close the surgically exposed anatomical site.

The invention also relates to a product comprising one or more coumarin-polymer conjugates or the mixture as defined in the description and in the claims whereby the coumarin components of different units of said coumarin-polymer conjugates are cross-linked via a (photo)cycloaddition reaction between different coumarin compounds.

In other words, the invention relates to a product obtainable by inducing via irradiation with a radiation source at a wavelength 700 nm < λ < 1000 nm (IR light for two-photon absorption), as described above, coumarin-polymer conjugates as defined in the present specification and claims, i.e.wherein the coumarin compound in the conjugate has maximum single-photon absorption at wavelength, Abs λ, from 300 to 450 nm, at least one electron-donor in positions 6 and/or 7 and/or at least an electron-acceptor group in positions 3 and/or 4 as defined in the claims and in the detailed description of the invention, and a polymer as defined in the detailed description and in the claims, in one embodiment the product further comprising additional compounds or biological materials.

Hence, the invention refers also to a method for the preparation of a product comprising cross-linked coumarin-polymer conjugates as defined in the specification and in the claims comprising the step of
subjecting to irradiation coumarin-polymer conjugates or a mixture thereof having maximum single-photon absorption at wavelength, Abs λ, from 310 to 450 nm, a maximum 2 photon absorption at wavelength from 700 to 1000 nm, at least one electron-donor in positions 6 and/or 7 and/or at least an electron-acceptor group in positions 3 and/or 4 as defined in the description and in the claims with a radiation source at a wavelength 700 nm < λ < 1000 nm.

In an embodiment, the method of the invention comprises the step of
subjecting to irradiation coumarin-polymer conjugates or a mixture thereof having maximum single-photon absorption at wavelength, Abs λ, from 310 to 450 nm, a maximum 2 photon absorption at wavelength from 700 to 1000 nm, at least one electron-donor in positions 6 and/or 7 and/or at least an electron-acceptor group in positions 3 and/or 4 and a polymer as defined in the description and in the claims with a radiation source at a wavelength 700 nm < λ < 1000 nm wherein said conjugate or mixture thereof is in combination with one or more drugs, including growth factors and/or synthetic drugs, and/or one or more cell type, including somatic differentiated cells, stem cells of human or another animal origin prior to irradiation.

An embodiment according to anyone of the embodiments herein disclosed or claimed, provides a method for the preparation and the subsequent disruption of a product comprising cross-linked coumarin-polymer conjugates as defined in the specification and in the claims comprising the step of

i) subjecting to irradiation a coumarin-polymer conjugates or a mixture thereof having maximum single-photon absorption at wavelength, Abs λ, from 310 to 450 nm, a maximum 2 photon absorption at wavelength from 700 to 1000 nm, at least one electron-donor in positions 6 and/or 7 and/or at least an electron-acceptor group in positions 3 and/or 4 as defined in the description and in the claims with a radiation source at a wavelength 700 nm < λ < 1000 nm
ii) subsequently subjecting the product thereby obtained to irradiation with a radiation source at a wavelength 700 nm < λ < 1000 nm

The disruption of the product (de-crosslinking) in ii) can be made after a suitable amount of time, decided depending on the intended use of the crosslinked product obtained in i). Additionally

All the detailed description of suitable coumarin compounds and polymers provided in the present specification and in the claims apply to the method generally described above. Therefore, any coumarin compound as defined in the present description and claims linked to any of the polymers as defined in the present description and in the claims can be used to form a product according to the present description and claims that can be irradiated as described above.

In one embodiment, the irradiation can be carried out on a mixture of one or more coumarin-polymer conjugated compounds as defined in the present specification in order to obtain a product comprising a mixture of cross-linked coumarin-polymer conjugated compounds as defined in the description and in the claims. The invention also provides a method of bioprinting wherein coumarin-polymer conjugates according as defined in the description and in the claims are deposited onto and/or injected into

the tissue of interest and exposed to the irradiation source to allow hydrogel crosslinking, or are mixed with cells or organoids and photo-crosslinked, or premixed with or loaded into pre-exiting gels containing cells and or organoids and then crosslinked by irradiation with the light of interest.

Several examples of the product are provided in the description and in the examples.

As biomaterials, including fillers for soft tissues augmentation in aesthetic plastic surgery, such as for filling soft tissues voids (including wrinkles, scars), or breast augmentation.

Nowadays, there are many injectable dermal filler based on natural and synthetic material used for the treatment of damaged and aged skin, but the gelation mechanism doesn't allow a spatial and shape control of the scaffold. Further, some subcutaneous filler on the market require surgical intervention that might result in medical defects. Instead, our biomaterial can be injected intradermally, subcutaneously or into internal tissues in a non-invasive manner and 3D

modelled by using IR-light photopolymerization based on previous CAD-assisted design in order to generate a scaffold with appropriate topographic characteristics (shape, size) respect to the area of interest and with desired stiffness. Further, this application can be performed without surgical intervention, but also in open surgery to reach deeper tissues.

According to the invention, the product of the invention may further comprise one or more drug and/or one or more cell type.

One application example includes the use of conjugates to allow controlled and spatially defined drug release by combining intravital imaging and intravital 3D printing. The polymeric solution containing drugs or drugs encapsulated into nano-size drug delivery systems, can be easily injected in the site of interest (i.e. a tumorigenic mass or other specific tissues that needs to be treated) and in situ crosslinked to have a spatially located hydrogel. This approach can ensure an higher dose at the site of interest, a prolonged drug release and a reduction of side effects on healthy tissues, improving the specificity of the treatment as well as reducing the systemic toxicity. The possibility to control the shape and the design of the hydrogel guarantees a local delivery system perfectly fitted and modulated to the geometry of the area of interest (higher contact with the area of interest) and to combine the advantages derived from the use of a controlled drug delivery system with the use of a 3D matrix for cell guidance.

Another application example includes the use of conjugates to allow DNA/RNA delivery for gene therapy. The biomaterial containing the nucleic acid of interest (DNA, RNA constructs or viral vectors) can be injected at a specific tissue through a needle or endoscope, avoiding invasive surgical procedure, and in situ crosslinked for in vivo gene therapy application. Such treatment can include both genetic diseases - i.e. delivery of the corrected/therapeutic form of the molecule of interest inside the tissue; or other diseases in which the nucleic acid or its transduced-product act as foreign molecules able to modify the host response

One application example includes the use of conjugates to allow delivery of active molecules for immunomodulation therapy. By combining intravital imaging and intravital 3D printing, active molecules (including antibodies) loaded/cross-linked to the hydrogels can be spatially located at the area of interest - i.e. embedding a tumorigenic mass or other specific tissues that needs to be treated; attraction or inhibition of immune system at the specific area of interest.

Another application example includes the use of conjugates to allow encapsulation of living cells for the secretion of active compounds as enzymes, growth factors, hormones, including indirect exosomes delivery. The application includes orthotropic or ectopic delivery of wild-type or genetically modified cells to promote expression of active molecules during aging, in genetic disorders, killer compounds for tumours or to treat hormonal deficiencies Cells encapsulation into a 3D scaffold represents an appealing alternative to current therapy, based on oral administration or injection of the missing substance, reducing the degradation risk of the active compounds, promoting a long-term secretion of the substance and higher dose of the molecule in the site of interest.

In an embodiment of the invention, the product can be in the form of a hydrogel.

A further object of the invention, is a medical device comprising the coumarin-conjugate, or the mixture or the product according to any embodiment provided in the description.

The product or the device of the invention may be printed in 3D in vitro or in vivo as described in the examples or more generally according to the methods provided below: The medical device according to the invention can be also in the form of an implantable device.

One example of the application of the invention can be the combination of the coumarin-conjugate of the invention to other synthetic, natural (decellularised organs/tissues) or combined-devices to give new characteristics to the original products and modify donor or host cell responses. This application can be performed ex-vivo or intravital. i.e. Micro-patterning of synthetic implants for vascular repair to promote functional regeneration; Functional 3D connection of the device (like natural sutures) in vivo to reduce inflammatory response, fibrotic tissue formation etc -one of the most adverse effect of implants are related to the "junction" between the device and the tissues. Treatment of the external surface of implantable device to promote or reduce host cell recruitment and deposition.

Another example is to use coumarin-conjugates to assist medical devices' implantation, including 3D localization of nano-objects or micrometrical objects in the body or integration of an electrical interface in the brain.

Yet another further object of the invention is its application for in vitro studies. In particular, use of 3D printing to create 3D scaffolds with micrometrical resolution, that can be used for biological studies. Examples are: 3D cell cultures -classical meaning at micrometric resolution; 3D patterning (mechanical and/or molecular) -i.e. generation of 3D structures of defined shape that have volumetric tunable mechanical properties in XYZ and/or composed by volumes of different molecules located at specific XYZ. Such structures can be used to grow cells on the top and drive their behaviour, including migration, self-assembly and differentiation; bioprinting - classical meaning; 3D patterned bioprinting -i.e. same as before but with cell loaded hydrogel; dynamic modification of 3D cell culture (cell loaded or cultured on the top). Modification of the hydrogel stiffness during cell culture; tunable 3D cultures in gelified substrates (including matrigel, mainly for organoids); static and dynamic (during cell culture) mechanical confinement to drive morphogenesis and differentiation; static and dynamic functional confinement to drive morphogenesis and differentiation -i.e. by using functional proteins conjugated with coumarin; 3D mechanical patterning (static and dynamic); Combination of all of them. Other examples are: 3D bioprinting in 3D scaffolds -i.e. inside decellularised tissues/organs or other scaffolds;

intracellular photo-crosslinking -i.e. use of cytoskeleton proteins (example tubulin) conjugated with coumarin to be injected or delivered into cell and photocrosslinked to stiffning/polarize the cytoskeleton; use of proteins that regulate organelles dynamics (mitochondria fusion or fission; Vesicle fusion for autophagy; ER/Golgi trafficking; neuro-vescicles) conjugated with coumarin to be injected or delivered into cell to modify their dynamics; membrane/junctions photo-crosslinking -i.e. use of molecule that fuse with the membranes and functionalised with coumarin to have stiffing of specific area of cell membrane/cell junctions.

Still, an other object of the invention is a scaffold construct comprising the combination of product of the invention and cells, as for building new structures that are not present or are damaged in the body, able to provide new functions that are not existing. Examples of such application are: reconstruction of pancreatic islets, also ectopically; reconstruction of crystalline in the eye; fabrication of bypass; fabrication of aortic valve in case of congenital or acquired heart valve defects.

The product or the device of the invention may be printed in 3D in vitro or in vivo as described in the examples or more generally according to the methods provided below: For in vitro cell culture application, hydrogels can be fabricated by (photo)cycloaddition, developed and then cells of interest can be seeded on the top of the fabricated hydrogels in presence or absence of coatings; cells can be resuspended into liquid coumarin-polymer conjugate solution and then the suspension can be photo-crosslinked (cell-laden hydrogels), developed and cultured in accordance with the experimental needs; for hydrogel fabrication within pre-existing 3D environment, liquid coumarin-polymer conjugate can be combined with natural gels in presence or absence of cells (as mixture of a liquid solution or loaded on the top of solid gels) and cultured in accordance with the experimental needs. Hydrogel can be fabricated at the beginning of the culture (time 0 of culture) as well as during cell culture. In the last case, liquid coumarin-polymer conjugate can be added over the culture in absence of culture media and (photo)cycloaddition can be performed in absence or presence of cells.

For in vivo application, coumarin-polymer conjugates, in presence or absence of donor cells (autologous or heterologous), are injected in or expose on the anatomical site of interest in living animals subjected to general anesthesia. In case it is required, the anatomical site can be surgically exposed to allow hydrogel localization in the area of interest. In case it will be required, after (photo)cycloaddition, sutures will be performed to close the surgically exposed anatomical site.

For in vivo application, hydrogels fabricated with (photo)cycloaddition according to anyone of the methods herein disclosed or claimed, can optionally undergo to a method for the decrosslinking of a coumarin-polymer conjugate or a mixture thereof, wherein said decrosslinking method comprises the step of subjecting said conjugate or mixture thereof to irradiation with a radiation source at a wavelength 700 nm < $\lambda$ < 1000 nm, where required from a medical or a therapeutic point of view.

Further, in an embodiment according to anyone of the embodiment described above or below the present description and claims, said decrosslinking method can be applied on the whole or on one or more selected portion of said hydrogel, allowing to modify target portions of said hydrogel.

The feature described above that allows to modify target portions of the hydrogel fabricated with (photo) cycloaddition according to anyone of the methods herein disclosed or claimed, may advantageously be used for any type of therapy or medical use that involves a localized degradation of the hydrogel structure, thereby allowing, by way of example, the construction of bypasses or channels within the hydrogel, or to give to the hydrogel structure a desired shape.

A further object of the invention is represented by the conjugates, mixtures, products according to any of the embodiments of the invention, for use in a medical treatment.

Finally, the invention also discloses medical treatments of regeneration or reparation, or of grafting of medical prosthesis or requiring delivery of therapeutic drugs, proteins, nucleic acids, cells, wherein a patient in need thereof is treated with the products of the invention, or wherein a patient in need thereof is subjected to in vivo 3D bioprinting with the coumarin conjugate of the invention, or with the mixture of the invention with the methods disclosed above and in the examples. These methods of treatment are not according to the invention as claimed.

The examples provided below are non-limiting examples for carrying out several embodiments of the invention.

EXAMPLES

MATERIALS AND METHODS

**1. Chemicals**

Two kDa methoxy PEG amine (mPEG-$NH_2$), 10 kDa 4-arm PEG amine pentaerythritol core (4arm-PEG-$NH_2$), 20 kDa 8-arm PEG amine tripentaerythritol core (8arm-PEG-$NH_2$) were obtained from Jenkem Technology (Allen, TX, USA). 7-hydroxycoumarin-3-carboxylic acid (HCCA) and ethyl 7-hydroxycoumarin-3-carboxylic acid (HCCA) were purchased from TCI Chemicals (Tokyo, Japan). Gelatin from porcine skin (Gel, type A, 300 bloom, cell culture tested), 7-(carboxymethoxy)-4-methylcoumarin (CMMC), 7-hydroxycoumarin (HC), 7-hydroxy-4-methylcoumarin (HMC), 7-hydroxy-4-(trifluoromethyl)coumarin (HTMC), 7-amino-4-methyl-3-coumarinylacetic acid (AMCAA), N-hydroxysuccinimide (NHS),

N,N'-dicyclohexylcarbodiimide (DCC), triethylamine (TEA), 2,4,6-trinitrobenzensulfonic acid (TNBS), 3-aminopropyltriethoxysilane (APTES), Dulbecco's Phosphate Buffered Saline without calcium and magnesium chloride (PBS), anhydrous dimethylsulfoxide (DMSO), diethyl ether, absolute ethanol, deuterated chloroform ($CDCl_3$), deuterated DMSO (DMSO-$d_6$), acetonitrile and trifluoroacetic acid were purchased from Sigma-Aldrich (St. Louis MO, USA). Water for the preparation of all solutions was "ultrapure" water (Milli-Q, 0.06 μSiemens $cm^{-1}$) produced with a Millipore Milli-Q purification system (MA, USA). Salts and buffers were purchased from Fluka Analytical (Buchs SG, Switzerland) and Sigma-Aldrich (St. Louis, MO, USA).

**2. Microscopes and imaging**

For *intravital* imaging, i3D bioprinting and *in vitro* imaging, Scientifica 2-photon microscope, equipped with a Nikon LWD 16x/0.80 W DIC N2 WD3.0 objective, and with a pulsed Ti:Sapphire laser (Chameleon Vision II, Coherent), of 140 fs pulse width at peak, and 80MHz repetition rate was used. Confocal microscopes Leica TCS SP5 or Zeiss LSM710 were used for confocal imaging acquisition. Epifluorescence microscopes Leica DMIL LED or Olympus BX60 equipped with Olympus DP50 digital camera were used for *in vitro* imaging. Fluorescence stereomicroscope Leica MZ16F

equipped with Canon EOS1000D camera were used to perform in brain hydrogel injection and imaging after i3D bioprinting. Confocal images acquired by single- or 2P-microscopes were processed with Fiji program for 3D reconstruction and video preparation.

**3. Animals**

All the procedures performed on animals were approved by the Ethics Committee of the University of Padova and authorized by the Italian Ministry of Health, animal license n. 601/2017-PR. Experiments were performed in 4-6 month old wild-type mice of the inbred C57BL/6J strain and in transgenic C57BL/6-(ACTB-EGFP)/J mice. Mice were housed in individual cages in an environmentally controlled room (23°C, 12 h light/12 h dark cycle) and provided food and water ad libitum.

**4. Analysis of absorbance spectra of coumarin derivatives**

Selected coumarin derivatives were dissolved in DMSO at a concentration of 5 mg/ml and diluted 1:500 in PBS, pH 7.4. The absorption spectra were recorded on an Evolution 201 UV-Vis spectrophotometer (Thermo Fisher Scientific, Waltham, MA, USA) in the wavelength range of 250-450 nm.

**PREPARATION EXAMPLES**

**1. SYNTHESIS OF 7-(CARBOXYMETHOXY)-4-METHYLCOUMARIN-GELATIN (CMMC-Gel)**

**1.1 MATERIALS AND EQUIPMENTS**

7-(carboxymethoxy)-4-methylcoumarin (CMMC), Gelatin from porcine skin (Gel, type A, 300 bloom, cell culture tested), N-hydroxysuccinimide (NHS), N,N'-dicyclohexylcarbodiimide (DCC), triethylamine (TEA), dimethylsulfoxide (DMSO) and anhydrous DMSO, diethyl ether, phosphate buffered saline w/o $Ca^{2+}$ and $Mg^{2+}$ (PBS) were purchased from Sigma-Aldrich (St. Louis MO, USA). Water for the preparation of all solutions was "ultrapure" water (Milli-Q-grade, 0.06 μSiemens $cm^{-1}$) produced with a Millipore Milli-Q purification system (MA, USA). Salts and buffers were purchased from Fluka Analytical (Buchs SG, Switzerland) and Sigma-Aldrich (St. Louis, MO, USA). Dialysis was performed with Spectra/Por dialysis membranes with a MW cutoff of 12-14 kDa (Spectrum Labs, San Francisco, CA, USA).

(scheme 1)

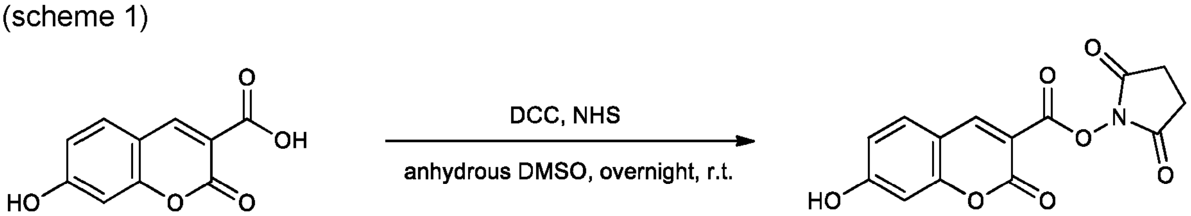


Schematic representation of the HCCA-NSE synthesis. Activation step of the carboxylic group of 7-Hydroxycoumarin-3-carboxylic acid to 7-Hydroxycoumarin-3-carboxylic acid N-succinimidyl ester using DCC and N-Hydroxysuccinimide (NHS) in anhydrous DMSO.

### 1.2 SYNTHESIS PROCEDURE

The synthesis of 7-(carboxymethoxy)-4-methylcoumarin-conjugated Gelatin (CMMC-Gel) was performed into two steps: the activation of the carboxylic group of 7-(carboxymethoxy)-4-methylcoumarin and the further conjugation to Gelatin.

**STEP 1: Activation of the carboxylic group of 7-(carboxymethoxy)-4-methylcoumarin**

- 7-(carboxymethoxy)-4-methylcoumarin (CMMC, 60.00 mg, 0.26 mmol) was dissolved in anhydrous dimethyl sulfoxide (DMSO, 1.2 mL) in a glass bottom flask, previously put in the oven overnight to let it anhydrous.
- N-hydroxysuccinimide (NHS, 58.97 mg, 0.51 mmol) was added to the solution.
- After 10 min, N,N'-dicyclohexylcarbodiimide (DCC, 105.72 mg, 0.51 mmol) was added.
- The reaction mixture was stirred overnight in the dark at room temperature
- The mixture was filtered with gooch filter (porosity 4) under vacuum to remove the insoluble dicyclohexylurea.
- The solution was precipitated dropwise in diethyl ether (45 mL) under vigorous stirring and centrifuged at 4000 rpm.
- The precipitate was rinsed with diethyl ether (30 mL × 3 times) and finally desiccated under vacuum. The product 7-(carboxymethoxy)-4-methylcoumarin N-succinimidyl ester (CMMC-NHS) was obtained as white solid.

**STEP 2: Conjugation of 7-(carboxymethoxy)-4-methylcoumarin N-succinimidyl ester to Gelatin**

- Gelatin (Gel, 1.0 g, 12.5 μmol) was dissolved in anhydrous DMSO (32 mL) at 50 °C.
- Triethylamine ($Et_3N$, 22.30 μL, 0.16 mmol) was added to the solution.
- Finally, CMMC-NHS (53.01 mg, 0.16 mmol) dissolved in 1 mL of anhydrous DMSO, was added to the mixture.
- After 12 h stirring at 50 °C in the dark, the reaction mixture was diluted with 50 mM phosphate buffer, pH 7.4 (PBS, 165 mL).
- The solution was dialyzed with a Spectra/Por dialysis membrane (MW cutoff = 12-14 kDa). The dialysis was performed for 30 h using a 9:1 (v/v) 50 mM PBS, pH 7.4/DMSO mixture as releasing medium and for 6 h using only water.
- Finally, the solution was freeze-dried, and the CMMC-Gel conjugate was obtained as yellow solid (yield: 0.98 g, 95%).

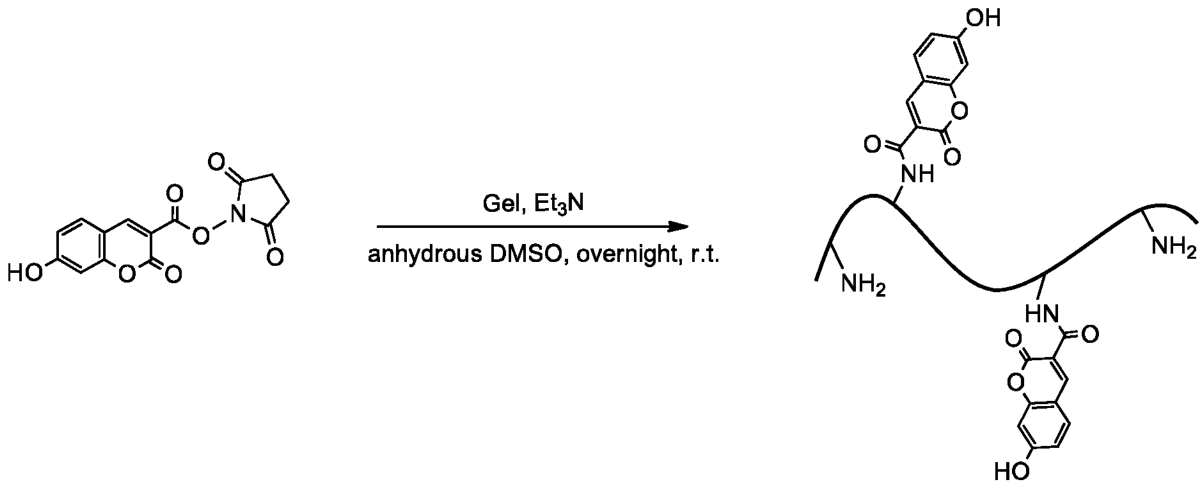


Schematic representation of the step required to conjugate HCCA-NSE to Gelatin (Gel) backbone. Conjugation of HCCA-NSE to Gel in anhydrous DMSO added of thriethylamine ($Et_3N$) to obtain HCC-Gel. The reaction was performed overnight at room temperature (r.t.).

### 2. CHARACTERIZATION OF CMMC-Gel

## 2.1 MATERIALS AND EQUIPMENTS

Sodium chloride (NaCl), phosphate buffered saline w/o $Ca^{2+}$ and $Mg^{2+}$ (PBS), boric acid, bicinchoninic acid, copper sulfate solution, 2,4,6-trinitrobenzensulfonic acid (TNBS), Glycylglycine (Gly-Gly), Gelatin from porcine skin (Gel, type A, 300 bloom, cell culture tested) and DMSO were purchased from Sigma-Aldrich (St. Louis MO, USA). Salts and buffers were purchased from Fluka Analytical (Buchs SG, Switzerland) and Sigma-Aldrich.

The size exclusion chromatography was performed using an AS-1555 HPLC system (Jasco, Tokyo, Japan) equipped with a PU-2089 Plus pump, a UV-2077 Plus detector and a Zorbax GF-250 column (4 μm, 4.6 x 250 mm, Agilent Technologies, Santa Clara, CA, USA).

Spectrophotometric assays were performed with an Evolution 201 UV-Vis spectrophotometer (Thermo Fisher Scientific, Waltham, MA, USA) in the wavelength range of 250-450 nm.

## 2.2 CHARACTERIZATION PROCEDURE

The CMMC-Gel conjugate was characterized by UV-Vis spectroscopy, size exclusion chromatography (SEC) and spectrophotometric assays to quantify the conjugation yield.

### UV-Vis spectroscopy

- CMMC-Gel was dissolved at 10 mg/ml in phosphate buffer (PBS), pH 7.4 and diluted 1:100 in the same buffer.
- The absorption spectrum was recorded was recorded with an UV-Vis spectrophotometer in the wavelength range of 250-450 nm.

### Size exclusion chromatography

- CMMC-Gel conjugate was dissolved in PBS, pH 7.4 at 0.4 mg/mL.
- Unconjugated CMMC (used as control) was dissolved in DMSO at 5.0 mg/mL and diluted 1:1000 in PBS, pH 7.4.
- The CMMC-Gel and CMMC samples were centrifuged at 14000 rpm for 5 min before injection.
- The analysis was performed using an AS-1555 HPLC system eluted with 63 mM phosphate buffer, 150 mM sodium chloride, pH 7.4. The flow rate was set at 0.3 mL/min and the UV-Vis detector at 320 nm for coumarin detection.

### Conjugation yield quantification

The amount of CMMC in CMMC-Gel sample was quantified by using a spectrophotometric assay:

- The absorbance of the CMMC-Gel sample was measured at 320 nm in 0.1 M phospate buffer pH 7.8.
- The absorbance measured was referred to a calibration curve obtained with standard solutions of CMMC in 0.1 M phospate buffer pH 7.8, in the concentration range of 0 - 64 μM.

For gelatin quantification in the conjugate, BCA protein assay was used (Sigma-Aldrich).

- 200 μL of the working reagent (bicinchoninic acid/copper sulfate solution 50:1) were added to 50 μL of a 0.80 mg/mL CMMC-Gel solution in PBS.
- After 30 min at 37 °C, the absorbance of the solution was measured at 562 nm.
- The absorbance measured was referred to a calibration curve of Gelatin obtained in the concentration range of 0 - 12.50 μM, using the same procedure described above.

The conjugation yield (reported as %) of CMMC-Gel was calculated by the [CMMC]/[gelatin] molar ratio compared to the maximum theoretical molar ratio ([CMMC]/[Gel] of 26:1). The gelatin conjugation yield was estimated to be 15%.

For the quantification of the maximum theoretical molar ratio, the content of primary amino groups of gelatin was determined by 2,4,6-trinitrobenzensulfonic acid (TNBS) assay.

- 160 μL of a 15.74 μM Gel solution in borate buffer (0.1 M, pH 9.3) were added to 810 μL of the same buffer followed by

30 μL of TNBS (5% w/v in Milli-Q water).

- After 30 min, the absorbance was measured at 420 nm.
- The amount of free amino groups was assessed using a calibration curve obtained with Glycylglycine, using the same procedure.

**3. SYNTHESIS OF 7-HYDROXYCOUMARIN-3-CARBOXAMIDE-GELATIN (HCC-Gel)**

**3.1 MATERIALS AND EQUIPMENTS**7-hydroxycoumarin-3-carboxylic acid (HCCA) was purchased from TCI Chemicals (Tokyo, Japan). Gelatin from porcine skin (Gel, type A, 300 bloom, cell culture tested), N-hydroxysuccinimide (NHS), N,N'-dicyclohexylcarbodiimide (DCC), triethylamine (TEA), dimethylsulfoxide (DMSO) and anhydrous DMSO, diethyl ether, phosphate buffered saline w/o $Ca^{2+}$ and $Mg^{2+}$ (PBS) were purchased from Sigma-Aldrich (St. Louis MO, USA). Water for the preparation of all solutions was "ultrapure" water (Milli-Q-qrade, 0.06 μSiemens cm-1) produced with a Millipore Milli-Q purification system (MA, USA). Salts and buffers were purchased from Fluka Analytical (Buchs SG, Switzerland) and Sigma-Aldrich (St. Louis, MO, USA). Dialysis was performed with Spectra/Por dialysis membranes with a MW cutoff of 12-14 kDa (Spectrum Labs, San Francisco, CA, USA).

**3.2 SYNTHESIS PROTOCOL**

The synthesis of 7-hydroxycoumarin-3-carboxamide-Gelatin (HCC-Gel) was performed into two steps: the activation of the carboxylic group of 7-hydroxycumarin-3-carboxylic acid and the further conjugation to Gelatin.

**STEP 1: Activation of the carboxylic group of 7-hydroxycumarin-3-carboxylic acid**

- 7-Hydroxycumarin-3-carboxylic acid (HCCA, 50.00 mg, 0.24 mmol) was dissolved in anhydrous dimethyl sulfoxide (DMSO, 1 mL) in a glass bottom flask, previously put in the oven overnight to let it anhydrous.
- N-hydroxysuccinimide (NHS, 55.83 mg, 0.49 mmol) was added to the solution.
- After 10 min, N,N'-dicyclohexylcarbodiimide (DCC, 100.09 mg, 0.49 mmol) was added.
- The reaction mixture was stirred overnight in the dark at room temperature.
- The mixture was filtered with gooch filter (porosity 4) under vacuum to remove the insoluble dicyclohexylurea.
- The solution was precipitated dropwise in diethyl ether (45 mL) under vigorous stirring and centrifuged at 4000 rpm.
- The precipitate was rinsed with diethyl ether (30 mL × 3 times) and finally desiccated under vacuum. The product 7-hydroxycoumarin-3-carboxylic acid N-succinimidyl ester (HCCA-NHS) was obtained as yellow solid.

**STEP 2: Conjugation of 7-hydroxycoumarin-3-carboxylic acid N-succinimidyl ester to Gelatin**

- Gelatin (Gel, 1.0 g, 12.5 μmol) was dissolved in anhydrous DMSO (32 mL) at 50 °C.
- Triethylamine ($Et_3N$, 22.30 μL, 0.16 mmol) was added to the solution.
- Finally, HCCA-NHS (48.52 mg, 0.16 mmol) dissolved in 1 mL of anhydrous DMSO, was added to the mixture.
- After 12 h stirring at 50 °C in the dark, the reaction mixture was diluted with 50 mM phosphate buffer, pH 7.4 (PBS, 165 mL).
- The solution was dialyzed with a Spectra/Por dialysis membrane (MW cutoff = 12-14 kDa). The dialysis was performed for 30 h using a 9:1 (v/v) 50 mM PBS, pH 7.4/DMSO mixture as releasing medium and for 6 h using only water.
- Finally, the solution was freeze-dried and the HCC-Gel conjugate was obtained as yellow solid (yield: 0.99 g, 96%).

**4. CHARACTERIZATION OF HCC-Gel**

**4.1 MATERIALS AND EQUIPMENTS**

Sodium chloride (NaCl), phosphate buffered saline w/o $Ca^{2+}$ and $Mg^{2+}$ (PBS), boric acid, bicinchoninic acid, copper sulfate solution, 2,4,6-trinitrobenzensulfonic acid (TNBS), Glycylglycine (Gly-Gly), Gelatin from porcine skin (Gel, type A, 300 bloom, cell culture tested) and DMSO were purchased from Sigma-Aldrich (St. Louis MO, USA). Salts and buffers were purchased from Fluka Analytical (Buchs SG, Switzerland) and Sigma-Aldrich.

The size exclusion chromatography was performed using an AS-1555 HPLC system (Jasco, Tokyo, Japan) equipped with a PU-2089 Plus pump, a UV-2077 Plus detector and a Zorbax GF-250 column (4 μm, 4.6 x 250 mm, Agilent

Technologies, Santa Clara, CA, USA).

Spectrophotometric assays were performed with an Evolution 201 UV-Vis spectrophotometer (Thermo Fisher Scientific, Waltham, MA, USA).

## 4.2 CHARACTERIZATION PROCEDURE

The HCC-Gel conjugate was characterized by UV-Vis spectroscopy, size exclusion chromatography (SEC) and spectrophotometric assays to quantify the conjugation yield.

### UV-Vis spectroscopy

- CMMC-Gel was dissolved at 10 mg/ml in phosphate buffer (PBS), pH 7.4 and diluted 1:100 in the same buffer.
- The absorption spectrum was recorded was recorded with an UV-Vis spectrophotometer in the wavelength range of 250-450 nm

### Size exclusion chromatography

- HCC-Gel conjugate was dissolved in PBS, pH 7.4 at 0.4 mg/mL.
- Unconjugated HCCA-NHS (used as control) was dissolved in DMSO at 5.0 mg/mL and diluted 1:1000 in PBS, pH 7.4.
- The HCC-Gel and HCCA-NHS samples were centrifuged at 14000 rpm for 5 min before injection.
- The analysis was performed using an AS-1555 HPLC system eluted with 63 mM phosphate buffer, 150 mM sodium chloride (NaCl), pH 7.4. The flow rate was set at 0.3 mL/min and the UV-Vis detector at 400 nm for coumarin detection.

### Conjugation yield quantification

The amount of HCCA in HCC-Gel sample was quantified by using a spectrophotometric assay:

- The absorbance of the HCC-Gel sample was measured at 410 nm in 0.1 M borate buffer, pH 8.3.
- The absorbance measured was referred to a calibration curve obtained with standard solutions of HCCA-NHS in 0.1 M borate buffer pH 8.3, in the concentration range of 0 - 33 μM.

For gelatin quantification in the conjugate, BCA protein assay was used:

- 200 μL of the working reagent (bicinconinic acid/copper sulfate solution 50:1) were added to 50 μL of a 0.80 mg/mL HCC-Gel solution in PBS.
- After 30 min at 37 °C, the absorbance of the solution was measured at 562 nm.
- The absorbance measured was referred to a calibration curve of Gelatin obtained in the concentration range of 0 - 12.50 μM, using the same procedure described above.

The conjugation yield (reported as %) of HCC-Gel was calculated by the [HCCA]/[gelatin] molar ratio compared to the maximum theoretical molar ratio ([HCCA]/[Gel] of 26:1). The gelatin conjugation yield was estimated to be 20%.

For the quantification of the maximum theoretical molar ratio, the content of primary amino groups of Gelatin was determined by 2,4,6-trinitrobenzensulfonic acid (TNBS) assay.

- 160 μL of a 15.74 μM Gel solution in borate buffer (0.1 M, pH 9.3) were added to 810 μL of the same buffer followed by 30 μL of TNBS (5% w/v in Milli-Q water).
- After 30 min, the absorbance was measured at 420 nm.
- The amount of free amino groups was assessed using a calibration curve obtained with Glycylglycine, using the same procedure.

## 5. SYNTHESIS OF 7-(CARBOXYMETHOXY)-4-METHYLCOUMARIN-PEG POLYMERS (CMMC-PEG)

## 5.1 MATERIALS AND EQUIPMENTS

10 kDa 4-arm PEG amine pentaerythritol core (4arm PEG-$NH_2$), 20 kDa 8-arm PEG amine tripentaerythritol core (8arm PEG-$NH_2$) were obtained from Jenkem Technology (Allen, TX, USA). 7-(carboxymethoxy)-4-methylcoumarin (CMMC), N-hydroxysuccinimide (NHS), N,N'-dicyclohexylcarbodiimide (DCC), triethylamine (TEA), dimethylsulfoxide (DMSO) and anhydrous DMSO, diethyl ether were purchased from Sigma-Aldrich (St. Louis MO, USA). Water for the preparation of all solutions was "ultrapure" water (Milli-Q-grade, 0.06 μSiemens $cm^{-1}$) produced with a Millipore Milli-Q purification system (MA, USA). Dialysis was performed with Spectra/Por dialysis membranes with a MW cutoff of 3.5 kDa for CMMC-4arm PEG and 12-14 kDa for CMMC-8arm PEG (Spectrum Labs, San Francisco, CA, USA).

(Scheme 2)

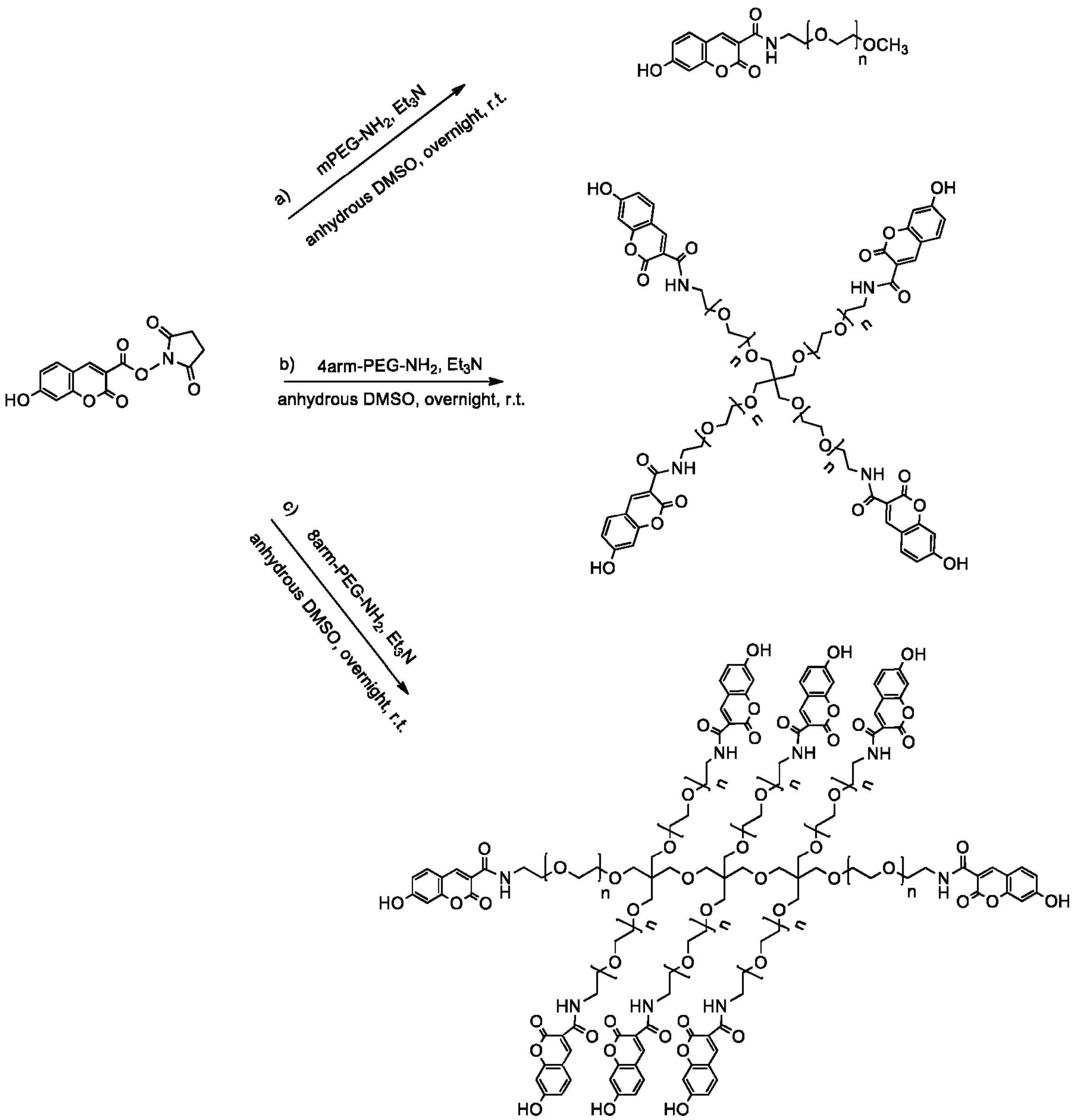


Schematic representation of the steps required to conjugate HCCA-NSE to the linear or branched PEG-backbones. Conjugation of HCCA-NSE to (a) mPEG-$NH_2$, (b) 4arm-PEG-$NH_2$ and (c) 8arm-PEG-$NH_2$ in anhydrous DMSO added of thriethylamine ($Et_3N$) to obtain linear HCC-PEG, HCC-4arm PEG and HCC-8arm PEG, respectively.

### 5.2 SYNTHESIS PROCEDURE

The synthesis of 7-(carboxymethoxy)-4-methylcoumarin-PEG polymers (CMMC-4arm PEG and CMMC-8arm PEG) was performed into two steps: the activation of the carboxylic group of 7-(carboxymethoxy)-4-methylcoumarin and the further conjugation to 4arm PEG-$NH_2$ and 8arm PEG-$NH_2$.

**STEP 1: Activation of the carboxylic group of 7-(carboxymethoxy)-4-methylcoumarin**

- 7-(carboxymethoxy)-4-methylcoumarin (CMMC, 1.0 g, 4.27 mmol) was dissolved in anhydrous dimethyl sulfoxide (DMSO, 20 mL) in a glass bottom flask, previously put in the oven overnight to let it anhydrous.
- N-hydroxysuccinimide (NHS, 0.98 g, 8.54 mmol) was added to the solution.
- After 10 min, N,N'-dicyclohexylcarbodiimide (DCC, 1.76 g, 8.54 mmol) was added.
- The reaction mixture was stirred overnight in the dark at room temperature.
- The mixture was filtered with gooch filter (porosity 4) under vacuum to remove the insoluble dicyclohexylurea.
- The solution was precipitated dropwise in diethyl ether (600 mL) and centrifuged at 4000 rpm.
- The precipitate was rinsed with diethyl ether (30 mL × 3 times) and finally desiccated under vacuum. The product 7-(carboxymethoxy)-4-methylcoumarin N-succinimidyl ester (CMMC-NHS) was obtained as white solid.

**STEP 2: Conjugation of 7-(carboxymethoxy)-4-methylcoumarin N-succinimidyl ester to branched PEG**

- 10 kDa 4-arm PEG amine pentaerythritol core (4arm PEG-$NH_2$, 1.0 g, 0.10 mmol) and 20 kDa 8-arm PEG amine tripentaerythritol core (8arm PEG-$NH_2$, 1.0 g, 0.05 mmol) were dissolved in anhydrous DMSO (10 mL each).
- The 4arm PEG-$NH_2$ solution was added of triethylamine (55.75 μL, 0.40 mmol) and CMMC-NHS (0.27 g, 0.80 mmol). The 8arm PEG-$NH_2$ solution was added of triethylamine (55.75 μL, 0.40 mmol) and CMMC-NHS (0.46 g, 1.40 mmol).
- After 12 h stirring at room temperature in the dark, the reaction mixtures were added dropwise to diethyl ether (300 mL) under vigorous stirring.
- The precipitates were recovered by centrifugation at 4000 rpm and dried under vacuum.
- The crude products were dissolved in 10 mL of a 9:1 (v/v) water/DMSO mixture.
- The solutions were dialyzed using Spectra/Por dialysis membranes (MW cutoff = 3.5 kDa, 12-14 kDa for CMMC-4arm PEG and CMMC-8arm PEG respectively). The dialysis was performed for 48 h using a 9:1 (v/v) water/DMSO mixture as releasing medium and for 4 h using only water.
- Finally, the solutions were freeze-dried, and the CMMC-conjugated PEG were obtained as white solids.

### 6. CHARACTERIZATION OF CMMC-PEG CONJUGATES

### 6.1 MATERIALS AND EQUIPMENTS

Phosphate buffered saline w/o $Ca^{2+}$ and $Mg^{2+}$ (PBS), deuterated chloroform and DMSO were purchased from Sigma-Aldrich (St. Louis MO, USA). Two kDa methoxy PEG amine (mPEG-$NH_2$) was obtained from Jenkem Technology (Allen, TX, USA). Salts and buffers were purchased from Fluka Analytical (Buchs SG, Switzerland) and Sigma-Aldrich.

Spectrophotometric assays were performed with an Evolution 201 UV-Vis spectrophotometer (Thermo Fisher Scientific, Waltham, MA, USA) in the wavelength range of 250-450 nm.

$^1H$ NMR spectra were recorded in deuterated chloroform using an AVANCE III 400 Ultrashield spectrometer (Bruker, Billerica, MA, USA). NMR data were processed with MestreNova 6.2.0 Software (Mestrelab Research SL, Santiago de Compostela, Spain). The reversed phase high-performance liquid chromatographic (RP-HPLC) was performed on a Jupiter analytic column (C18, 5 μ, 300 Å, 250 x 4.6 mm, Phenomenex, Torrance, CA, USA)

### 6.2 CHARACTERIZATION PROCEDURE

The CMMC-PEG conjugates were characterized by UV-Vis spectroscopy, $^1H$ NMR spectroscopy and spectrophotometric assays to quantify the conjugation yield.

**UV-Vis spectroscopy**

- CMMC-4arm PEG and CMMC-8arm PEG were dissolved at 10 mg/ml in phosphate buffer (PBS), pH 7.4 and diluted 1:100 in the same buffer.
- The absorption spectra were recorded with an UV-Vis spectrophotometer in the wavelength range of 250-450 nm.

**$^1$H NMR spectroscopy**

The composition of CMMC-PEG polymers was assessed by identifying the characteristic peaks of PEG and CMMC units, as follows:

- CMMC-4arm PEG. $^1$H NMR ($CDCl_3$, 400 MHz), δ (ppm): 2.41 (d, 3H, Ar-C*H$_3$* of CMMC), 3.64 (bs, ~900H, -C*H$_2$*C*H$_2$*-O- of PEG chain), 4.56 (s, 2H, -NHCO-C*H$_2$*-O- of CMMC), 6.17 (s, 1H, =C*H* of CMMC), 6.85 (d, 1H, Ar-*H* of CMMC), 6.91 (dd, 1H, Ar-*H* of CMMC), 7.05 (bs, 1H, -CON*H*-PEG), 7.55 (d, 1H, Ar-*H* of CMMC).
- CMMC-8arm PEG. $^1$H NMR ($CDCl_3$, 400 MHz), δ (ppm): 2.41 (d, 3H, Ar-C*H$_3$* of CMMC), 3.64 (bs, ~1800H, -C*H$_2$*C*H$_2$*-O- of PEG chain), 4.56 (s, 2H, -NHCO-C*H$_2$*-O- of CMMC), 6.17 (s, 1H, =C*H* of CMMC), 6.85 (d, 1H, Ar-*H* of CMMC), 6.91 (dd, 1H, Ar-*H* of CMMC), 7.05 (bs, 1H, -CON*H*-PEG), 7.55 (d, 1H, Ar-*H* of CMMC).

**Quantification of conjugation degree**

The amount of CMMC in CMMC-4arm PEG and CMMC-8arm PEG samples was quantified by using a spectrophotometric assay:

- The absorbance of the CMMC-4arm PEG and CMMC-8arm PEG solutions was measured at 320 nm in 0.1 M phosphate buffer, pH 7.8.
- The absorbances measured were referred to a calibration curve obtained with standard solutions of CMMC in 0.1 M phosphate buffer pH 7.8, in the concentration range of 0 - 64 μM.

For PEG quantification in CMMC-4arm PEG and CMMC-8arm PEG samples, iodine assay was used:

- each conjugate was dissolved in milli-Q water and diluted to 0.01 mg/mL.
- Barium chloride (5% w/v in 1 M HCl) and iodine solution (1.27 g of $I_2$ in a 2% KI solution in Milli-Q water) were added 1:4 to CMMC-4arm PEG and CMMC-8arm

PEG samples.

- After 15 min, absorbance was measured at 535 nm.
- The absorbance values were referred to a calibration curve of mPEG-$NH_2$ obtained with the same procedure.

The absence of free unconjugated CMMC in the samples was tested by RP-HPLC. The analysis was performed on a Jupiter column eluted with a linear gradient of water/acetonitrile containing 0.1 % (v/v) trifluoroacetic acid over 40 min. The flow rate was set at 1.0 mL/min and the UV-Vis detector at 320 nm.

The conjugation yield (reported as %) of CMMC-4arm PEG and CMMC-8arm PEG was calculated by the [CMMC]/[PEG] molar ratio compared to the maximum theoretical molar ratio ([CMMC]/[PEG] of 4:1 for CMMC-4arm PEG and [CMMC]/[PEG] of 8:1 for CMMC-8arm PEG). The conjugation yield was estimated to be 94% for CMMC-4arm PEG, 92% for CMMC-8arm PEG.

## 7. SYNTHESIS OF 7-HYDROXYCOUMARIN-3-CARBOXAMIDE-PEG POLYMERS (HCC-PEG)

### 7.1 MATERIALS AND EQUIPMENTS

7-hydroxycoumarin-3-carboxylic acid (HCCA) was purchased from TCI Chemicals (Tokyo, Japan). 10 kDa 4-arm PEG amine pentaerythritol core (4arm PEG-$NH_2$), 20 kDa 8-arm PEG amine tripentaerythritol core (8arm PEG-$NH_2$) were obtained from Jenkem Technology (Allen, TX, USA). N-hydroxysuccinimide (NHS), N,N'-dicyclohexylcarbodiimide (DCC), triethylamine (TEA), dimethylsulfoxide (DMSO) and anhydrous DMSO, diethyl ether were purchased from Sigma-Aldrich (St. Louis MO, USA). Water for the preparation of all solutions was "ultrapure" water (Milli-Q-grade, 0.06 μSiemens

$cm^{-1}$) produced with a Millipore Milli-Q purification system (MA, USA). Dialysis was performed with Spectra/Por dialysis membranes with a MW cutoff of 3.5 kDa for CMMC-4arm PEG and 12-14 kDa for CMMC-8arm PEG (Spectrum Labs, San Francisco, CA, USA).

## 7.2 SYNTHESIS PROCEDURE

The synthesis of 7-hydroxycoumarin-3-carboxamide-PEG polymers (HCC-4arm PEG and HCC-8arm PEG) was performed in two steps: the activation of the carboxylic group of 7-hydroxycumarin-3-carboxylic acid and the further conjugation to 4arm PEG-$NH_2$ and 8arm PEG-$NH_2$.

### STEP 1: Activation of the carboxylic group of 7-hydroxycumarin-3-carboxylic acid

- 7-Hydroxycumarin-3-carboxylic acid (HCCA, 1.0 g, 4.85 mmol) was dissolved in anhydrous dimethyl sulfoxide (DMSO, 20 mL) in a glass bottom flask, previously put in the oven overnight to let it anhydrous.
- N-hydroxysuccinimide (NHS, 1.12 g, 9.70 mmol) was added to the solution.
- After 10 min, N,N'-dicyclohexylcarbodiimide (DCC, 2.0 g, 9.70 mmol) was added.
- The reaction mixture was stirred overnight in the dark at room temperature.
- The mixture was filtered with gooch filter (porosity 4) under vacuum to remove the insoluble dicyclohexylurea.
- The solution was precipitated dropwise in diethyl ether (600 mL) and centrifuged at 4000 rpm.
- The precipitate was rinsed with diethyl ether (30 mL × 3 times) and finally desiccated under vacuum. The product 7-hydroxycoumarin-3-carboxylic acid N-succinimidyl ester (HCCA-NHS) was obtained as yellow solid.

### STEP 2: Conjugation of 7-hydroxycoumarin-3-carboxylic acid N-succinimidyl ester to branched PEG

- 10 kDa 4-arm PEG amine pentaerythritol core (4arm PEG-$NH_2$, 1.0 g, 0.10 mmol) and 20 kDa 8-arm PEG amine tripentaerythritol core (8arm PEG-$NH_2$, 1.0 g, 0.05 mmol) were dissolved in anhydrous DMSO (10 mL each).
- The 4arm PEG-$NH_2$ solution was added of triethylamine (55.75 μL, 0.40 mmol) and HCCA-NHS (0.24 g, 0.80 mmol). The 8arm PEG-$NH_2$ solution was added of triethylamine (55.75 uL, 0.40 mmol) and HCCA-NHS (0.42 g, 1.40 mmol).
- After 12 h stirring at room temperature in the dark, the reaction mixtures were added dropwise to diethyl ether (300 mL) under vigorous stirring.
- The precipitates were recovered by centrifugation at 4000 rpm and dried under vacuum.
- The crude products were dissolved in 10 mL of a 9:1 (v/v) water/DMSO mixture
- The solutions were dialyzed using Spectra/Por dialysis membranes (MW cutoff = 3.5 kDa, 12-14 kDa for HCC-4arm PEG and HCC-8arm PEG respectively). The dialysis was performed for 48 h using a 9:1 (v/v) water/DMSO mixture as releasing medium and for 4 h using only water.
- Finally, the solutions were freeze-dried, and the HCC-conjugated polymers were obtained as yellow solids (HCC-4arm PEG yield: 1.05 g, 91%; HCC-8arm PEG yield: 0.99 g, 93%).

## 8. CHARACTERIZATION OF HCC-PEG CONJUGATES

## 8.1 MATERIALS AND EQUIPMENTS

Phosphate buffered saline w/o $Ca^{2+}$ and $Mg^{2+}$ (PBS), deuterated chloroform and DMSO were purchased from Sigma-Aldrich (St. Louis MO, USA). Two kDa methoxy PEG amine (mPEG-$NH_2$) was obtained from Jenkem Technology (Allen, TX, USA). Salts and buffers were purchased from Fluka Analytical (Buchs SG, Switzerland) and Sigma-Aldrich.

Spectrophotometric assays were performed with an Evolution 201 UV-Vis spectrophotometer (Thermo Fisher Scientific, Waltham, MA, USA) in the wavelength range of 250-450 nm.

$^1H$ NMR spectra were recorded in deuterated chloroform using an AVANCE III 400 Ultrashield spectrometer (Bruker, Billerica, MA, USA). NMR data were processed with MestreNova 6.2.0 Software (Mestrelab Research SL, Santiago de Compostela, Spain). The reversed phase high-performance liquid chromatographic (RP-HPLC) was performed on a Jupiter analytic column (C18, 5 μ, 300 Å, 250 x 4.6 mm, Phenomenex, Torrance, CA, USA).

FT-IR spectra were recorded on a SPECTRUM BXII FTIR (Perkin Elmer, Norwoworlk, CT, USA).

## 8.2 CHARACTERIZATION PROCEDURE

The HCC-PEG conjugates were characterized by UV-Vis spectroscopy, $^{1}H$ NMR spectroscopy, FT-IR analysis and spectrophotometric assays to quantify the conjugation yield.

### UV-Vis spectroscopy

- HCC-4arm PEG and HCC-8arm PEG were dissolved at 10 mg/ml in phosphate buffer (PBS), pH 7.4 and diluted 1:100 in the same buffer.
- The absorption spectra were recorded with an UV-Vis spectrophotometer in the wavelength range of 250-450 nm.

### $^{1}H$ NMR spectroscopy

The composition of HCC-conjugated polymers was assessed by identifying the characteristic peaks of PEG and HCC units, as follows:

- HCC-4arm PEG. $^{1}H$ NMR ($CDCl_3$, 400 MHz), δ (ppm): 3.64 (bs, ~900H, - C*H*$_2$C*H*$_2$-O- of PEG chain), 6.88-6.90 (m, 2H, Ar-*H* of HCC), 7.51 (d, 1H, Ar-*H* of HCC), 8.79 (s, 1H, =C*H* of HCC), 9.03 (bs, 1H, -CON*H*-PEG).
- HCC-8arm PEG. $^{1}H$ NMR ($CDCl_3$, 400 MHz), δ (ppm): 3.64 (bs, ~1800H, - C*H*$_2$C*H*$_2$O- of PEG chain), 6.88-6.90 (m, 2H, Ar-*H* of HCC), 7.51 (d, 1H, Ar-*H* of HCC), 8.79 (s, 1*H*, =C*H* of HCC), 9.03 (bs, 1H, -CON*H*-PEG).

### FT-IR analysis

- HCC-4arm PEG and HCC-8arm PEG samples were thoroughly grounded with potassium bromide and pellets were prepared by compression.
- FT-IR spectra were recorded with a resolution of 4 $cm^{-1}$.
- 4armPEG-$NH_2$ was used as control and treated as the other samples.
  - 4armPEG-$NH_2$. FT-IR: v 3413, 2887, 1467, 1360, 1343, 1280, 1242, 1149, 1113, 1060, 963, 947, 842, 529 $cm^{-1}$.
  - HCC-4arm PEG. FT-IR: v 3448, 2885, 1707, 1541, 1466, 1360, 1344, 1281, 1242, 1147, 1113, 1061, 963, 842, 520 $cm^{-1}$.
  - HCC-8arm PEG. FT-IR: v 3455, 2885, 1708, 1542, 1466, 1360, 1344, 1281, 1242, 1147, 1113, 963, 842, 519 $cm^{-1}$.

### Quantification of conjugation degree

The amount of HCCA in HCC-4arm PEG and HCC-8arm PEG samples was quantified by using a spectrophotometric assay:

- The absorbance of the HCC-4arm PEG and HCC-8arm PEG solutions was measured at 410 nm in 0.1 M borate buffer, pH 8.3.
- The absorbance measured was referred to a calibration curve obtained with standard solutions of HCCA-NHS in 0.1 M borate buffer pH 8.3, in the concentration range of 0 - 33 μM.

For PEG quantification in HCC-4arm PEG and HCC-8arm PEG samples, iodine assay was used:

- each conjugate was dissolved in milli-Q water and diluted to 0.01 mg/mL.
- Barium chloride (5% w/v in 1 M HCl) and iodine solution (1.27 g of $I_2$ in a 2% KI solution in Milli-Q water) were added 1:4 to HCC-4arm PEG and HCC-8arm PEG samples.
- After 15 min, absorbance was measured at 535 nm
- The absorbance values were referred to a calibration curve of mPEG-$NH_2$ obtained with the same procedure.

The absence of free unconjugated HCCA in the samples was tested by RP-HPLC. The analysis was performed on a Jupiter column eluted with a linear gradient of water/acetonitrile containing 0.1 % (v/v) trifluoroacetic acid over 40 min. The flow rate was set at 1.0 mL/min and the UV-Vis detector at 360 nm.

The conjugation yield (reported as %) of HCC-4arm PEG and HCC-8arm PEG was calculated by the [HCCA]/[-PEG] molar ratio compared to the maximum theoretical molar ratio ([HCCA]/[PEG] of 4:1 for HCC-4arm PEG and [HCCA]/[PEG] of 8:1 for HCC-8arm PEG). The conjugation yield was estimated to be 95% for HCC-4arm PEG, 90% for HCC-8arm PEG.

### 9. Characterization of HCCA [2+2] cycloaddition reaction by $^1H$ NMR spectroscopy and electrospray ionization time-of-flight mass spectrometry (ESI-TOF MS)

HCCA in solution was used to confirm the [2+2] cycloaddition reaction of HCCA-conjugated polymers.

A 60 mg/mL solution of HCCA in deuterated DMSO (80 μL) was irradiated with a 365 nm lamp (BlueWave 50 UV light lamp). The solution was irradiated at 1 cm distance for 1 h and analyzed before and after irradiation by $^1H$ NMR spectroscopy with a Bruker DMX 600 spectrometer (Billerica, MA, USA) and by ESI-TOF-MS with an Applied Biosystems Mariner ESI-TOF spectrometer (Monza, MI, Italy). HCCA monomers and dimers were identified by the characteristic signals in the $^1H$ NMR spectra, as follows:

- HCCA solution before irradiation. $^1H$ NMR (DMSO-$d_6$, 600 MHz), δ (ppm): 6.73 (d, 1H, Ar-*H* of HCCA monomer), 6.84 (dd, 1H, Ar-*H* of HCCA monomer), 7.74 (d, 1H, Ar-*H* of HCCA monomer), 8.68 (s, 1H, =C*H* of HCCA monomer), 11.07 (bs, 1H, O*H* of HCCA monomer), 12.86 (bs, 1H, COO*H* of HCCA monomer).
- HCCA solution after UV irradiation. $^1H$ NMR (DMSO-$d_6$, 600 MHz), δ (ppm): 4.63 (s, 1H, cyclobutyl-C*H* of HCCA dimer), 6.38 (d, 1H, Ar-*H* of HCCA dimer), 6.62 (dd, 1H, Ar-*H* of HCCA dimer), 6.73 (d, 1H, Ar-*H* of HCCA monomer), 6.84 (dd, 1H, Ar-*H* of HCCA monomer), 7.03 ( d, 1H, Ar-*H* of HCCA dimer), 7.74 (d, 1H, Ar-*H* of HCCA monomer), 8.68 (s, 1H, =C*H* of HCCA monomer), 11.07 (s, 1H, O*H* of HCCA monomer), 12.86 (bs, 1H, COO*H* of HCCA monomer).

Before and after irradiation, the HCCA solution in DMSO underwent ESI-TOF analysis.

- HCCA solution before irradiation. ESI-TOF: m/z $[M - H]^-$ calcd for HCCA monomer 205.02; found 205.02.
- HCCA solution after UV irradiation. ESI-TOF: m/z $[M - H]^-$ calcd for HCCA monomer 205.02; found 205.02. m/z $[M - H]^-$ calcd for HCCA dimer 411.30; found 409.02, consistent with the formation of HCCA dimer undergoing a keto-enol tautomerism induced by the gas-phase ionization.

### 10. Transmission electron microscopy (TEM) analysis of HCC-PEG self-assembled nanostructures

A 20 mg/mL HCC-PEG solution in milli-Q water was diluited to 0.5 mg/mL and placed on a carbon coated copper grid. After the removal of the volume excess with a filter paper, the samples were negatively stained with uranyl acetate (2% in distilled water) for 5 minutes at room temperature and analysed using a Tecnai G2 transmission electron microscope (FEI, Oregon, USA). A solution of mPEG-$NH_2$ was used as negative control and treated with the same procedure.

### 11. Analysis of HCC-PEG after single-photon or two-photon (2P) absorption.

For single photon excitation, HCC-PEG was dissolved in PBS, pH 7.4 at 20 mg/mL. The solution (20 μL) was irradiated with a BlueWave 50 UV light lamp (Dymax Corporation, Torrington, CT, USA) at 365 nm for 1, 2, 3, 4, 5, 6, 7, 8, 9, 10, 20 and 30 minutes. The dimerization degree was calculated by measuring the absorbance of the samples at 403 nm before and after irradiation using the following equation:

$$Dimerization\ (\%) = (1 - \frac{A_t}{A_0}) \times 100$$

where $A_0$ is the absorbance of the sample before irradiation, while At is the absorbance of the samples after irradiation, both measured at 403 nm, i.e. the wavelength of the maximum absorption peak.

For 2P absorption, HCC-PEG was patterned onto HCCA-functionalised glass coverslips following the highly spatially localized 2P excitation given by a multiphoton laser-scanning microscope. HCCA-functionalization on coverslip was achieved as follows. Coverslips were cleaned and extensively rinsed with deionized water and finally oxidized by the PDC-002-CE Plasma Cleaner (Harrick Plasma, Ithaca, NY, USA) for 30 seconds. The glass slide was incubated in a 2% (v/v) APTES solution in absolute ethanol (300 μL) under gently stirring. After 6 minutes, the substrate was washed three times with ethanol to remove unbound APTES and finally dried at 110° C for 2 hours. The glass slide was treated with 300 μL of a 5 mg/mL solution of HCCA-NHS in anhydrous DMSO added with a catalytic amount of TEA. The reaction mixture was maintained under stirring overnight at room temperature in the dark. Finally, coverslip was washed three times with

DMSO, milli-Q water, absolute ethanol and dried under a nitrogen stream. To assess each functionalization step, the glass slide was characterized by static contact angle analysis using a DSA 30 Drop Shape Analysis System (KRUSS GmbH, Hamburg, Germany). For 2P absorption, HCC-PEG solution (1 mg/mL in PBS) was drop-cast onto the coverslip previously functionalised with HCCA. 2P microscope was used to irradiate surfaces in contact with HCC-PEG solution and produce 2P cross-linked area of 850x850 $\mu m^2$, 425x425 $\mu m^2$ and 280x280 $\mu m^2$ (laser wavelength of 800 nm; laser power 0.7 mW measured before objective back aperture; writing time of 1.14 ms/line and rate of 1.72 frame/s; 4 frame per plane). After photo-exposition, samples were washed with PBS, fixed in 4% (w/v) paraformaldehyde (PFA, Sigma-Aldrich) in PBS for 7 min at room temperature and maintained at 4 °C in PBS, then processed for immunofluorescence analysis and confocal imaging acquisition.

**12. HCC-4 arm PEG, HCC-8 arm PEG and HCC-Gel 2P hydrogel polymerization.**

With the aim to provide glass surface HCCA-groups for covalent bonding HCC-branched polymers solutions were drop-cast onto glass coverslip previously functionalized with HCCA-as described in Section 10. HCC-4arm PEG and HCC-8arm PEG were dissolved in PBS (300 mg/mL) at room temperature under agitation. HCC-Gel was dissolved in PBS (100 mg/mL) under agitation at 60 °C for 10 min. When coverslips were used, a second coverslip was placed on top of the drop of HCC-polymeric solutions to limit water evaporation during the irradiation process. For crosslinking reaction analysis, HCC-Gel solution was prepared at the following concentrations, 100 mg/mL, 120 mg/mL, 140 mg/mL, 160 mg/mL, 180 mg/mL and 200 mg/mL. Photosensitive polymers were also mixed in a 50/50 ratio with Matrigel or loaded into pre-gelified Matrigel by incubating a drop of liquid solution onto the pre-gelified Matrigel at 37 °C. 2P hydrogel crosslinking was achieved by using Scientifica 2-Photon microscope. Laser power is measured before objective back aperture, corresponding to about 1% of the power on the sample plane. In a typical working condition, standard line scan velocity was 0.8 mm/ms, pixel dwell 2 $\mu$s and z spacing of 1 $\mu$m. The time required to polymerize a volume of 1 $mm^3$ is about 30 min. Other multiphoton microscopes (as Leica TCS SP8 MP or Zeiss Examiner.Z1 LSM800 confocal) has been used to photo-crosslink hydrogels.

**13. CMMC-4arm PEG and CMMC-Gel UV-mediated hydrogel crosslinking.**

For crosslinking reaction analysis, 5 $\mu$L CMMC-4 arm PEG (300 mg/mL) or CMMC-Gel (100 mg/mL) solution was located in a humid chamber at 3 cm from a 365nm UV LED Gen 2 Emitter LZ4-04UV00 (Led Engin) and irradiated for different minutes as reported. When CMMC-Gel solution was analysed at different concentrations (100 mg/mL, 120 mg/mL, 140 mg/mL, 160 mg/mL, 180 mg/mL and 200 mg/mL), 5 $\mu$L of solution was located in a humid chamber at 3 cm from a 365nm UV LED Gen 2 Emitter LZ4-04UV00 (Led Engin) and irradiated for 3 minutes.

**14. Atomic Force Measurement (AFM)**

After development in PBS, HCC-4arm PEG and HCC-Gel hydrogels (212 $\mu$m x 212 $\mu$m x 150 $\mu$m) or were maintained in PBS. For HCC-Gel/Matrigel 3D structure measurements, Matrigel was dissociated with cold PBS-EDTA solution (0.5mM EDTA, Invitrogen #AM9260G) under gentle agitation overnight at 4°C. For cell-laden HCC-Gel constructs, samples were prepared as reported above and maintained into PBS for further analysis. All the samples were analyzed by using an Atomic Force Microscope, mounted on an Inverted Optical Microscope (XEBio, Park Systems, Korea). This combination enabled positioning of the AFM tip on the area of interest of the samples. All force-displacement curves were collected using PPP-CONTSCR-10 pyramidal tips mounted on $Si_3N_4$ cantilevers with a nominal spring constant of 0.2N/m (NanoSensors, Neuchatel, Switzerland). Cantilever spring constants were calibrated by the manufacturer prior use. Indentations were performed at a rate of 0.2 $\mu$m/s. All AFM measurements were done in a fluid environment (PBS) at room temperature.

The Young's modulus was calculated applying a fit of the Hertz model to the force curve, assuming a Poisson ratio of 0.5. The moduli of at least three samples of each lot were calculated as an average over 3-5 sites per sample.

**15. Cell cultures and availability assay**

HUVEC were transduced with a lentiviral vector encoding for eGFP (3) and cultured in Endothelial Growth Media (EGM, Lonza). For 2D culture on hydrogel, 20 x $10^4$ cells were seeded onto HCC-Gel structures (fabrication details reported in Table S4), located in a 12 multiwell culture dish and cultured for 24 h at 37 °C and 5% CO2 in cell incubator. After culture, cells were fixed in 4% (w/v) paraformaldehyde (PFA, Sigma-Aldrich) in PBS for 7 min at room temperature and maintained at 4 °C in PBS until use. Neural stem cell (NSC)/neural progenitor cell (NPC) culture were derived from human embryonic stem cells (hESCs). Briefly, hESCs were detached using Dispase and plated in low-adhesion dishes to generate embryoid bodies (EB) in the presence of two small molecules, SB-431542 (10$\mu$M) and Dorsomorphin (DSO,

3μM), which inhibit TGFβ and BMP respectively, and Rock-inhibitor (10μM, Miltenyi Biotec). After 5 days in culture, EBs were collected and plated on Matrigel coated dishes in EB medium supplemented with DSO (1μM) and Fgf-2 (10 ng/ml) to obtain neural rosette. On day 6 EB medium was changed to N2 medium with DSO and Fgf-2 and changed daily until neural rosettes were formed. On day 12 after seeding, neural rosettes were picked and grown in suspension for 2 days in N2 medium with Fgf-2 (20 ng/ml). Finally, on day 14, floating rosettes were collected, digested with 0,025% Trypsin-EDTA and plated on tissue culture petri dish coated with poly-ornithine/laminin in N2 medium with Fgf-2 (10 ng/ml), Egf (10 ng/ml) and B27 supplement (1:1000) to obtain a NSC/NPC line. NSCs/NPCs were split with 0,025% Trypsin-EDTA every 3 days at 1:2/1:3 ratio. EB medium: DMEM/F-12 (Dulbecco's Modified Eagle Medium/Nutrient Mixture F-12, Thermofisher #11320-033), KSR 20% (Thermofisher), b-Mercaptoethanol 1:1000 (Gibco), Non-Essential Amino Acids (NEAA) 1:100 (Gibco), L-Glutamine 1:100 (Thermofisher Scientific, #25030024). N2 medium: DMEM/F-12 (Dulbecco's Modified Eagle Medium/Nutrient Mixture F-12, Thermofisher #11320-033), N-2 supplement 1% (Thermofisher) and D-glucose (Thermofisher, 350μl/100 ml). For 2D culture on hydrogel, 3.2 x $10^5$ cells were seeded onto HCC-Gel structures, located in a 12 multiwell culture dish and cultured for 3 days at 37 °C and 5% $CO_2$ in cell incubator. After culture, cells were fixed in 4% (w/v) paraformaldehyde (PFA, Sigma-Aldrich) in PBS for 7 min at room temperature and maintained at 4 °C in PBS until use.

Primary muscular fibroblasts (FBs) were isolated from wild-type or transgenic C57BL/6-(ACTB-EGFP)/J mice as previously described[64]. Upon 3 passages of expansion, cells were transduced with a lentiviral vector encoding for mCherry[63]. After 2 passages, mCherry$^+$FBs were purified through Fluorescence Assisted Cell Sorting (FACS) at the Flow Cytometry BD FACS Aria Illu, based on the epifluorescence signal. Only mCherry$^+$FBs were collected and further expanded. For 3D cell laden culture, $10^4$ cells were seeded into Matrigel, or Matrigel or CMMC-Gel exposed to UV light for 3 minutes at an irradiation distance of 3 millimeters, located in a 24 multiwell culture dish, cultured for 2 days at 37 °C and 5% $CO_2$ in cell incubator before performing cell viability assay as described above. For 2P cell-laden HCC-Gel 3D bioprinting, 4 x $10^4$ mCherry$^+$FBs or wild-type FBs were resuspended in 10 μL of HCC-Gel solution, drop cast on glass coverslip and processed. 2P-cell laden constructs were analyzed by imaging or AFM as described above.

Muscle stem cell-derived cells (MuSCs) were isolated from wild-type or transgenic C57BL/6-(ACTB-EGFP)/J mice as previously described. Cells were expanded for 1 passage at 37 °C in cell incubator, using proliferating medium composed by DMEM supplemented with 20% horse serum, 10% fetal bovine serum (Gibco), 1% chicken embryo extract (Sera Laboratories International), bFGF (25ng/μl, Invitrogen #PHG0264) and 1% Pen/Strep (Thermofisher). For 2P cell-laden 3D bioprinting, 2 x $10^4$ MuSCs were resuspended in 5 μL of HCC-Gel solution, drop cast on glass coverslip and processed. For 3D cell-laden culture, 1 x $10^4$ cells were seeded into Matrigel, or CMMC-Gel exposed to UV light for 3 minutes at an irradiation distance of 3 millimeters. Samples were located in a 24 multiwell culture dish, cultured for 2 days at 37 °C and 5% $CO_2$ in cell incubator before performing cell viability assay as described above. For 2P cell-laden HCC-Gel 3D bioprinting, 2 x $10^4$ MuSCs were resuspended in 10 μL of HCC-Gel solution, drop cast on glass coverslip and processed. Samples were cultured in proliferating media and analyzed 2 days after 3D bioprinting (cell directionality was quantified as reported above). For long term 3D culture, 2 x $10^4$ cells were seeded onto 3D elongated-shaped HCC-Gel 2P crosslinked hydrogels. After 5 days of culture in proliferating medium, differentiation medium (DMEM supplemented with 2% horse serum and 1% Pen/Strep) was used for the following 5 days of cell culture. After 10 days, samples were fixed in 4% (w/v) paraformaldehyde (PFA, Sigma-Aldrich) in PBS for 7 min at room temperature and maintained at 4 °C in PBS until use. Human small intestinal crypt stem cells were isolated from a small intestine pediatric biopsy following a well-established dissociation protocol. Isolated crypts were cultured in pure matrigel growth factor reduced (BD 354230) droplets and expanded in medium:

| Component | Stock conc. | Final conc. |
|---|---|---|
| Advanced DMEM F-12 (Thermo 12634) | - | To volume |
| HEPES (Thermo 15630080) | 1 M | 10 mM |
| Glutamax (Thermo 35050061) | 100 X | 2 mM |
| B-27 supplement minus vitamin A (Thermo 12587010) | 50 X | 1 X |
| n-acetylcysteine (Sigma A9165) | 500 mM | 1.25 mM |
| Pen/Strep (Thermo 15140122) | 100 % | 1% |
| Wnt-3A (Peprotech 315-20) | 50 μg/mL | 100 ng/mL |
| R-spondin 1 (Peprotech 120-38) | 100 μg/mL | 500 ng/mL |
| Noggin (R&D 6057-NG) | 100 μg/mL | 100 ng/mL |
| EGF (Thermo PMG8043) | 500 μg/mL | 50 ng/mL |

(continued)

| Component | Stock conc. | Final conc. |
|---|---|---|
| Gastrin (Sigma G9020) | 100 μM | 10 nM |
| GSK-3 inhibitor (CHIR 99021) (Tocris 4423) | 3 mM | 3 μM |
| TGFb inhibitor (A83-01) (Sigma SML0788) | 500 μM | 500 nM |
| P38 inhibitor (SB202190) (Sigma S7067) | 30 mM | 10 μM |
| Prostaglandin E2 (Cambridge cay14010) | 100 μM | 10 nM |

When indicated, small intestinal enteroids were transfected with lentiviral vector for Green Fluorescent Protein ($GFP^+$). Enteroids were passaged every 6-8 days by 5 min TrypLE (Thermo 12605010) dissociation and seeded in new Matrigel droplets. For the multi-photon experiments, hSIOs that were recently passaged (1-2 days) were washed thoroughly from the old Matrigel with cold AdDMEM+++. Cells were pelleted at 400 g and surnatant discarded. For HCC-Gel/Matrigel 50/50 experiments, HCC-Gel 10% solution was liquefied at 60 °C for 5 min and quickly mixed 1:1 to liquid matrigel at 4 °C to form a pre-gel with final concentration of 5% HCC-Gel and 50% Matrigel. Cell pellet was quickly resuspended in the pre-gel mixture and the droplets were allowed to solidify for 15 min at 37 °C, before proceeding to 2P crosslinking. Alternatively, for experiments in which HCC-Gel or HCC-PEG was loaded into gelified Matrigel, cells were pelleted at 400 g, replated in 5 ul drops in the well-plate and let the Matrigel polymerize at 37°C for 5 minutes, then 5 ul of HCC-solution was added onto the gelified Matrigel and incubated at 37°C for 10 minutes, before proceeding to 2P crosslinking. When loaded into gelified Matrigel, photo-sensitive polymers can be added at the desired time point of the organoid culture. After 2P printing, samples were cultured for 2, 3, 6 or 8 days in the above WENR media before being use for analysis. For basal/apical ratio and basal/apical major axis, measurements were performed on confocal fluorescence images stained for nuclei and actin by using ImageJ software (Feret's angle was quantified as reported above).

For cell viability assay, cells were passaged to 1:1 pre-gel solution and seeded in quadruplicates. Enteroids were expanded for 2 days and tested for combined gel cytocompatibility. Vitality assay was performed using LIVE/DEAD™ Viability/Cytotoxicity Kit, for mammalian cells (Thermo L3224), following supplier instructions. Briefly, organoids were washed with basal DMEM F-12 and incubated in basal medium with Hoechst (Thermo H139910 μg/mL), calcein-AM (3 μM) and ethidium homodimer-1 (3 μM). Cells in matrigel droplets were washed twice and analysed. For immunofluorescence analysis, matrigel droplets with embedded enteroids and two-photon printed geometries were fixed in 2% glutaraldehyde dissolved in PBS with Ca/Mg for 1 h at room temperature, and then washed.

**16. Tissue preparation and histological analysis staining.**

The tissue samples were embedded in O.C.T. cryomold (VWR Q-Path Chemicals, VWRQ00411243) and placed in cryostat (Leica 1860 cryostat) at -24°C. Cross or longitudinal cryo-sections (10 μm thick) were first air-dried, then processed for staining. Hematoxylin & eosin (Bio-Optica, Bio-Optica Milano Spa, 04-061010) or Masson's Trichrome Stain (Bio-Optica, Bio-Optica Milano Spa, 04-010802) were performed following manufacturing instructions. Samples were examined under a light microscope (Olympus BX60 microscope).

**17. Investigation of apoptosis.** *In vitro* cultures and tissues cryo-sections were fixed with 4% paraformaldehyde (PFA, Sigma, P6148) and Terminal deoxynucleotidyl transferase (TdT) dUTP Nick-End Labeling (TUNEL) assay was performed using the Click-iT Tunel Alexa Fluor 594 Imaging Assay (Invitrogen, C10246). Randomly chosen visual fields were observed under a 20x objective by epifluorescence or confocal imaging.

**18. Immunofluorescence analysis.**

For *in vitro* cultures and tissues cryo-sections, samples were fixed in 4% PFA and blocked using PBS-Triton 0.5% with bovine serum albumin 1% (BSA, Gibco). For Matrigel droplets with embedded enteroids and 2P printed geometries, samples were fixed in 2% glutaraldehyde dissolved in PBS with $Ca^{2+}$/$Mg^{2+}$ for 1 h at room temperature, and then washed. When required, samples were incubated 1 hour at room temperature in $NH_4Cl$ 0.1M in PBS to reduce hydrogel autofluorescence. Samples were incubated in blocking solution 1 h at 37 °C or overnight at +4 °C with the primary antibody. Secondary antibody and eventually phalloidin-647 (ThermoFisher, A22287) were incubated in blocking buffer for 1 h at room temperature (2 h at 37 °C for cultured organoids). Samples were analyzed by confocal imaging (Leica TCS SP5 microscope). The following primary antibodies were used: 1:100 Rat anti-α-Laminin (Sigma, L0663), 1:50 Rabbit anti-GFP (Invitrogen, A11122), 1:25 Mouse anti-eMyHC (DSHB, F1.652-s), 1:100 Rabbit anti-F4/80 (Santa Cruz Biotechnology, sc-25830), 1:25 Rabbit anti-MyoG (Santa Cruz Biotechnology, sc-576), 1:100 Rabbit anti-Human Ezrin (Thermo, PA5-29358); 1:100 Rat anti-integrin beta-4 (Abcam, ab110167); 1:50 Mouse anti-PEG (GenScript, 5E10E9); 1:500 Mouse

anti-hNestin (Millipore, MAB 5326); 1:5000 Mouse anti-BIII-Tubulin (Tuj1 - Biolegend, 801202); 1:50 Goat anti-Sox1 (R&D, AF3369); 1:200 Rabbit anti-Pax6 (Biolegend, 901301); 1:2000 Rabbit anti-p75 (Promega, G323A); 1:1000 Goat anti-GFAP (Abcam, ab53554); 1:10 Goat anti-collagen I (SouthernBiotech, 1310-01); 1:50 Rabbit anti-caspase-8 p18 (H-134; Sigma, sc-7890). The following secondary antibodies were used: 1:200 Donkey anti-mouse 488 (ThermoFisher, A21202); 1:200 Donkey anti-rabbit 488 (ThermoFisher, A21206); 1:200 Donkey anti-mouse 594 (ThermoFisher, A21203); 1:200 Donkey anti-rabbit 594 (ThermoFisher, A31573); 1:500 Goat anti-mouse 594 (ThermoFisher R37121); 1:500 Goat anti-Rabbit 568 (ThermoFisher, A-11011); 10 μg/mL Hoechst 33342 (ThermoFisher, H1399); 1:100 Goat anti-Rat Cy2 (Jackson, 112-225-167).

**19. Intravital HCC-4arm PEG, HCC-8arm PEG, HCC-Gel 3D printing and intravital HCC-Gel bioprinting.**

Mice were anaesthetized using *i.p.* injection of Zoletil® 50/50 (40 mg/kg) and Rompun® 2% Xilazina (5 mg/kg) in physiologic solution. Induction of deep surgical anaesthesia was confirmed by absence of reaction to hind foot pinch. The surgical procedure underwent in aseptic conditions (skin preparation, sterilized instruments, gloves and drapes). Photosensitive polymers were either autoclaved (HCC-4arm PEG or HCC-8arm PEG) or 0.22 μm filtered (HCC-Gel) before *in vivo* application. For blood flow analysis, Rhodamine B isothiocyanate-Dextran (Sigma, R9379) was injected in tail vein at 50 mg/mL in physiologic solution. When required, skin wound was closed with Vicryl 6-0 suture and no movement or space restriction were applied to animals after treatments. At the required time point, animals were deeply anesthetized, subjected to stereomicroscope and/or 2P intravital imaging, then sacrificed for tissues collection.

For i3D printing into skin, intra-dermal injection of 20 μL of HCC-4arm PEG or HCC-8arm PEG solutions (300 mg/mL in PBS) was performed at the abdomen, ear or hindlimb by using insulin syringes (Sacco, MED320924). Defined ROIs were subject to 2P absorption across the epidermis for hydrogel crosslinking and animals analyzed after i3D bioprinting.

For i3D printing into muscle, a skin incision was performed to expose the epimysium of the lateral hindlimb. Then, 10 μL of HCC-4arm PEG or HCC-8arm PEG solution (300 mg/mL in PBS) were injected between the epimysium and the skeletal muscle by using insulin syringes (Sacco, MED320924). Defined ROIs were subject to 2P absorption for hydrogel crosslinking between the epimysium and myofibers and animals analyzed after i3D bioprinting, 4 hours or 4 days after treatment.

For i3D printing into brain, a skin incision was performed to expose the skull. A small window in the skull (approx. 3 mm diameter) was performed with a drill around bregma to expose the brain. Then, 4 μL of HCC-8arm PEG solution (300 mg/mL in PBS) was injected sub-meningeal by using an Hamilton syringe connected to a glass capillary. For all the procedure, a glass coverslip was located above the site of the injection to allow objective immersion in PBS, before proceeding to i3D printing and intravital imaging.

For i3D bioprinting of mCherry$^+$FBs, 1.4 x $10^4$ cells were resuspended in 8 μl of HCC-Gel and then injected sub-epidermally or between the epimysium and the skeletal muscle by using insulin syringes, then eventually processed for i3D bioprinting and analyzed 14 or 21 days after treatment.

For i3D bioprinting of muscular cells, a mix of 1.4 x $10^4$ GFP$^+$MuSCs and 6 x $10^3$ mCherry$^+$FBs were used (70:30) were resuspended in 8 μl of HCC-Gel, PBS or Matrigel and then injected between the epimysium and the skeletal muscle of wild-type animals by using insulin syringes, then eventually processed for i3D bioprinting. All the animals were analyzed 7 days after treatment.

**19. Image preparation and analysis.**

ImageJ software were used for adjustments of levels and contrast, maximum and standard deviation intensity projections, 3D reconstructions and thresholding to create binary mask used for directionality and ferret angle measurements. To quantify the directionality of i3D bioprinted cells, at least 9 fluorescence images of independent biological triplicates for each sample were converted in binary masks, then analyzed with directionality plugin of ImageJ software (that indicates the amount of structures in a given direction). To quantify the shape of enclosed hSIOs, at least 9 fluorescence images of independent biological triplicates for each sample were converted in binary masks, then analyzed with measure plugin of ImageJ software to quantify the Feret's angle (the angle between the longest distance of the axis of any two points along the selection boundary (Feret's diameter) and a line parallel to the x-axis of the image).

All mentioned ImageJ plugins have source code available and are licensed under open-source GNU GPL v3 license.

**20. Statistical analysis.**

All the analysis were performed with GraphPad prism 6 software. Data were expressed as means ± s.e.m or s.d (when indicated). Statistical significance was determined by unequal variance Student's t-test or one-way ANOVA and

Tukey's multiple comparison test, or Kruskal-Wallis and Dunn's multiple comparison test. A P value of less than 0.05 was considered statistically significant.

**21. Experimentation with coumarin-polymer conjugates of the invention.**

58 known coumarin derivatives were considered, and 8 of them selected and further characterized (**Fig. 1** and **Fig. 5A**). 7-Hydroxycoumarin-3-carboxylic acid (HCCA) was defined as potential candidate for i3D bioprinting application, because it has electron-donating substituents in 7-positions and electron-withdrawing substituents in the 3-positions, which show a red shift of the single-photon absorption peak (350<λ<420 nm). Moreover, the presence of carboxylic acid allow conjugation of 7-Hydroxycoumarin-3-carboxylic (HCC) with linear PEG, branched PEG, and gelatin (Gel; **Fig. 3, 5B** and **schemes 1 and 2.** Hydrogen nuclear magnetic resonance ($^1$H-NMR) and Fourier-transform infrared (FT-IR) analysis confirmed Coum-PEG conjugation, and size-exclusion chromatography (SEC) analysis demonstrated HCC-Gel conjugation (**Fig. 5**); the conjugation degree was also quantified and absorbance spectra were analysed for all the HCC-polymers (**Fig. 4**).

Light-mediated cycloaddition was firstly investigated and characterized *in vitro* by using HCC-conjugated linear PEG (HCC-PEG). Single-photon excitation (λ = 365 nm) was able to induce HCC-PEG intermolecular cycloaddition in solution, resulting in dimerization of HCC-PEG as showed by spectrophotometric analysis (**Fig. 5C**). The ability of HCCA to undergo cycloaddition was further confirmed by $^1$H-NMR spectroscopy and electrospray ionization mass spectrometry analysis of HCCA solution (**Fig. 2**). Moreover, HCC underwent cycloaddition after 2P excitation at a wavelength of 850 nm using a multi-photon laser-scanning microscope, as showed by the rapid 2D HCC-PEG patterning (~10 sec per 850 $\mu m^2$) onto HCCA-functionalized glass slides with high spatial precision (**Fig. 5D**).

3D hydrogel constructs were obtained by 2P-crosslinking branched polymers conjugated with HCC in wide wavelength window (700<λ<950 nm). The fabrication proceeds according to a designed geometry in a layer-by-layer fashion. Crosslinking occurs only within the focal region (voxel) and is spatially controlled by a laser-scanning path of the multiphoton microscope. Typically, Z-stacks of regions of interest were exposed, selecting a Z-spacing of 1 μm between adjacent planes.

Single 3D objects made of HCC-4arm PEG, HCC-8arm PEG and HCC-Gel hydrogels were easily fabricated by 2P excitation, using near-IR laser at different wavelengths (700<λ<950 nm) at constant power, and remained stable after removal of non-cross-liked solution (**Fig. 6**). 3D structures with complex morphology can be easily fabricated by sequential addition of multiple objects (**Fig. 5E, Fig.7**), including a readable barcode (**Fig. 7C**), words (Fig. 7C-**D**) or an empty cube (**Fig. 7E**). Figure 5E shows parallelepipeds that are 45° rotated on same z plane to form an asterisk. Accuracy of 3D photo-crosslinking was further confirmed after fabrication of hydrogels by photo-crosslinking multiple Z positions, (**Fig. 8A**) or by assembling additively multiple parallel linear objects. The minimal linewidth (1.9±0.2 μm) of fabricated linear objects (**Fig. 8B**) and the sub-micrometric resolution of the bioprinting (**Fig. 8C**) was achieved. Accordingly, a 3D single line flower-shaped hydrogel of HCC-Gel was fabricated using a computer drawing pad combined to a free line scan program of the multi-photon microscope (**Fig. 8D**). Additionally, maximum 3D object dimension was tested (ΔZ ~2 mm) that was possible to fabricate without changing printing parameters (scanning speed and laser power; **Fig. 8e**). Considering the final *in vivo* application and taking in account the displacement due to animal respiratory movements and pulsed blood flow, An optimal writing time was quantified per single line (ms/line), which allows 3D hydrogel production with structural integrity (**Fig. 8F**). The optimal writing time of 1 ms/line was therefore used for the i3D bioprinting experiments; lower levels of laser powers does not trigger 2P-crosslinking, whereas higher powers may induce heat damage. Multiphoton microscope also allows to fine tune laser power, which in turn can modulate hydrogel photo-crosslinking, and subsequently the cross-linking density and the hydrogel mechanical properties. Based on the well-known biological role of stiffness in regulating cell behavior, the above characteristics were used to generate 8arm-PEG-hydrogels with tunable mechanical properties, that fall into biological-matched ranges of stiffness (1 kPa - 20 kPa) (**Fig. 8G**). 7-Carboxymethoxy-4-methylcoumarin (CMMC)-4arm PEG and CMMC-Gel were specifically designed and produced to perform 1-photon UV mediated cycloaddition (**Fig. 3D,E** and **Fig. 5A**). The elastic modulus increases to asymptotic values by increasing the irradiation time, showing a sigmoidal temporal profile of elastic modulus that reached the polymer crosslinking saturation, independently from the polymeric backbone used (**Fig. 8H**). Interestingly, the modulation of elastic modulus of hydrogels obtained with 1P and 2P photo-crosslinking (with CMMC-Gel and HCC-gel, respectively) by varying the polymer concentration showed remarkable similarity (**Fig. 8I**).

It was already reported that irradiation of the nano-aggregates of coumarin (7-(diethylamino)-3-coumarin carboxylic acid, 7-DAC) conjugated dipeptide at 365 nm induces dimerization of the coumarin groups. The authors observed through transmission electron microscopy (TEM) analysis that PEG-Coum forms nano-aggregates (**Fig. 9**). This experimental evidence further confirmed that HCC-polymer in aqueous solution could self-assembly into supramolecular structures, in which HCC moieties interact each other into the hydrophobic pocket of the nano-structure. In this view, the near-IR photo-active groups are in close proximity and undergo to fast cycloaddition leading to efficient crosslinking reaction within the confined 2P optical voxel. Differently, conventional 2P polymerized hydrogels require the presence of a

photo-initiator and possible co-initiator or sensitizer molecules (J. Torgersen et al., Hydrogels for two-photon polymerization: A toolbox for mimicking the extracellular matrix. Adv. Funct. Mater. 23, 4542-4554 (2013)) that when excited at 365 nm, activate free radical chemical crosslinking and release of moieties through diffusion-reaction mechanism, which can be harmful for living tissues (C. G. Williams, A. N. Malik, T. K. Kim, P. N. Manson, J. H. Elisseeff, Variable cytocompatibility of six cell lines with photoinitiators used for polymerizing hydrogels and cell encapsulation. Biomaterials. 26, 1211-1218 (2005)).

To investigate biocompatibility of 2P crosslinked hydrogels, firstly the ability of cells to adhere, survive and be cultured onto Coum-Gel hydrogel micropatterning was confirmed (**Fig. 10, 11 and 12A**). Subsequently, the ability of our experimental setup to allow cell-laden 3D bioprinting without affecting cell viability was proved (**Fig. 12B,D**). The elastic modulus of bioprinted structures was not strongly changed by the presence of cells (Young's modulus was reduced by about 6% in presence of cells, **Fig. 12C**), suggesting that the 2P-crosslinking and hydrogel fabrication were not substantially affected by cells suspended in the HCC-Gel solution. The authors then asked whether the shape of 3D bioprinted cell-laden HCC-Gel could provide specific topological cues. To investigate this, The ability of murine muscle-derived stem cells (MuSC) was used to differentiate into myotubes and to adapt their morphology and orientation based on microenvironment topology. As confirmed by quantification of cell directionality, single-nucleated or polynucleated cells within elongated-shaped HCC-Gel structures appeared oriented along major axis of the 3D structures (**Fig. 12E,** left panel; **Fig. 12f**), whereas cells located in cuboid-shaped HCC-Gel structures were randomly oriented (**Fig. 12E,** right panel; **Fig. 12F**). Finally, to evaluate whether HCC-Gel hydrogels were able to support cell culture for longer time and cell functionality, MuSCs were cultured on parallelepiped-shaped HCC-Gel structures for 10 days. Microscopic analysis demonstrated that parallelepiped-shaped HCC-Gel hydrogels support the formation of aligned multinucleated myotubes (**Fig. 12G**) that showed spontaneous contraction 5 days after induction of cell differentiation. These data provide a proof of concept that the setup disclosed in the present invention can be employed synergistically to conventional 3D bioprinting (C. S. Ong et al. 2017).

However, as mentioned before, i3D bioprinting involves additional complexities related to the fact that 3D bioprinting needs to be performed in a peculiar site of living animals, namely a tissue or an empty body cavity or interstitial space that may include a variety of components present into the extracellular matrix domain. Thus, the authors then challenged the possibility to fabricate 3D hydrogel objects into a pre-formed 3D culture system. As 3D model, the authors used a 2 mm diameter droplet of Matrigel, a gelatinous protein mixture widely used for gel-embedded form-free 3D cultures of cells or organoids. The data provided demonstrate that by mixing 50/50 HCC-Gel/Matrigel or by loading HCC-polymers within preformed Matrigel, it is possible to crosslink 3D structures of defined XYZ position and orientation within the 3D droplet (**Fig. 13A**). The authors observed that 2P crosslinking efficiency was comparable to pure HCC-Gel solution exhibiting fully bio-orthogonal reactions, as showed by comparable elastic modulus of hydrogel fabricated in absence of Matrigel (**Fig. 13B**). The inventors next verified that the HCC-Gel/Matrigel 3D gel, before cross-linking, was able to support the viability and the growth of human small intestinal organoids, which is a challenging *in vitro* 3D human culture. Therefore, the mixture of HCC-Gel and Matrigel seems to maintain the functional support of organoid culture while preserving the possibility of fabricating cross-linked object within the gel. With this in mind, the authors fabricated 3D parallelepipeds accurately positioned and oriented relative to selected human small intestinal organoid within the 3D culture in HCC-Gel/Matrigel 3D gel (**Fig. 13C**). HCC-Gel hydrogel cytocompatibility on hSIOs enclosed in the structures was demonstrated by live/dead assay performed after 2 days and 8 days of culture (**Fig. 13D**) Then, the authors investigated whether bioprinted HCC-Gel walls within Matrigel and specifically positioned around individual hSIOs could affect their biological behavior (**Fig. 13C**). hSIOs confined inside an open square-box (formed by 4 orthogonal walls) grew normally and after few days of culture touched and deformed HCC-Gel hydrogel walls (**Fig. 13D**,**E**). Between day 6 and day 8 of culture, hSIOs enclosed in HCC-Gel-based hydrogel showed marked morphology changes, which lead to well-developed columnar epithelium (**Fig. 13F**). This morphology was characterized by thickening of the organoid wall, F-actin localization in apical position and nuclei constriction in the basal domain as quantified by the basal-apical distance ratio and the basal/apical axis (**Fig. 13G**-I). In comparison, hSIOs cultured in the same hydrogel composition but without any contact with HCC-Gel structures did not show such morphological changes (**Fig. 13F**). Interestingly, the basal localization of beta4-integrin observed in hSIOs enclosed in HCC-Gel structures (**Fig. 13I**) suggests that cell adhesion could be one possible mechanism involved in the observed phenotype. To dissect the contributions of extrinsic mechanical vs biochemical signals in triggering the organoid morphological changes, hSIOs cultured in Matrigel were enclosed inside an open square-box made of HCC-8arm PEG-based hydrogel, which is well-known to display cell repellent properties. The inventors proved that HCC-8arm PEG/Matrigel allowed hSIO survival and growth in culture (**Fig. 13J**). However, differently from what observed with HCC-Gel, hSIOs enclosed in HCC-8arm PEG-based hydrogel did not show columnar epithelium after 8 days of culture, hydrogel deformation, actin apical localization nor nuclei constriction (**Fig. 13K,L**). Instead, hSIOs morphology reflects the 3D shape of the enclosed open-box made by HCC-8arm PEG-based hydrogel. As confirmed by Feret's angle quantification, only hSIOs enclosed by HCC-8arm PEG acquired a cuboidal cystic structure 6 days after culture, when compared to HCC-Gel enclosed hSIOs (**Fig. 13M**). Our results suggest that the formation of columnar epithelium of hSIO could be correlated to the hydrogel biochemical composition rather than its pure mechanical

properties, since HCC-Gel and HCC-PEG were fabricated for having the same Young's modulus. It would be of future interest to use this approach to investigate the cues that give rise to columnar epithelium in hSIOs. This feature represents an advance to conventional bioprinting technology and a step forward for i3D bioprinting in living animals, in which fabrication of 3D object within pre-existing tissue at precise time are essential prerequisites. Moreover, the [2+2] cycloaddition seems independent from media and biological components, confirming the bio-orthogonal 2P HCC crosslinking chemistry.

The authors next goal was to provide experimental evidence that i3D bioprinting is possible across tissues without open surgery. Skin was identified as an optimal target organ. The experimental strategy is straightforward: first, HCC-polymer solutions is delivered into skin by a simple sub-epidermal injection; second, the photosensitive solution retained under the epidermis is exposed to focalized pulsed near-IR laser light for 3D hydrogel fabrication using a multiphoton microscope device. To effectively translate *in vivo* the 3D hydrogel fabrication, laser power and fabrication speed was optimized across tissues balancing the crosslinking efficiency, velocity of 3D bioprinting (to reduce the effect of movements associated to the breath and the cardiac beat of a live animal), and heating-induced tissue damage. Wild type- or green fluorescent protein ($GFP^+$) transgenic-mice were anesthetized, subjected to sub-epidermal injection of HCC-polymer solutions and transferred under the multiphoton microscope for i3D bioprinting. With this setup, the authors succeeded in efficiently fabricate isolated 3D objects into dermis of living animals at different districts of the body (**Fig. 14-18**). Figure **14A** shows a manufactured object located into dermis of $GFP^+$ mice (to take advantages of fluorescence imaging of host tissues) and the integrity of the epidermis above the cross-linked hydrogel. The exact localization can be revealed by the hair bulb position. The high compatibility of i3D bioprinting was further confirmed by the absence of evident skin damage observed upon the entire procedure, comparable to untreated tissue (Fig. 14A**-C**). Furthermore, as demonstrated *in vitro,* the authors showed that i3D bioprinting across epidermis allow the manufacture of 3D hydrogel structures with micrometric linewidth and the fabrication of inserts with a variety of morphologies in virtue of the precise XYZ positioning (**Fig. 15**).

The use of minimally invasive surgical procedures, including laparoscopy and endoscopy, together with the optical imaging-guided surgery, which includes the use of multiphoton lasers, allow real-time visualization of different internal organs and the anatomic area of interest. Starting from that, the authors challenged the possibility to apply i3D bioprinting to other organs rather than skin still preserving minimal invasiveness to the target organs and fine resolution and positioning of hydrogel fabrication. A minimal surgery procedure was applied to expose intact skeletal muscle and brain that were then treated without performing direct surgery to the organ *per se*, rather to the containing structures, by injecting HCC-polymer solutions across the connective tissues that anatomically surround those organs.

In particular, photosensitive solutions injected under the epymisium of skeletal muscle were photo-crosslinked to fabricate a 3D hydrogel upon muscle fibers without evident tissue damage (Fig. 14D**-H, Fig. 16-18**). This experimental setup allows real-time imaging during and after i3D bioprinting, giving the possibility of positioning the hydrogel to be manufactured according to a biological entity of interest, i.e. a single myofibre with respect to grouped myofibres. Given the natural patterning of myofibres, skeletal muscle was also used to verify the ability of fabricating i3D bioprinted hydrogels by precisely tuning their XYZ position (**Fig. 14F, Fig. 16**), both compared to the myofibre orientation and to multiple objects orientation in parallel or perpendicular fashion (**Fig. 14H, Fig. 16**). The high efficiency of HCC-cycloaddition upon 2P excitation and the versatility of the fabrication protocol was further proved by performing i3D bioprinting of an infinite-shaped hydrogel of HCC-Gel, using the design realized with a computer drawing pad as input for the free line scan program of the multiphoton microscope (**Fig. 17**). i3D bioprinting was also possible without tissue damage in a delicate organ such as brain. Burr hole was performed into the skull of mice to allow HCC-polymeric solution injection across meninges; hydrogel polymerization was achieved by using 2P excitation through near-IR multiphoton microscopy (**Fig. 14G**). The persistent and correct maintenance of blood flow into the brain and the absence of bleeding foci, located at the area of i3D bioprinting, further confirmed the low invasive procedure of 2P crosslinking (**Fig. 14G, Fig. 18A**). Most importantly, the authors found that 2P crosslinked hydrogels can surround and embed the vasculature network without damaging the vessels and preserving blood flow as shown by red-dextran tracing (**Fig. 14H** and **Fig. 18B**).

To reach the above results and effectively translate 3D hydrogel fabrication *in vivo,* laser power and writing time across tissues were optimized balancing crosslinking efficiency, velocity of 3D bioprinting (to reduce the effect of movements associated to breathing and cardiac beating), and heat-induced tissue damage. The optimal writing time (1 ms/line) was combined with the optimal laser power (1 mW) to obtain a stable hydrogel under epimysium without myofiber damage, as the appearance of heat-induced damage, myofiber contraction and cleavage of cell death-marker caspase-8 were absent (**Fig. 19**). Myofiber damage was observed only after 2P irradiation with 3-fold increased laser power and ~17-fold increased time of irradiation per single confocal plane, when compared to i3D bioprinting condition (**Fig. 19**). Therefore, the biocompatibility of HCC-4arm PEG and HCC-Gel solution was investigated. HCC-4arm PEG crosslinked hydrogels were found to be preserved *in vivo* 4 days after i3D printing across epimysium, with no evident tissue damage at the site of 2P-crosslinking as shown by imaging analysis (**Fig. 20**). Consistent with PEG biocompatibility studies[43], immunofluorescence analysis showed recruitment of macrophages to the site of hydrogel crosslinking, despite the fact no evident histological changes were observed (**Fig. 20**). Since PEG hydrogels are cell repellent, and based on

previous *in vitro* results, *in vivo* biocompatibility of our injectable HCC-Gel hydrogels before challenging longer term i3D printing in the presence of donor cells (i3D bioprinting) was further investigated. In order to prove biocompatibility of HCC-Gel, the polymeric solution was subcutaneously injected into wild type mice and histological (Haematoxylin-eosin and Masson trichromic) staining, TUNEL test, and immunofluorescence staining for macrophages were performed after 4, 7 and 21 days. When compared to animals treated with PBS, no evident histological modification, cell apoptosis or macrophage infiltration were observed (**Fig. 21**).

Lastly, the inventors aimed at showing that i3D bioprinting could be effectively employed in live animals for *i*) delivering cells in a spatially controlled manner; *ii*) allowing precise cell grafting into a defined anatomical site; and *iii*) supporting a proper structural organization of *de novo* tissues. The inventors then investigated if i3D bioprinting could instruct spatial control of cell engraftment by firstly employing HCC-Gel laden with primary mCherry$^+$FBs, delivered across the epimysium of murine hind limb of isogeneic immunocompetent animals. Distinct freshly fabricated HCC-Gel structures containing embedded mCherry$^+$FBs were located between the epimysium and skeletal muscle of host wild-type mice accordingly with the bioprinted structures (**Fig. 21A**). Then, longer term studies were performed for investigating the effect of cell-laden i3D bioprinting on donor cells. The mCherry$^+$FBs suspended into HCC-Gel solution were injected under the epimysium (no i3D bioprinting) or injected and subjected to i3D bioprinting for the generation of parallelepiped-shaped constructs. Donor mCherry$^+$FBs were identified in host animals 21 days after delivery in both experimental conditions (Fig. 21B-**H**). However, imaging analysis showed elongated clusters of mCherry$^+$FBs only in i3D bioprinted animals, which followed the posteroanterior axis of the parallelepiped-shaped constructs defined during i3D bioprinting (**Fig. 21b-e**). Conversely, injected cells that did not undergo i3D bioprinting were organized into rounded clusters (**Fig. 21B,F-H**). To extend these results to another tissue, i3D bioprinting was also performed across the epidermis. Elongated localization of mCherry$^+$FBs was observed 14 days after intra-dermal injection and i3D bioprinting of embedded donor cells into parallelepiped-shaped constructs, when compared to mCherry$^+$FBs injected only, that conversely remained localized into rounded clusters (**Fig. 21I**). These data strongly suggested that i3D bioprinting can elicit a desired spatial organization of delivered cells.

Finally, the inventors aimed at demonstrating that i3D bioprinting has the potential to support *de novo* tissue formation. As a tissue model, skeletal muscle was used since its formation requires anisotropic cell alignment during the early stages of skeletal muscle regeneration. An experimental strategy was designed in which HCC-Gel solution laden with both FBs and muscle stem cells (MuSCs) was employed for testing whether i3D bioprinting of parallelepipeds could promote *de novo* skeletal muscle formation. A defined ratio of GFP$^+$MuSCs to mCherry$^+$ in HCC-Gel solution were injected and then i3D bioprinted into multiple parallelepiped-shaped constructs (**Fig. 7a**). As controls, animals were also injected with cells resuspended in PBS, or in Matrigel or HCC-Gel solution without 2P absorption (**Fig. 23A**). One week after treatments, confined bundle of elongated GFP$^+$ cells was observed only in animals that underwent i3D bioprinting (**Fig. 23A,B**). Conversely, after injection without i3D bioprinting, sparse cells and randomly organized myotubes were found in the host tissue (**Fig. 23B**). Remarkably, the bundle of elongated GFP$^+$ cells were integrated by the host tissue, with a functional three-dimensionally organized vascular network observed only in i3D bioprinted animals (**Fig. 23B**). In agreement with previous results, quantification of cell directionality showed that i3D bioprinting allows the generation of a spatially controlled cell engraftment (**Fig. 23C**). Immunofluorescence analysis confirmed that *de novo* formed tissue (GFP-positive) is constitute of regenerating myofibers expressing embryonic myosin heavy chain protein (eMHC), which also revealed striated organization of the muscular cytoskeleton, as well as presence of some centrally nucleated myofibers (Fig. 23D-**F**). Moreover, mCherry$^+$FBs were founded at the periphery of GFP$^+$ myofibers derived from donor GFP$^+$MuSCs. Immunostainig for GFP, laminin and nuclei was used to quantify centrally nucleated myofibers. The analysis revealed that donor myofibers are mostly centrally nucleated, conversely host myofibers do not show centrally located nuclei (**Fig. 23G**). Altogether, these results indicate that i3D bioprinting supports *de novo* skeletal muscle tissue formation, which was characterized by proper structural organization and integration with the host vascular system.

The inventors then investigated whether their photosensitive hydrogels were also able to undergo decrosslinking when exposed to multiphoton NIR light. Therefore HCC-based or CMMC-based hydrogels were fabricated by multiphoton or UV light irradiation as previously shown. Upon development, z-stacks of regions of interest (ROI) were excited with the multiphoton microscope at different laser wavelength and 1 mW laser power. The inventors found that hydrogels were efficiently removed only within the laser-scanning path of the multiphoton microscope within the IR light range of $700\ nm < \lambda < 900\ nm$, providing a 3D spatial control of the decrosslinking (Fig. 24A). Confirmation of the complete removal of the hydrogels in the ROI after multiphoton irradiation was performed by evaluating the ability of a microfiber (Fig. 24B), or of autofluorescence microbeads (Fig. 24C) or of FITC-dextrans 500 kDa solution (Fig. 24D) to freely enter into channels generated within the photosensitive hydrogels via multiphoton-mediated decrosslinking.

The inventors previously showed that by tuning laser power they were able to modulate hydrogel photo-crosslinking rate and, consequently, the hydrogel mechanical properties. Therefore, the inventors investigated whether hydrogel crosslinking and decrosslinking could be modulated by controlling the multiphoton irradiation of specific ROI within fabricated hydrogels. Multiphoton irradiation of z-stacks of ROI with laser power < 1mW were used to achieve a partial and progressive photo-crosslinking or photodegradation of the hydrogels according to the multiphoton path defined

by the specific ROI. To demonstrate that multiphoton irradiation of fabricated hydrogel can modulate hydrogel crosslinking/decrosslinking, AFM was performed on untreated HCC-based or CMMC-based hydrogels or on hydrogel subjected to 1 to 6 cycles of multiphoton irradiation at 800 nm wavelength of a same ROI (Fig. 24E). The inventors observed that 1 or 2 cycles of irradiation increased the hydrogel stiffness, indicating that hydrogel can undergo to a crosslinking reaction within the irradiated ROI of the hydrogel (Fig 24E, points 1 and 2). Differently, 3 to 6 multiple cycles of irradiation allowed progressive reduction in hydrogel stiffness, indicating a multiphoton-mediated decrosslinking event (Fig 24E, points 3 to 6). Seven cycles of multiphoton irradiation led to complete loss of hydrogels in the specific ROI.

In summary, the authors show that i3D bioprinting allows the fabrication of hydrogels that can support the maintenance of cell functionality and can be used to modulate their behavior. With this approach, bioprinting is possible not only *in vitro* into pre-existing 3D environments (Matrigel), but also across tissues in different target organs in live animals together with real-time imaging.

By allowing hydrogel fabrication into pre-existing 3D environments, i3D bioprinting technology represents an innovative strategy that could be used to advance our understanding of *in vitro* organoid models. Deliberate regulation of self-organization and morphogenesis hold the potential to ameliorate organoid topology and reproducibility, as well as to dissect mechanotransduction and biochemical mechanisms underpinning specific cell responses in 3D culture conditions. Furthermore, i3D bioprinting is envisioned as a mean to perform *in vivo* 3D confined cell delivery, capable of instructing cell structural organization within tissues. This represents a novel strategy to improve engraftment of cells after injection within tissues, avoiding fast dispersion and the short life-time of cells that typically occurs with this delivery strategy.

The i3D technology offers an unprecedented opportunity to study cell biology and physiology in 3D environments, where the internal structure and organization can be modified according to the biological needs both *in vitro* and *in vivo.* It is worth to underline that this approach could suffer from limitations related to the multi-photon microscopy, such as the size and depth of hydrogel crosslinking that falls into millimeter scale. Conversely, research in multi-photon microscopy/lithography is rapidly developing novel strategies to overcome such limitations, including deep tissue penetration with 3 photon excitation, optical fiber guidance and multi-photon holographic technology. At the current stage i3D bioprinting is limited to anatomical sites that can be exposed to the light source. However, the inventors envision that in the future i3D bioprinting can benefit from the above developing strategies and can be coupled with emerging optical imaging-guided surgery, opening new paradigm in minimally invasive-surgical techniques suitable for organ repair, reconstruction and *de novo* fabrication.

## Claims

**1.** A coumarin-polymer conjugate comprising a coumarin compound and a polymer, **characterized in that**

said coumarin compound has maximum single-photon absorption at wavelength, Abs λ, from 310 to 450 nm, a maximum multi-photon absorption at wavelength Abs λ , from 700 to 1000 nm, at least one electron-donor in positions 6 and/or 7 and/or at least an electron-acceptor group in positions 3 and/or 4 wherein said coumarin compound is selected from
7-Carboxymethoxy-4-methylcoumarin
7-Hydroxy-4-methylcoumarin
7-Amino-4-methyl-3-coumarinylacetic acid
7-Hydroxy-4-(trifluoromethyl)coumarin
7-Hydroxycoumarin-3-carboxylic acid
Ethyl 7-Hydroxycoumarin-3-carboxylate
7-Hydroxycoumarin-3-carboxylic acid N-succinimidyl ester.

**2.** The coumarin-polymer conjugate of claim 1 wherein said coumarin compound has an electron-donor group in each of positions 6 and 7 and an electron-acceptor group in each of position 3 and 4, and/or

wherein said coumarin compound is optionally substituted, with the provision that, when a substituent is in positions 6 and/or 7 at least one substituent has an electron-donor group and/or when a substituent is in positions 3 and/or 4, at least one substituent has an electron-acceptor group and said substituted coumarin compound has maximum single-photon absorption at wavelength, Abs λ, from 310 to 450 nm and a maximum multi-photon absorption at wavelength Abs λ , from 700 to 1000 nm and/or
wherein said coumarin compound is directly conjugated by a covalent bond to said polymer, or wherein said coumarin compound is conjugated by a linker to said polymer or wherein said coumarin compound is linked to said polymer by a linker molecule.

3. The coumarin-polymer conjugate of claim 1 or 2 wherein the substituted coumarin compound is 7-(carboxymethoxy)-4-methylcoumarin or 7-hydroxycoumarin-3-carboxylic acid.

4. The coumarin-polymer conjugate of anyone of claims 1 to 3 wherein said polymer is natural polymer or a synthetic polymer.

5. The coumarin-polymer conjugate of anyone of claims 1 to 4 wherein said polymer is a biomedical and/or a biocompatible polymer, preferably wherein said natural polymer is selected from: collagen, Gelatin, Fibrin, Elastin, Silk, Bulk decellularized extracellular matrix, protein extract from decellularized extracellular matrix, Cellulose and derivatives, including methyl cellulose (MC), hydroxypropyl cellulose (HPC), hydroxypropylmethyl cellulose (HPMC), and carboxymethyl cellulose (CMC) thereof, Alginates, Dextran, Chitosan, Hyaluronic acid, Chondroitin sulfate or wherein said synthetic polymer is selected from: polyesters, polyurethanes, polyanhydrides, polyphosphazenes, polypropylene fumarate, poly ethylene, poly propylene, poly(methyl methacrylate) (PMMA), poly(dimethylsiloxane), poly(ethylene oxide) , poly(propylene oxide), poly(vinyl alcohol) (PVA).

6. The coumarin-polymer conjugate of anyone of claims 1 to 5 in combination with one or more drugs and/or one or more cell type.

7. The coumarin-polymer conjugate anyone of claims 1 to 6 wherein, when said conjugate is irradiated with IR light at a wavelength $\lambda$ from 700 nm to 1000 nm, the coumarin components of different units of said coumarin-polymer conjugate undergo a cycloaddition reaction thereby forming a crosslinked product, preferably wherein said wavelength $\lambda$ is from 800 nm to 1000 nm; from 800 nm to 950 nm; from 850 nm to 950 nm or from 900 to 950 nm.

8. A mixture of coumarin-polymer conjugates according to anyone of claims 1 to 7 preferably comprising at least coumarin-natural polymer conjugate and at least one coumarin-synthetic polymer conjugate.

9. The mixture of coumarin-polymer conjugates of claim 8 in combination with one or more drugs and/or one or more cell type.

10. A product comprising one or more coumarin-polymer conjugates according to anyone of claims 1 to 7 whereby the coumarin components of different units of said coumarin-polymer conjugates are cross-linked preferably further comprising one or more drug and/or one or more cell type wherein said product is preferably in the form of a hydrogel.

11. A medical device comprising the coumarin-polymer conjugate of anyone of claims 1 to 7, or the mixture of claims 8 or 9, or the product of claim 10, preferably in the form of an implantable device.

12. An organoid or a scaffold comprising the product of claim 10 and tissue specific cells or stem cells.

13. A method for the preparation of a product comprising crosslinked coumarin-polymer conjugates or a mixture thereof according to claim 10 comprising the step of
subjecting to irradiation with a radiation source at a wavelength or 700 nm $< \lambda <$ 1000 nm a coumarin-polymer conjugate or a mixture thereof according to anyone of claims 1 to 7, preferably wherein said conjugate or mixture thereof is in combination with one or more drugs, and/or one or more cell type prior to irradiation.

14. Use of coumarin-polymer conjugates according to anyone of clams 1 to 7 for the generation of cross-linked hydrogels for in vitro cell culture, cell-laden bioprinting and bioprinting into pre-existing 3D materials) or as drug or cell delivery systems.

15. A method for the de-crosslinking of a coumarin-polymer conjugate or a mixture thereof in a product according to claim10 comprising the step of
subjecting said conjugate or mixture thereof to irradiation with a radiation source at a wavelength 700 nm $< \lambda <$ 1000 nm.

**Patentansprüche**

1. Cumarin-Polymer-Konjugat, umfassend eine Cumarinverbindung und ein Polymer, **dadurch gekennzeichnet, dass**

die Cumarinverbindung eine maximale Einphotonen-Absorption bei einer Wellenlänge, Abs λ, von 310 bis 450 nm, eine maximale Mehrphotonen-Absorption bei einer Wellenlänge Abs λ, von 700 bis 1000 nm, mindestens einen Elektronendonor in den Positionen 6 und/oder 7 und/oder mindestens eine Elektronenakzeptorgruppe in den Positionen 3 und/oder 4 aufweist, wobei die Cumarinverbindung ausgewählt ist aus
7-Carboxymethoxy-4-methylcumarin
7-Hydroxy-4-methylcumarin
7-Amino-4-methyl-3-coumarinylaceticsäure
7-Hydroxy-4-(trifluormethyl)cumarin
7-Hydroxycumarin-3-carbonsäure
Ethyl-7-hydroxycumarin-3-carboxylat 7-Hydroxycumarin-3-carbonsäure-N-succinimidylester.

2. Cumarin-Polymer-Konjugat nach Anspruch 1, wobei die Cumarinverbindung eine Elektronendonorgruppe in jeder der Positionen 6 und 7 und eine Elektronenakzeptorgruppe in jeder der Positionen 3 und 4 aufweist, und/oder

wobei die Cumarinverbindung optional substituiert ist, mit der Maßgabe, dass, wenn ein Substituent in der Position 6 und/oder 7 ist, mindestens ein Substituent eine Elektronendonorgruppe aufweist und/oder wenn ein Substituent in der Position 3 und/oder 4 ist, mindestens ein Substituent eine Elektronenakzeptorgruppe aufweist und die substituierte Cumarinverbindung eine maximale Einphotonen-Absorption bei einer Wellenlänge, Abs λ, von 310 bis 450 nm und eine maximale Mehrphotonen-Absorption bei einer Wellenlänge Abs λ von 700 bis 1000 nm aufweist und/oder
wobei die Cumarinverbindung direkt durch eine kovalente Bindung an das Polymer konjugiert ist, oder wobei die Cumarinverbindung durch einen Linker an das Polymer konjugiert ist oder wobei die Cumarinverbindung durch ein Linkermolekül an das Polymer gelinkt ist.

3. Cumarin-Polymer-Konjugat nach Anspruch 1 oder 2, wobei die substituierte Cumarinverbindung 7-(Carboxymethoxy)-4-methylcumarin oder 7-Hydroxycumarin-3-carbonsäure ist.

4. Cumarin-Polymer-Konjugat nach einem der Ansprüche 1 bis 3, wobei das Polymer ein natürliches Polymer oder ein synthetisches Polymer ist.

5. Das Cumarin-Polymer-Konjugat nach einem der Ansprüche 1 bis 4, wobei das Polymer ein biomedizinisches und/oder ein biokompatibles Polymer ist, vorzugsweise wobei das natürliche Polymer ausgewählt ist aus: Kollagen, Gelatine, Fibrin, Elastin, Seide, Bulk-dezellularisierte extrazelluläre Matrix, Proteinextrakt aus dezellularisierter extrazellulärer Matrix, Cellulose und Derivaten, einschließlich Methylcellulose (MC), Hydroxypropylcellulose (HPC), Hydroxypropylmethylcellulose (HPMC) und Carboxymethylcellulose (CMC) davon, Alginaten, Dextran, Chitosan, Hyaluronsäure, Chondroitinsulfat oder wobei das synthetische Polymer ausgewählt ist aus: Polyester, Polyurethane, Polyanhydriden, Polyphosphazenen, Polypropylenfumarat, Polyethylen, Polypropylen, Poly(methylmethacrylat) (PMMA), Poly(dimethylsiloxan), Poly(ethylenoxid), Poly(propylenoxid), Poly(vinylalkohol) (PVA).

6. Cumarin-Polymer-Konjugat nach einem der Ansprüche 1 bis 5 in Kombination mit einem oder mehreren Arzneimitteln und/oder einem oder mehreren Zelltypen.

7. Cumarin-Polymer-Konjugat nach einem der Ansprüche 1 bis 6, wobei, wenn das Konjugat mit IR-Licht bei einer Wellenlänge λ von 700 nm bis 1000 nm bestrahlt wird, die Cumarin-Komponenten unterschiedlicher Einheiten des Cumarin-Polymer-Konjugats eine Cycloadditionsreaktion durchlaufen, wodurch ein vernetztes Produkt ausgebildet wird, vorzugsweise wobei die Wellenlänge λ von 800 nm bis 1000 nm, von 800 nm bis 950 nm; von 850 nm bis 950 nm oder von 900 bis 950 nm beträgt.

8. Mischung von Cumarin-Polymer-Konjugaten nach einem der Ansprüche 1 bis 7, vorzugsweise umfassend mindestens ein Cumarin-Naturpolymer-Konjugat und mindestens ein Cumarin-Synthetikpolymer-Konjugat.

9. Mischung von Cumarin-Polymer-Konjugaten nach Anspruch 8 in Kombination mit einem oder mehreren Arzneimitteln und/oder einem oder mehreren Zelltypen.

10. Produkt, umfassend ein oder mehrere Cumarin-Polymer-Konjugate nach einem der Ansprüche 1 bis 7, wobei die Cumarin-Komponenten unterschiedlicher Einheiten der Cumarin-Polymer-Konjugate vernetzt sind, vorzugsweise ferner umfassend ein oder mehrere Arzneimittel und/oder einen oder mehrere Zelltypen, wobei das Produkt vorzugsweise in Form eines Hydrogels vorliegt.

11. Medizinische Vorrichtung, umfassend das Cumarin-Polymer-Konjugat nach einem der Ansprüche 1 bis 7, oder die Mischung nach Anspruch 8 oder 9, oder das Produkt nach Anspruch 10, vorzugsweise in Form einer implantierbaren Vorrichtung.

12. Organoid oder Gerüst, umfassend das Produkt von Anspruch 10 und gewebespezifische Zellen oder Stammzellen.

13. Verfahren zum Herstellen eines Produkts, umfassend vernetzte Cumarin-Polymer-Konjugate oder eine Mischung davon nach Anspruch 10 umfassend den Schritt:
Unterziehen einer Bestrahlung mit einer Strahlungsquelle bei einer Wellenlänge von 700 nm < $\lambda$ < 1000 nm eines Cumarin-Polymer-Konjugats oder einer Mischung davon nach einem der Ansprüche 1 bis 7, vorzugsweise wobei das Konjugat oder die Mischung davon in Kombination mit einem oder mehreren Arzneimitteln und/oder einem oder mehreren Zelltypen vor der Bestrahlung vorliegt.

14. Verwendung von Cumarin-Polymer-Konjugaten nach einem der Ansprüche 1 bis 7 für die Erzeugung von vernetzten Hydrogelen für eine in vitro Zellkultur, zellbeladenes Bioprinting und Bioprinting in bereits vorhandene 3D-Materialien oder als Arzneimittel- oder Zellabgabesysteme.

15. Verfahren zum Entvernetzen eines Cumarin-Polymer-Konjugats oder einer Mischung davon in einem Produkt nach Anspruch 10, umfassend den Schritt:
Unterziehen des Konjugats oder der Mischung davon einer Bestrahlung mit einer Strahlungsquelle bei einer Wellenlänge 700 nm < $\lambda$ < 1000 nm.

## Revendications

1. Conjugué coumarine-polymère comprenant un composé de coumarine et un polymère, **caractérisé en ce que**

    ledit composé de coumarine a une absorption monophotonique maximale à la longueur d'onde, Abs $\lambda$, allant de 310 à 450 nm, une absorption multiphotonique maximale à la longueur d'onde Abs $\lambda$, allant de 700 à 1000 nm, au moins un donneur d'électrons en positions 6 et/ou 7 et/ou au moins groupe accepteur d'électrons aux positions 3 et/ou 4 dans lequel ledit composé de coumarine est choisi parmi
    7-carboxyméthoxy-4-méthylcoumarine
    7-hydroxy-4-méthylcoumarine
    acide 7-amino-4-méthyl-3-coumarinylacétique
    7-hydroxy-4-(trifluorométhyl)coumarine
    acide 7-hydroxycoumarine-3-carboxylique
    7-dydroxycoumarine-3-carboxylate d'éthyle
    ester N-succinimidylique d'acide 7-hydroxycoumarine-3-carboxylique.

2. Conjugué coumarine-polymère selon la revendication 1 dans lequel ledit composé de coumarine a un groupe donneur d'électrons dans chacune des positions 6 et 7 et un groupe accepteur d'électrons dans chacune des positions 3 et 4, et/ou

    dans lequel ledit composé de coumarine est facultativement substitué, à condition que, lorsqu'un substituant est en positions 6 et/ou 7 au moins un substituant a un groupe donneur d'électrons et/ou lorsqu'un substituant est en positions 3 et/ou 4, au moins un substituant a un groupe accepteur d'électrons et ledit composé de coumarine substitué a une absorption monophotonique maximale à la longueur d'onde, Abs $\lambda$, allant de 310 à 450 nm et une absorption multiphotonique maximale à la longueur d'onde Abs $\lambda$, allant de 700 à 1000 nm et/ou
    dans lequel ledit composé de coumarine est directement conjugué par une liaison covalente audit polymère, ou dans lequel ledit composé de coumarine est conjugué par un lieur audit polymère ou dans lequel ledit composé de coumarine est lié audit polymère par une molécule de lieur.

3. Conjugué coumarine-polymère selon la revendication 1 ou 2 dans lequel le composé de coumarine substitué est 7-(carboxyméthoxy)-4-méthylcoumarine ou acide 7-hydroxycoumarin-3-carboxylique.

4. Conjugué coumarine-polymère selon l'une quelconque des revendications 1 à 3 dans lequel ledit polymère est un polymère naturel ou un polymère synthétique.

5. Conjugué coumarine-polymère selon l'une quelconque des revendications 1 à 4 dans lequel ledit polymère est un polymère biomédical et/ou biocompatible, de préférence dans lequel ledit polymère naturel est choisi parmi : collagène, gélatine, fibrine, élastine, soie, matrice extracellulaire décellularisée en vrac, extrait de protéine provenant de matrice extracellulaire décellularisée, cellulose et dérivés, y compris méthylcellulose (MC), hydroxypropylcellulose (HPC), hydroxypropylméthylcellulose (HPMC) et carboxyméthylcellulose (CMC) de celle-ci, alginates, dextrane, chitosane, acide hyaluronique, sulfate de chondroïtine ou dans lequel ledit polymère synthétique est choisi parmi : polyesters, polyuréthanes, polyanhydrides, polyphosphazènes, fumarate de polypropylène, polyéthylène, polypropylène, poly(méthacrylate de méthyle) (PMMA), poly(diméthylsiloxane), poly(oxyde d'éthylène), poly(oxyde de propylène), poly(alcool de vinyle) (PVA).

6. Conjugué coumarine-polymère selon l'une quelconque des revendications 1 à 5 en combinaison avec un ou plusieurs médicaments et/ou un ou plusieurs types de cellule.

7. Conjugué coumarine-polymère selon l'une quelconque des revendications 1 à 6 dans lequel, lorsque ledit conjugué est irradié avec de la lumière IR à une longueur d'onde λ allant de 700 nm à 1000 nm, les composants de coumarine de différents motifs dudit conjugué coumarine-polymère subissent une réaction de cycloaddition en formant de ce fait un produit réticulé, de préférence dans lequel ladite longueur d'onde λ va de 800 nm à 1000 nm ; de 800 nm à 950 nm ; de 850 nm à 950 nm ou de 900 à 950 nm.

8. Mélange de conjugués coumarine-polymère selon l'une quelconque des revendications 1 à 7 comprenant de préférence au moins un conjugué coumarine-polymère naturel et au moins un conjugué coumarine-polymère synthétique.

9. Mélange de conjugués coumarine-polymère selon la revendication 8 en combinaison avec un ou plusieurs médicaments et/ou un ou plusieurs types de cellule.

10. Produit comprenant un ou plusieurs conjugués coumarine-polymère selon l'une quelconque des revendications 1 à 7 moyennant quoi les composants de coumarine de différents motifs desdits conjugués coumarine-polymère sont réticulés de préférence comprenant en outre un ou plusieurs médicaments et/ou un ou plusieurs types de cellule dans lequel ledit produit est de préférence sous la forme d'un hydrogel.

11. Dispositif médical comprenant le conjugué coumarine-polymère selon l'une quelconque des revendications 1 à 7, ou le mélange selon les revendications 8 ou 9, ou le produit selon la revendication 10, de préférence sous la forme d'un dispositif implantable.

12. Organoïde ou échafaudage comprenant le produit selon la revendication 10 et des cellules spécifiques au tissu ou des cellules souches.

13. Procédé destiné à la préparation d'un produit comprenant des conjugués coumarine-polymère réticulés ou un mélange de ceux-ci selon la revendication 10 comprenant l'étape consistant à
soumettre à une irradiation avec une source de rayonnement à une longueur d'onde de $700 \text{ nm} < \lambda < 1000 \text{ nm}$ un conjugué coumarine-polymère ou un mélange de celui-ci selon l'une quelconque des revendications 1 à 7, de préférence dans lequel ledit conjugué ou mélange de celui-ci est en combinaison avec un ou plusieurs médicaments, et/ou un ou plusieurs types de cellule avant irradiation.

14. Utilisation de conjugués coumarine-polymère selon l'une quelconque des revendications 1 à 7 pour la génération d'hydrogels réticulés destinés à une culture cellulaire in vitro, une bio-impression chargée de cellules et une bio-impression dans des matériaux 3D préexistants) ou en tant que systèmes de délivrance de médicament ou de cellule.

15. Procédé destiné à la dé-réticulation d'un conjugué coumarine-polymère ou d'un mélange de celui-ci dans un produit selon la revendication 10 comprenant l'étape consistant à
soumettre ledit conjugué ou mélange de celui-ci à une irradiation avec une source de rayonnement à une longueur d'onde de $700 \text{ nm} < \lambda < 1000 \text{ nm}$.

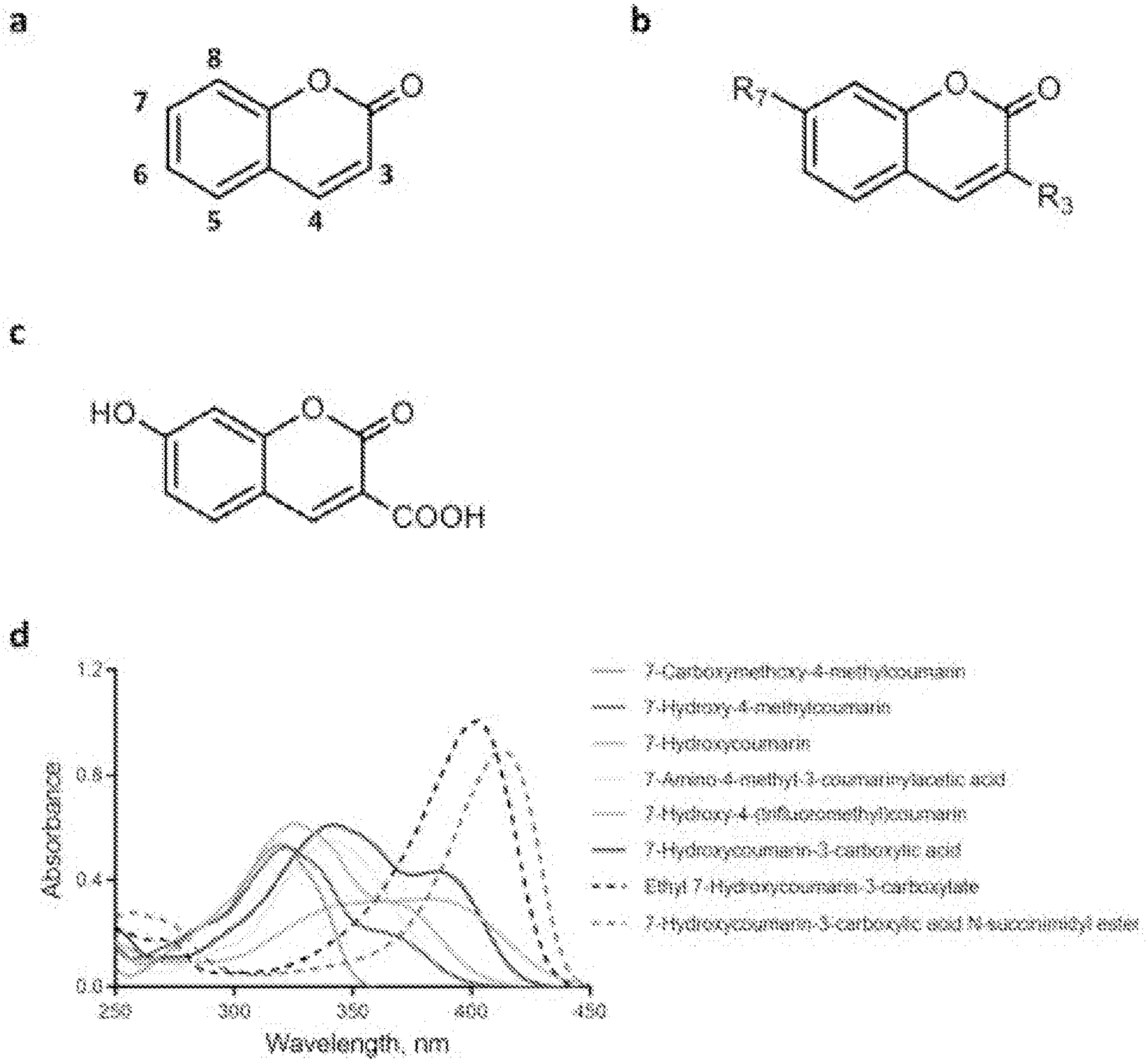
a
b
c
d
Absorbance
Wavelength, nm
1.2
0.8
0.4
0.0
250
300
350
400
450
7-Carboxymethoxy-4-methylcoumarin
7-Hydroxy-4-methylcoumarin
7-Hydroxycoumarin
7-Amino-4-methyl-3-coumarinylacetic acid
7-Hydroxy-4-(trifluoromethyl)coumarin
7-Hydroxycoumarin-3-carboxylic acid
Ethyl 7-Hydroxycoumarin-3-carboxylate
7-Hydroxycoumarin-3-carboxylic acid N-succinimidyl ester

Fig 1

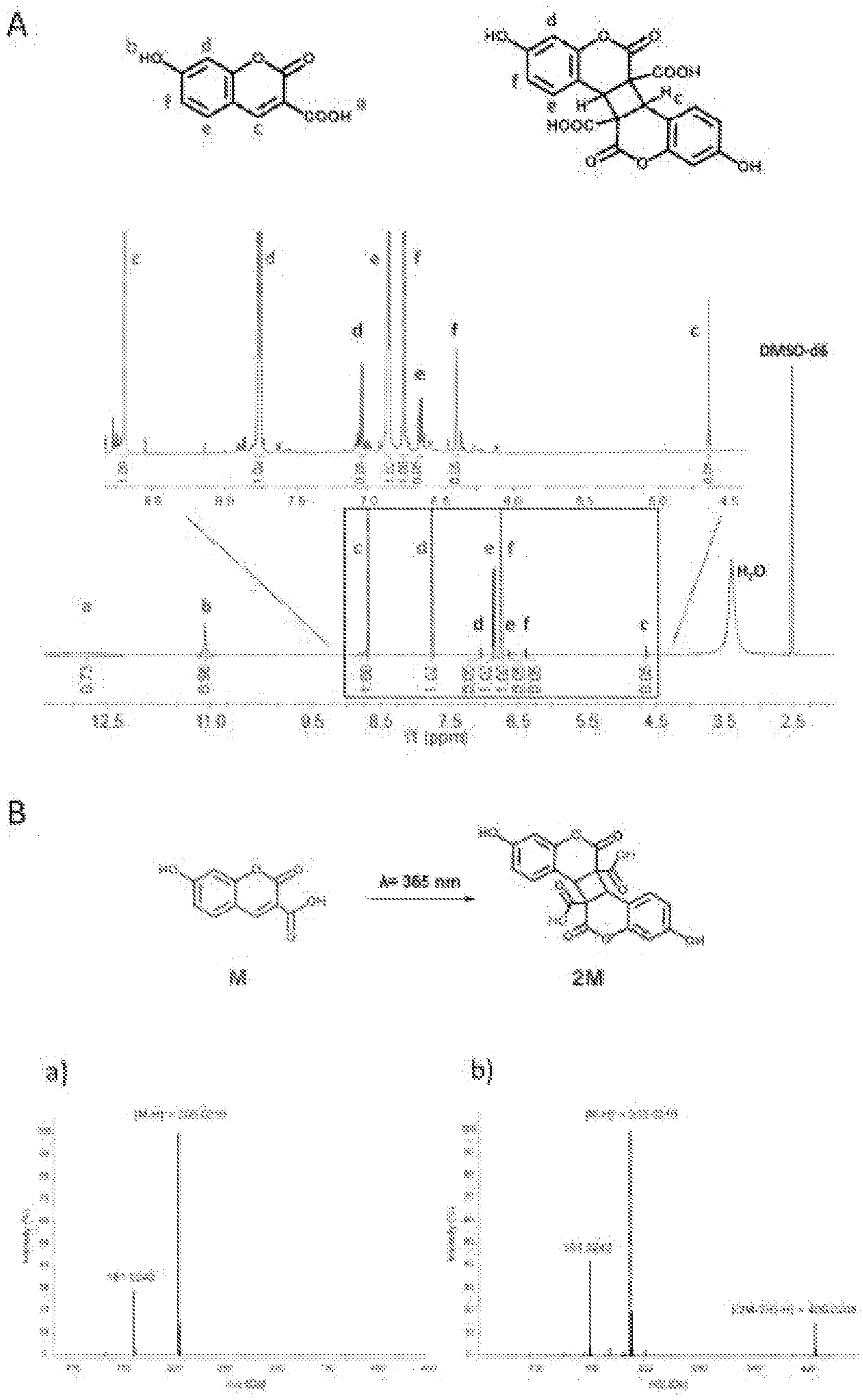


Fig. 2

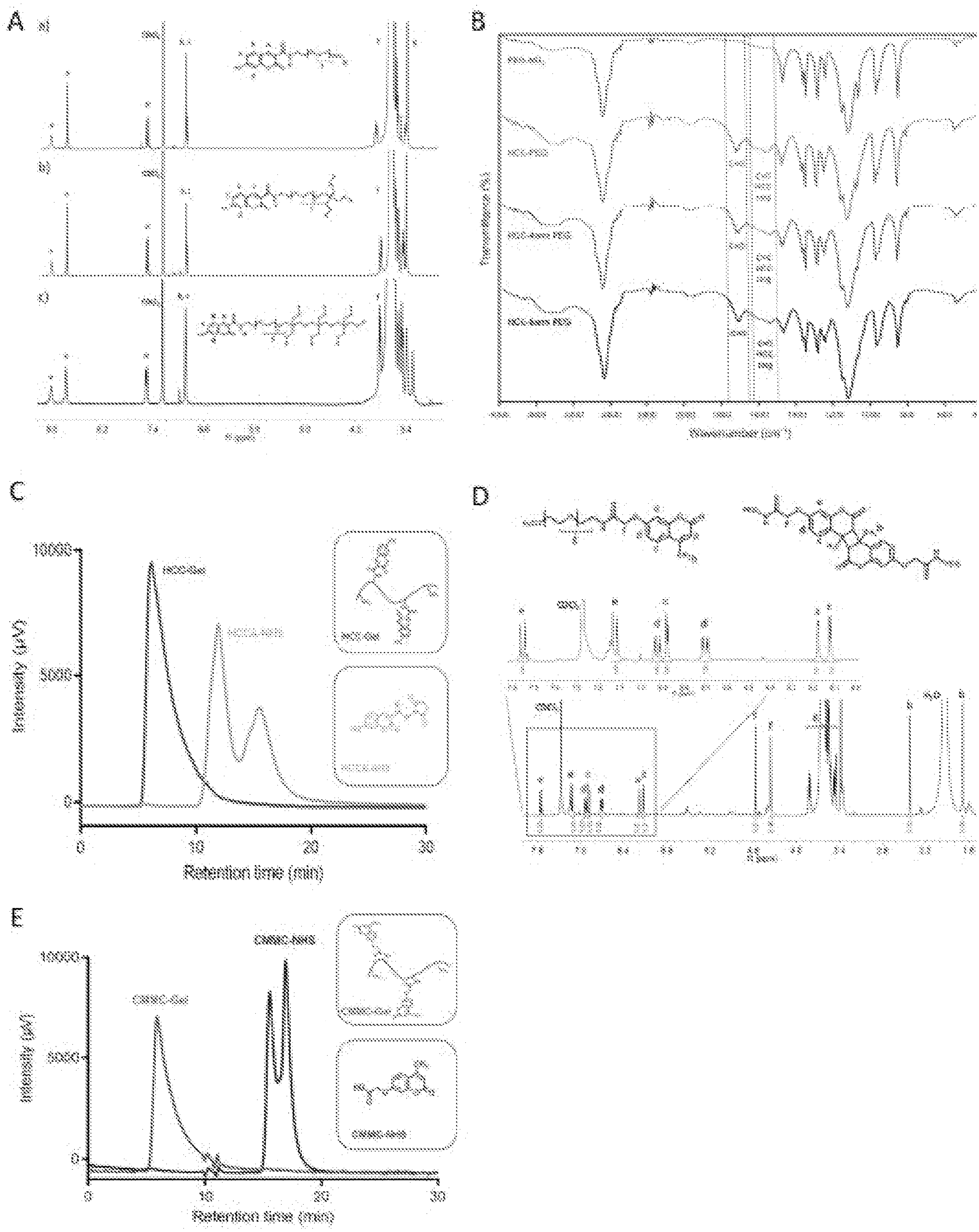
A
B
C
10000
5000
0
0
10
20
30
Intensity (μV)
Retention time (min)
D
E
10000
5000
0
0
10
20
30
Intensity (μV)
Retention time (min)

Fig. 3

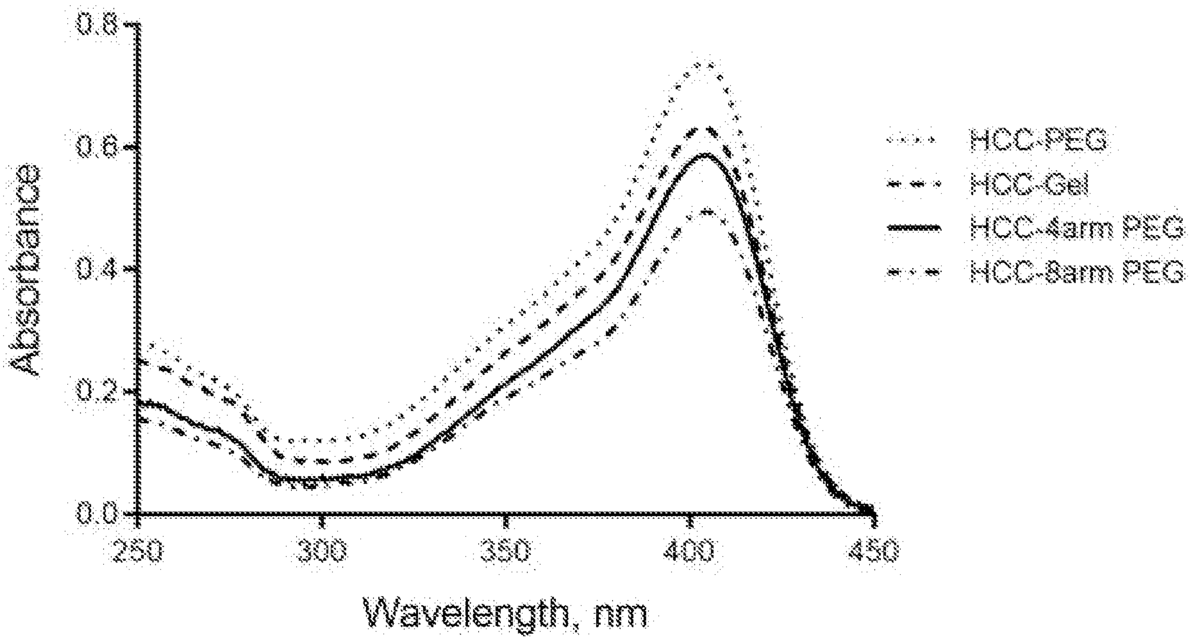
0.8
0.6
0.4
0.2
0.0
Absorbance
250
300
350
400
450
Wavelength, nm
HCC-PEG
HCC-Gel
HCC-4arm PEG
HCC-8arm PEG

Fig. 4

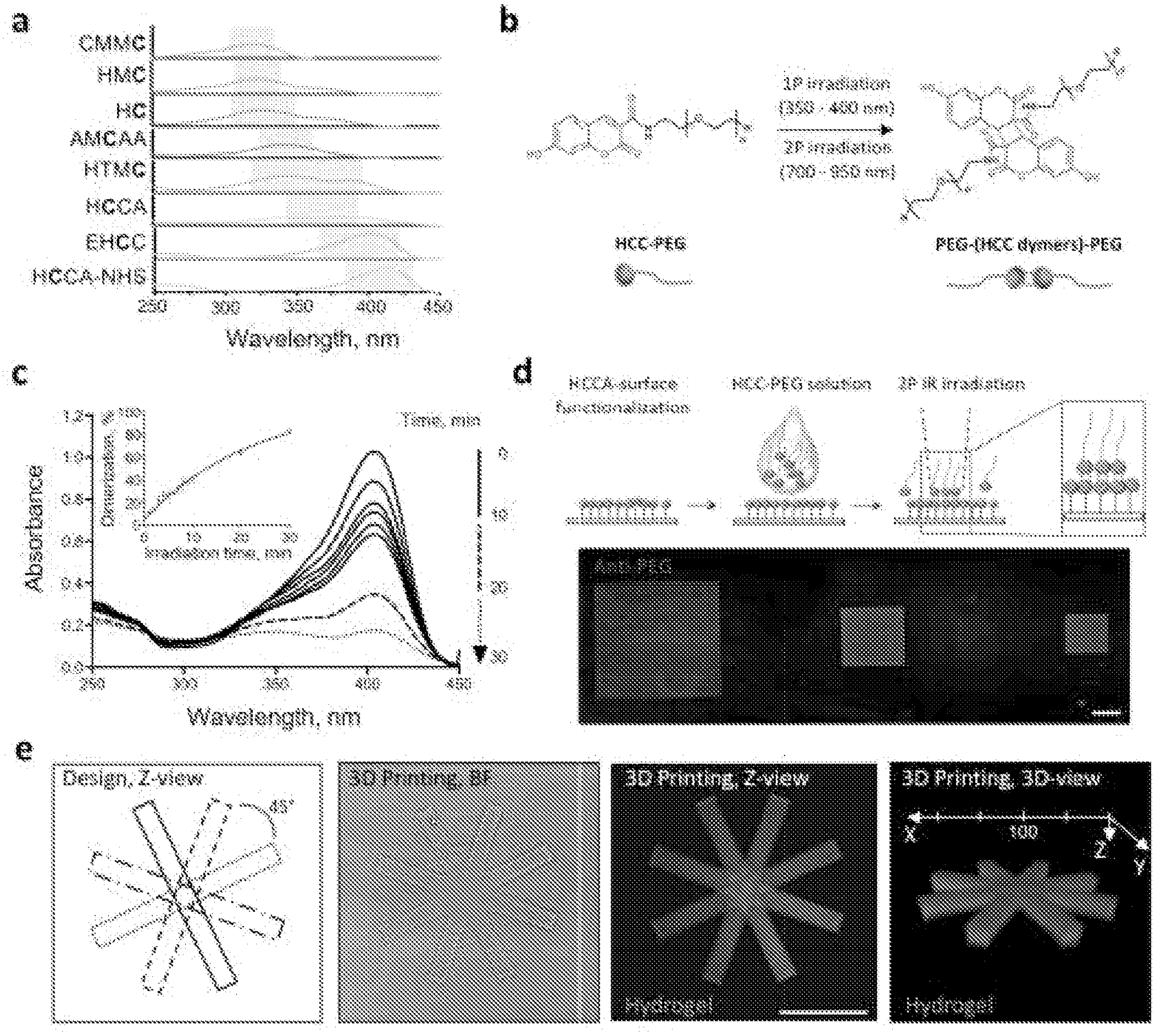
a
CMMC
HMC
HC
AMCAA
HTMC
HCCA
EHCC
HCCA-NHS
250
300
350
400
450
Wavelength, nm
b
1P irradiation
(350 - 400 nm)
2P irradiation
(700 - 950 nm)
HCC-PEG
PEG-(HCC dymers)-PEG
c
Absorbance
Time, min
Dimerization, %
Irradiation time, min
Wavelength, nm
d
HCCA-surface functionalization
HCC-PEG solution
2P IR irradiation
Anti-PEG
e
Design, Z-view
45°
3D Printing, BF
3D Printing, Z-view
Hydrogel
3D Printing, 3D-view
X
100
Z
Y
Hydrogel

Fig. 5

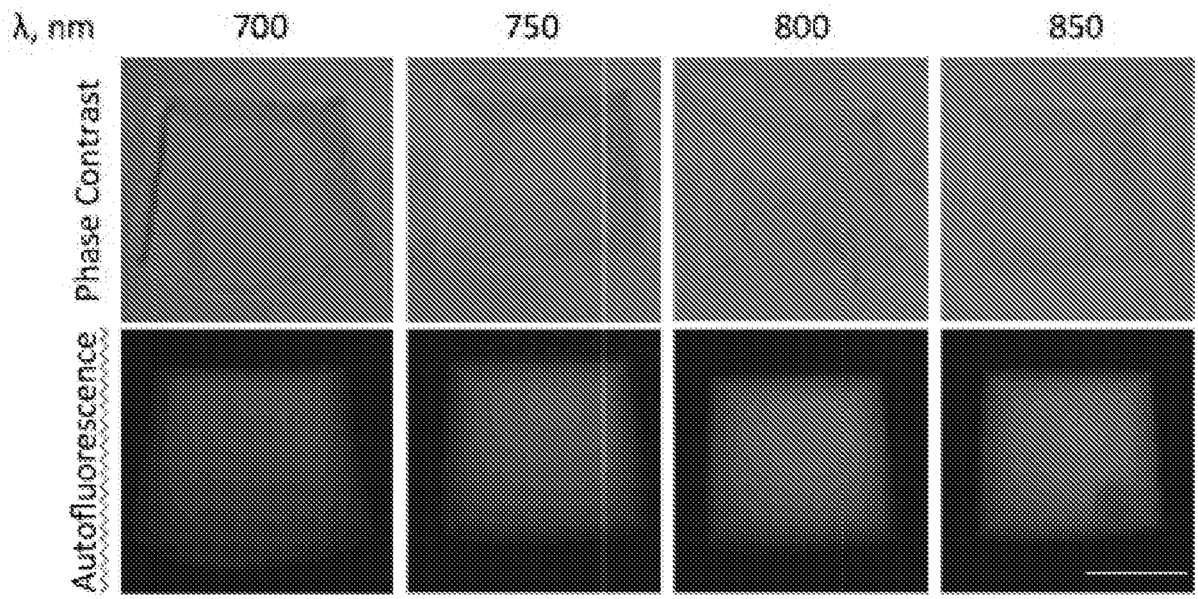


Fig. 6

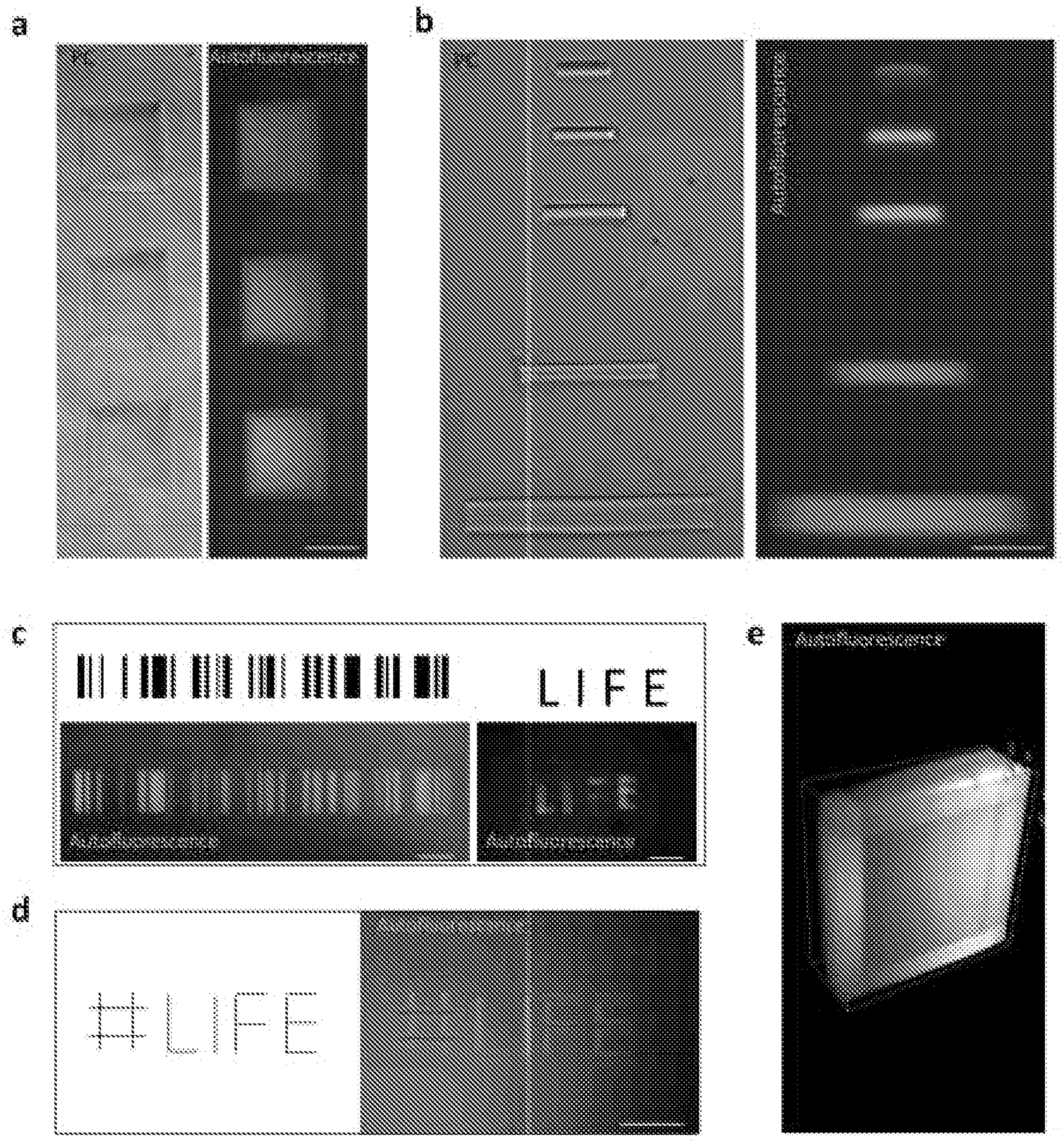
a
b
c
LIFE
Autofluorescence
Autofluorescence
d
#LIFE
e
Autofluorescence

Fig. 7

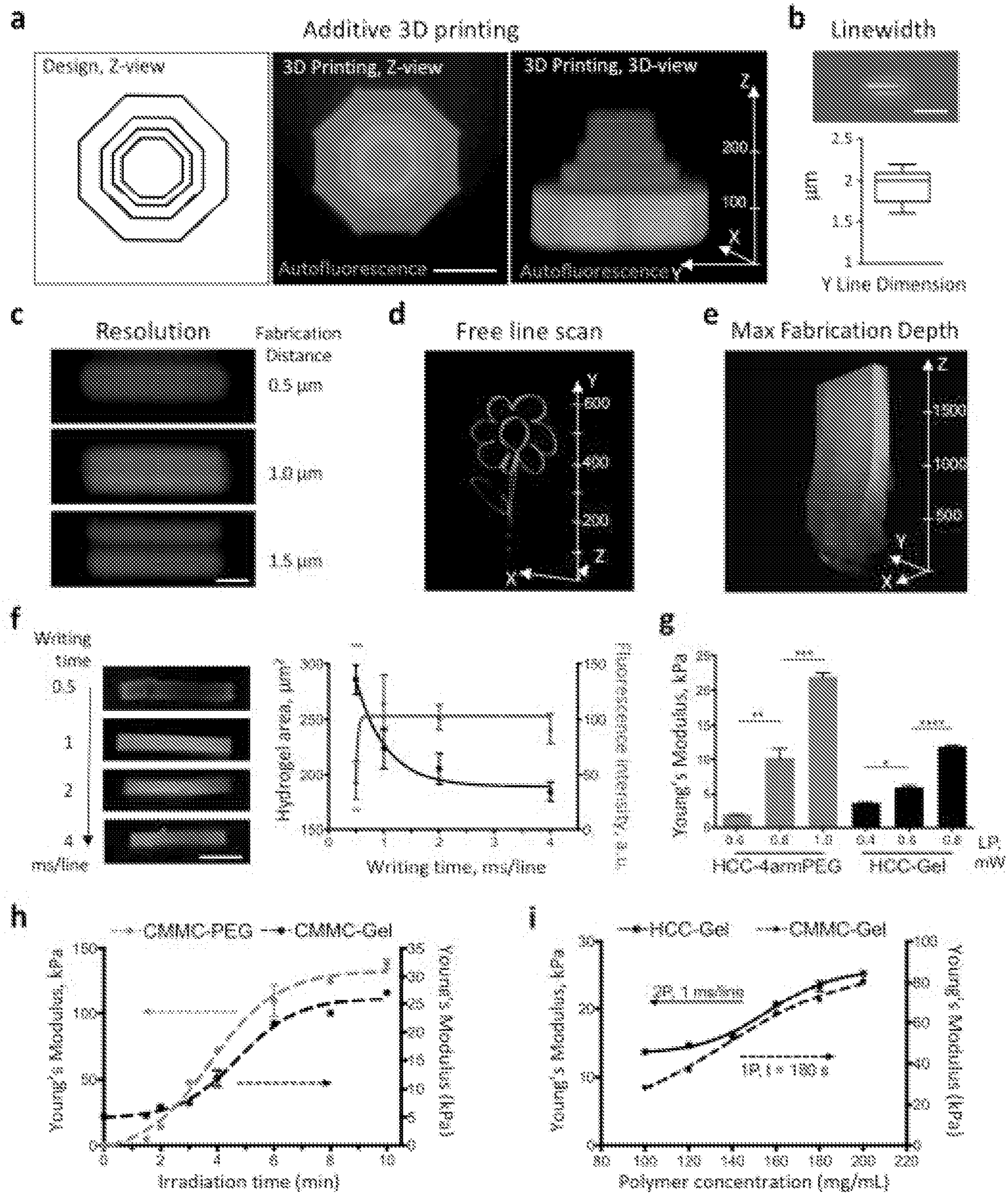
a
Additive 3D printing
Design, Z-view
3D Printing, Z-view
3D Printing, 3D-view
Autofluorescence
b
Linewidth
µm
Y Line Dimension
c
Resolution
Fabrication Distance
0.5 µm
1.0 µm
1.5 µm
d
Free line scan
e
Max Fabrication Depth
f
Writing time
ms/line
Hydrogel area, µm²
Fluorescence intensity, a.u.
Writing time, ms/line
g
Young's Modulus, kPa
HCC-4armPEG
HCC-Gel
LP, mW
h
CMMC-PEG
CMMC-Gel
Young's Modulus, kPa
Young's Modulus (kPa)
Irradiation time (min)
i
HCC-Gel
CMMC-Gel
2P, 1 ms/line
1P, t = 180 s
Young's Modulus, kPa
Young's Modulus (kPa)
Polymer concentration (mg/mL)

Fig. 8

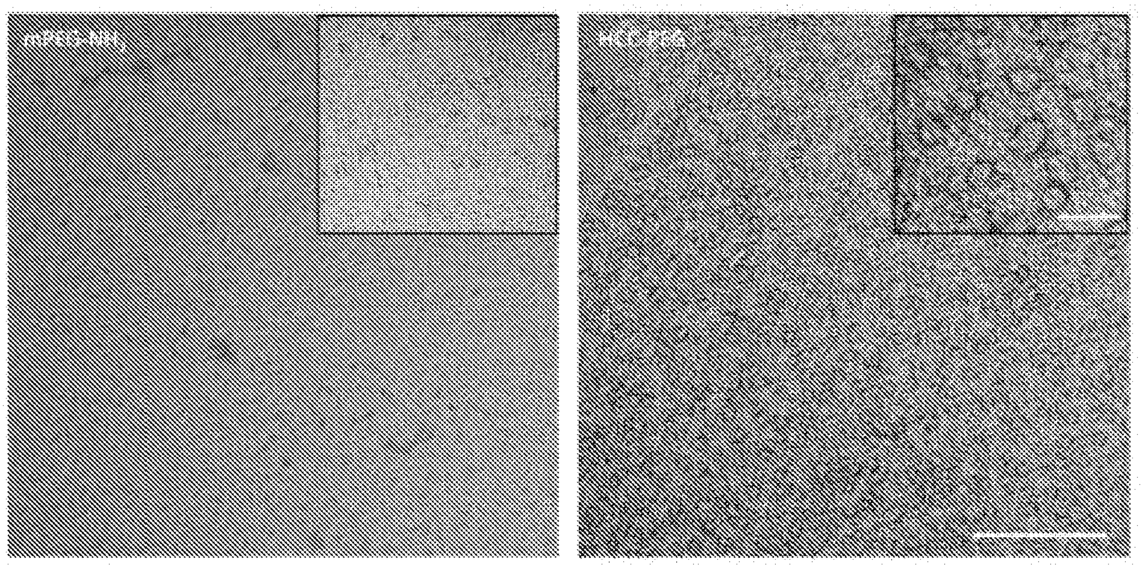
mPEG-NH2

Fig. 9

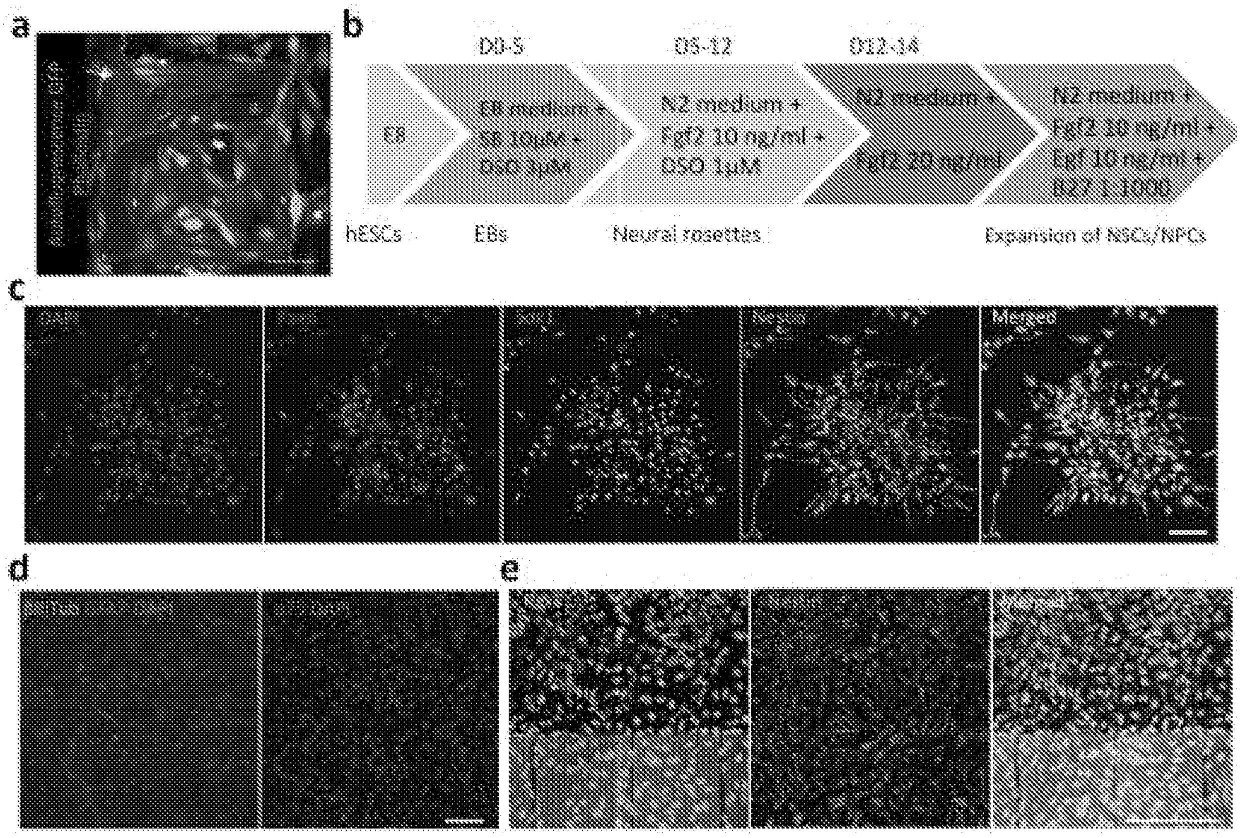
a
b
D0-5
D5-12
D12-14
E8
EB medium +
SB 10μM +
DSO 1μM
N2 medium +
Fgf2 10 ng/ml +
DSO 1μM
N2 medium +
Fgf2 20 ng/ml
N2 medium +
Fgf2 10 ng/ml +
Egf 10 ng/ml +
B27 1:1000
hESCs
EBs
Neural rosettes
Expansion of NSCs/NPCs
c
Merged
d
e

Fig. 10

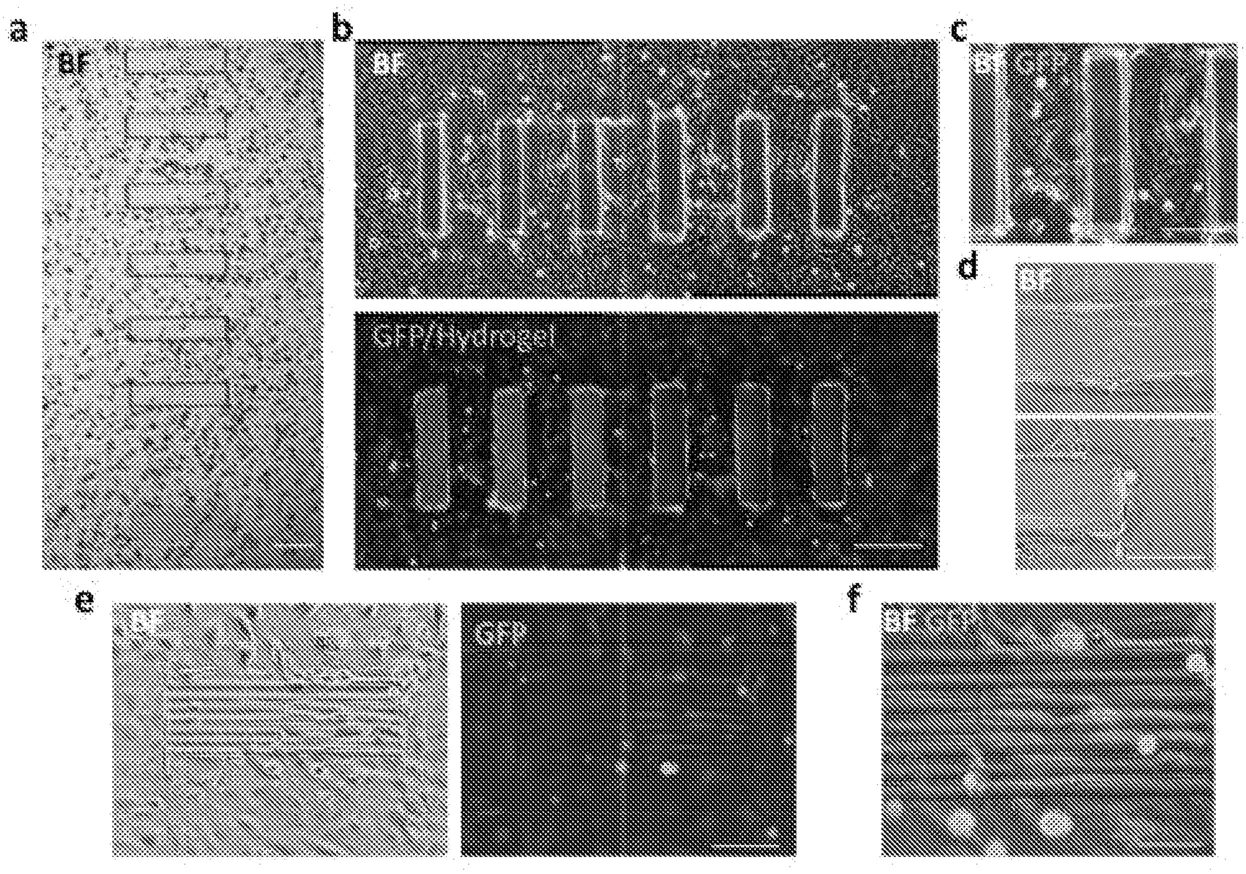


Fig. 11

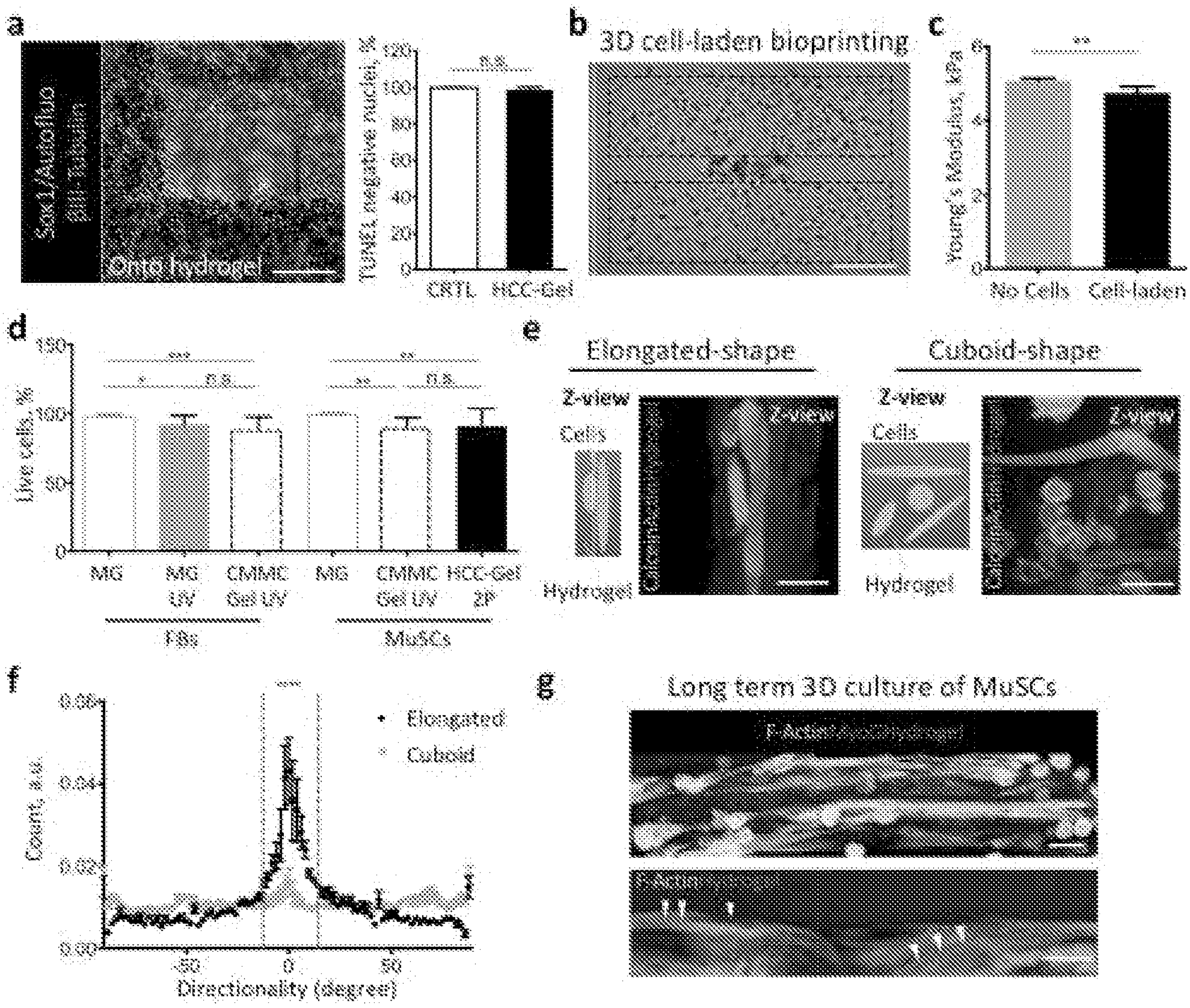
a
Sox1/Autofluo
βIII-Tubulin
Onto hydrogel
TUNEL negative nuclei, %
n.s.
CRTL
HCC-Gel
b
3D cell-laden bioprinting
c
Young's Modulus, kPa
No Cells
Cell-laden
d
Live cells, %
n.s.
MG
MG UV
CMMC Gel UV
HCC-Gel 2P
FBs
MuSCs
e
Elongated-shape
Cuboid-shape
Z-view
Cells
Hydrogel
f
Count, a.u.
Elongated
Cuboid
Directionality (degree)
g
Long term 3D culture of MuSCs

Fig 12

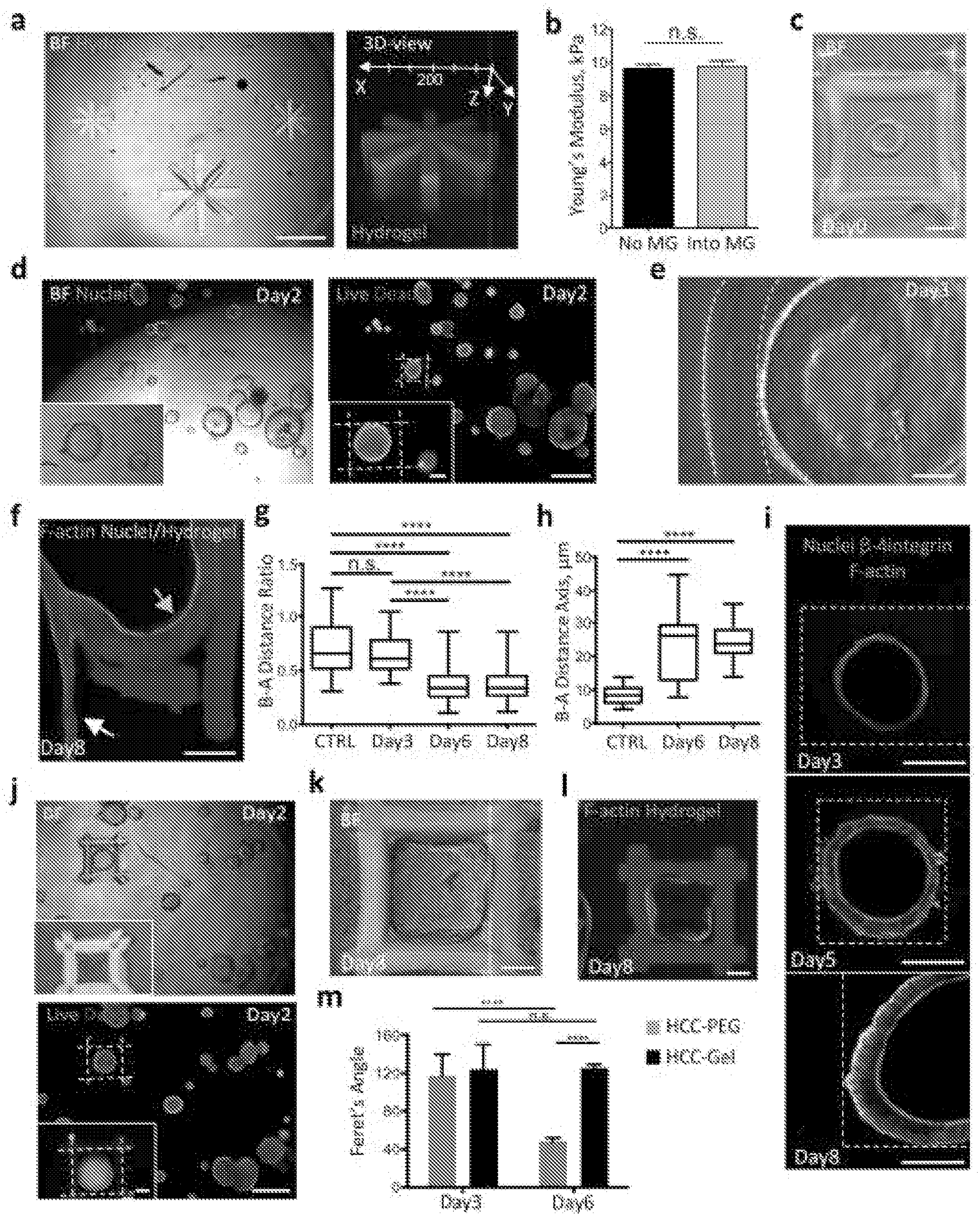
a
BF
3D view
200
X
Z
Y
Hydrogel
b
n.s.
Young's Modulus, kPa
No MG
Into MG
c
BF
Day0
d
BF Nuclei
Day2
Live Dead
Day2
e
Day3
f
F-actin Nuclei/Hydrogel
Day8
g
****
****
n.s.
****
****
B-A Distance Ratio
CTRL
Day3
Day6
Day8
h
****
****
B-A Distance Axis, µm
CTRL
Day6
Day8
i
Nuclei β-4integrin
F-actin
Day3
Day5
Day8
j
Day2
Live D
Day2
k
BF
l
F-actin Hydrogel
Day8
m
n.s.
****
HCC-PEG
HCC-Gel
Feret's Angle
Day3
Day6

Fig. 13

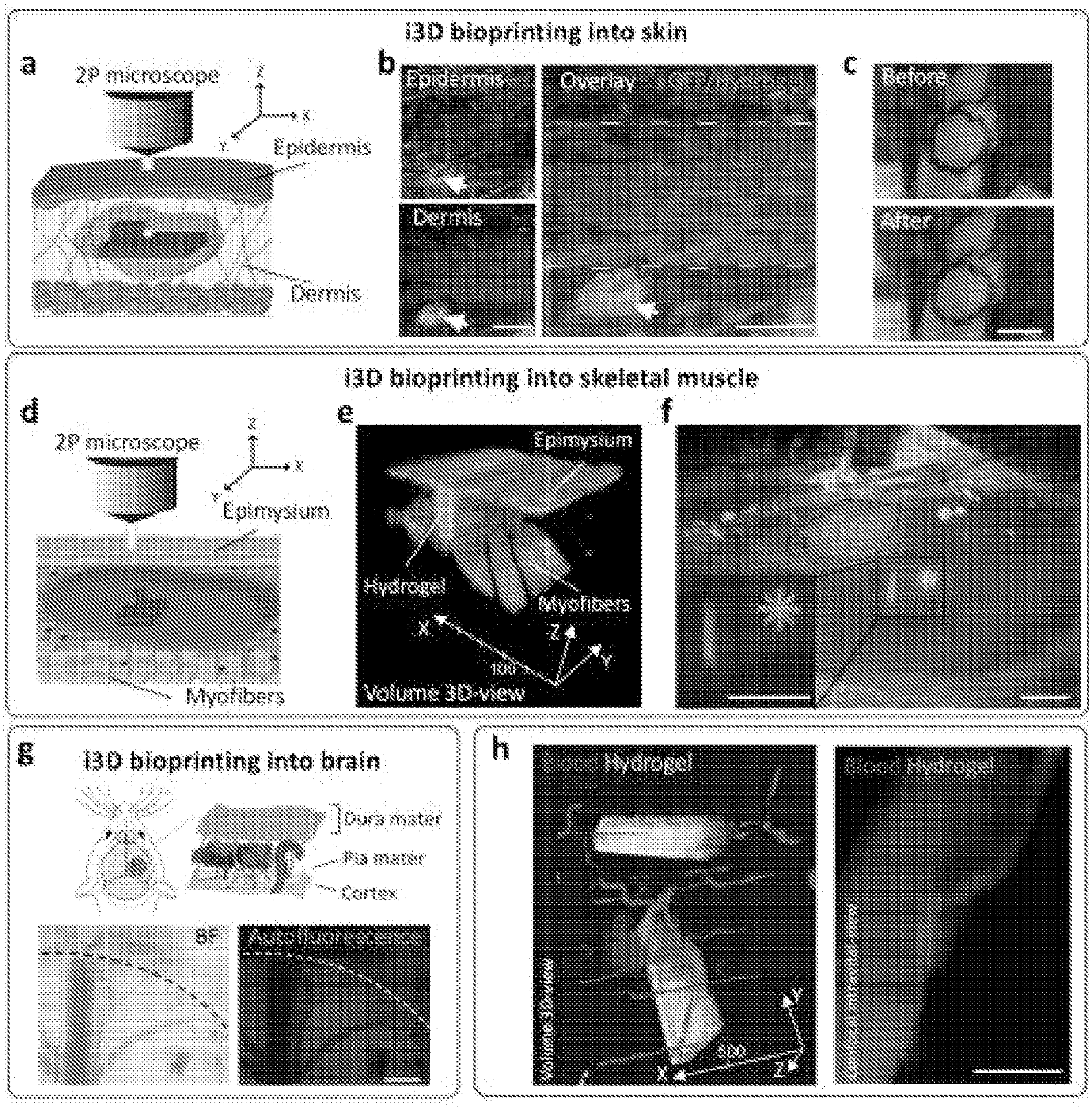
i3D bioprinting into skin
a
2P microscope
Epidermis
Dermis
b
Epidermis
Dermis
Overlay
c
Before
After
i3D bioprinting into skeletal muscle
d
2P microscope
Epimysium
Myofibers
e
Epimysium
Hydrogel
Myofibers
100
Volume 3D-view
f
g
i3D bioprinting into brain
Dura mater
Pia mater
Cortex
BF
Autofluorescence
h
Hydrogel
500

Fig. 14

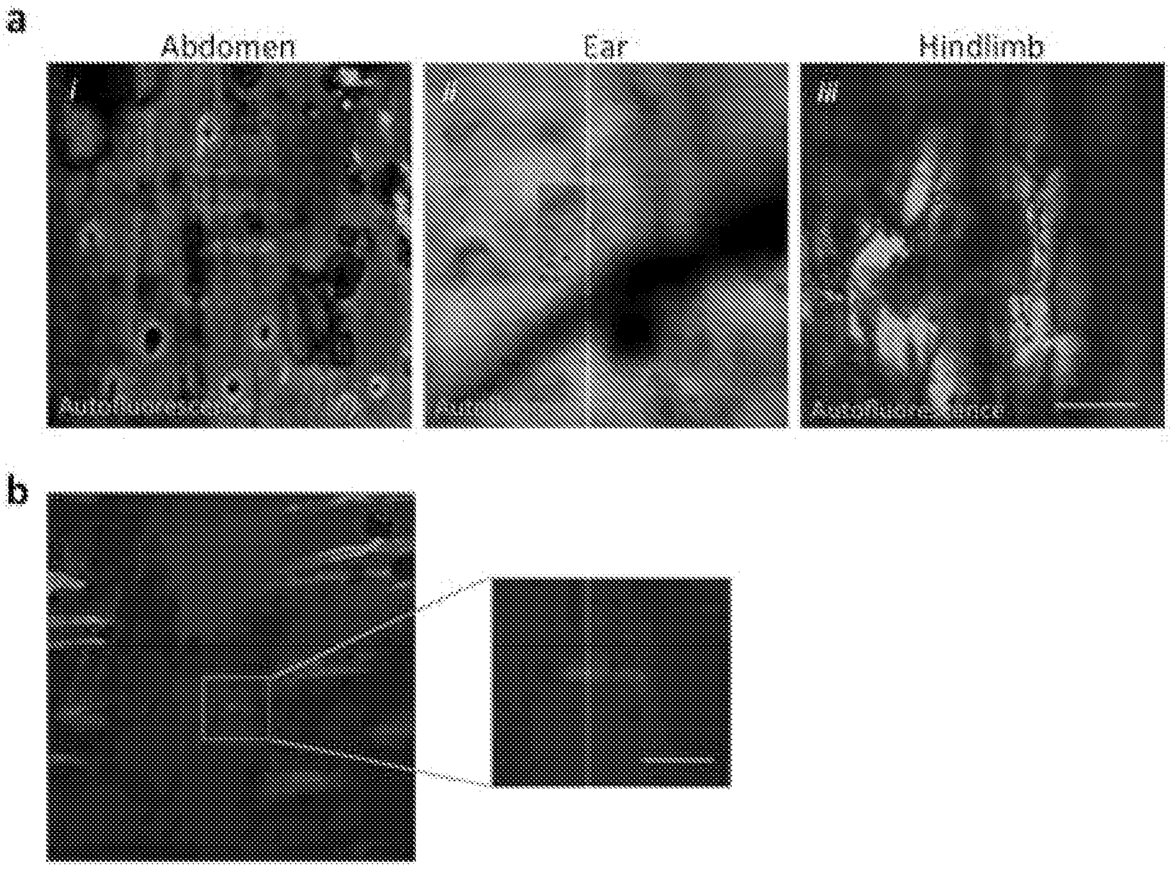


Fig. 15

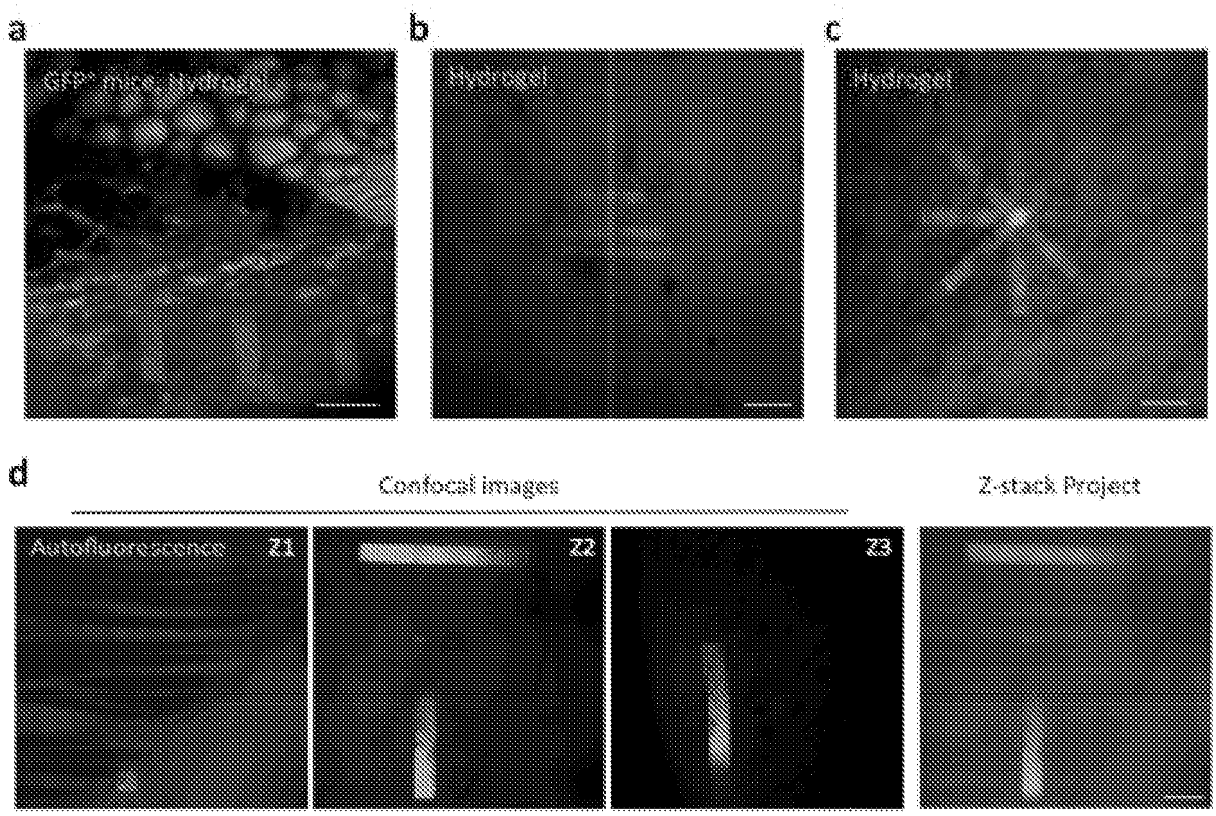
a
Hydrogel
b
Hydrogel
c
d
Confocal images
Z-stack Project
Autofluorescence
Z1
Z2
Z3

Fig. 16

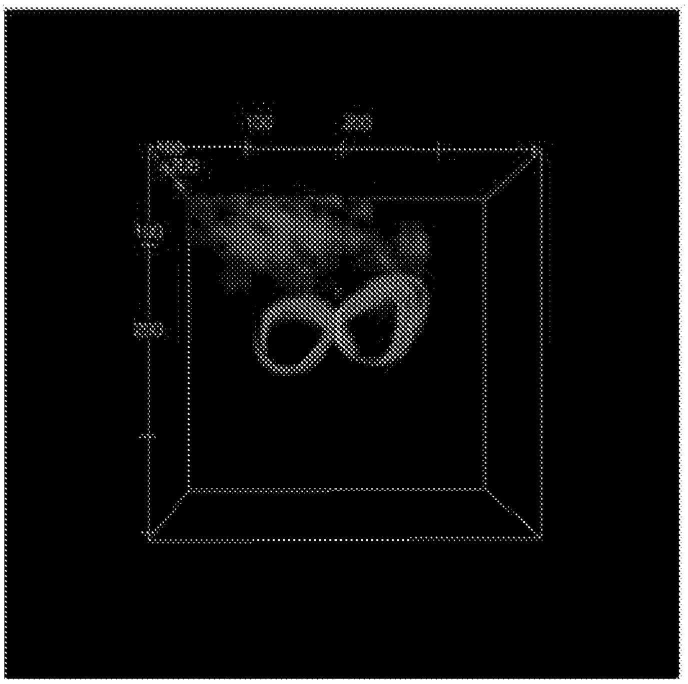

Fig 17

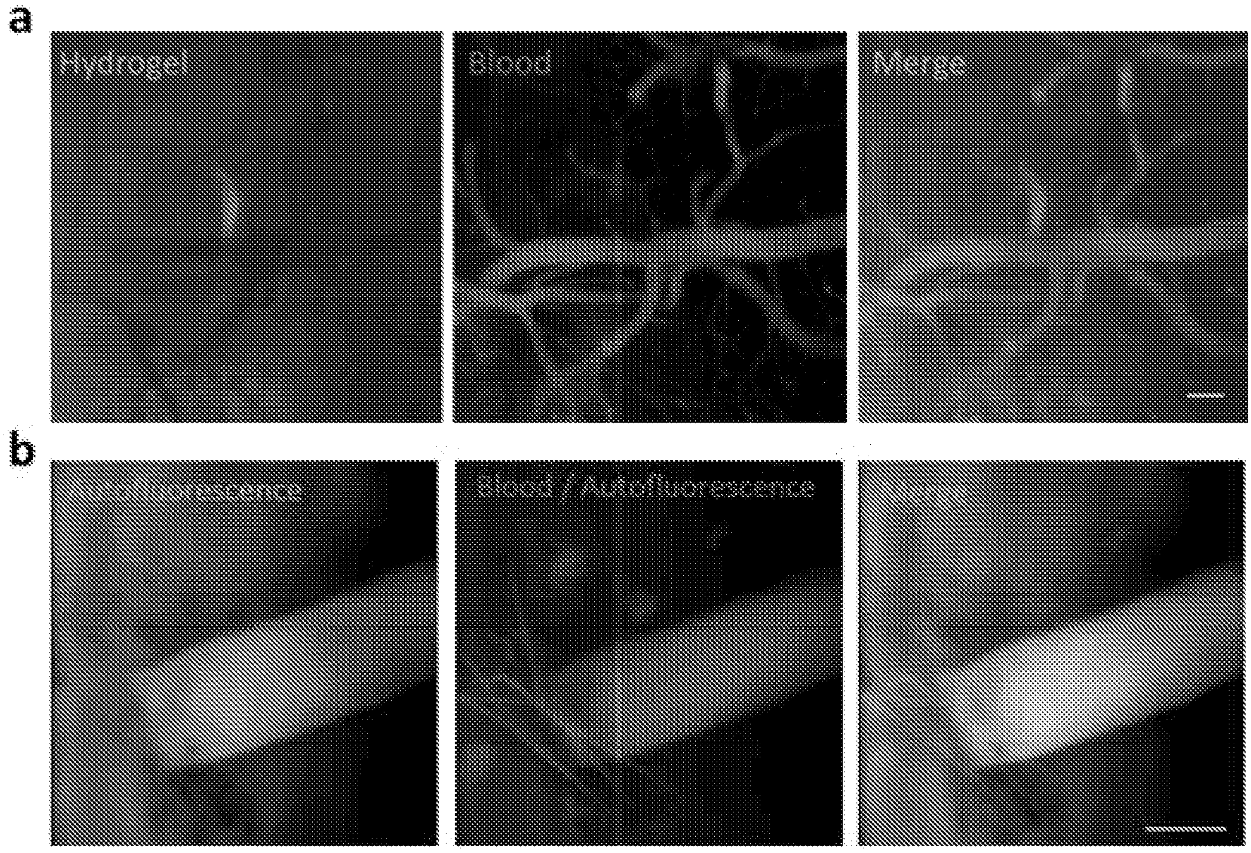


Fig.18

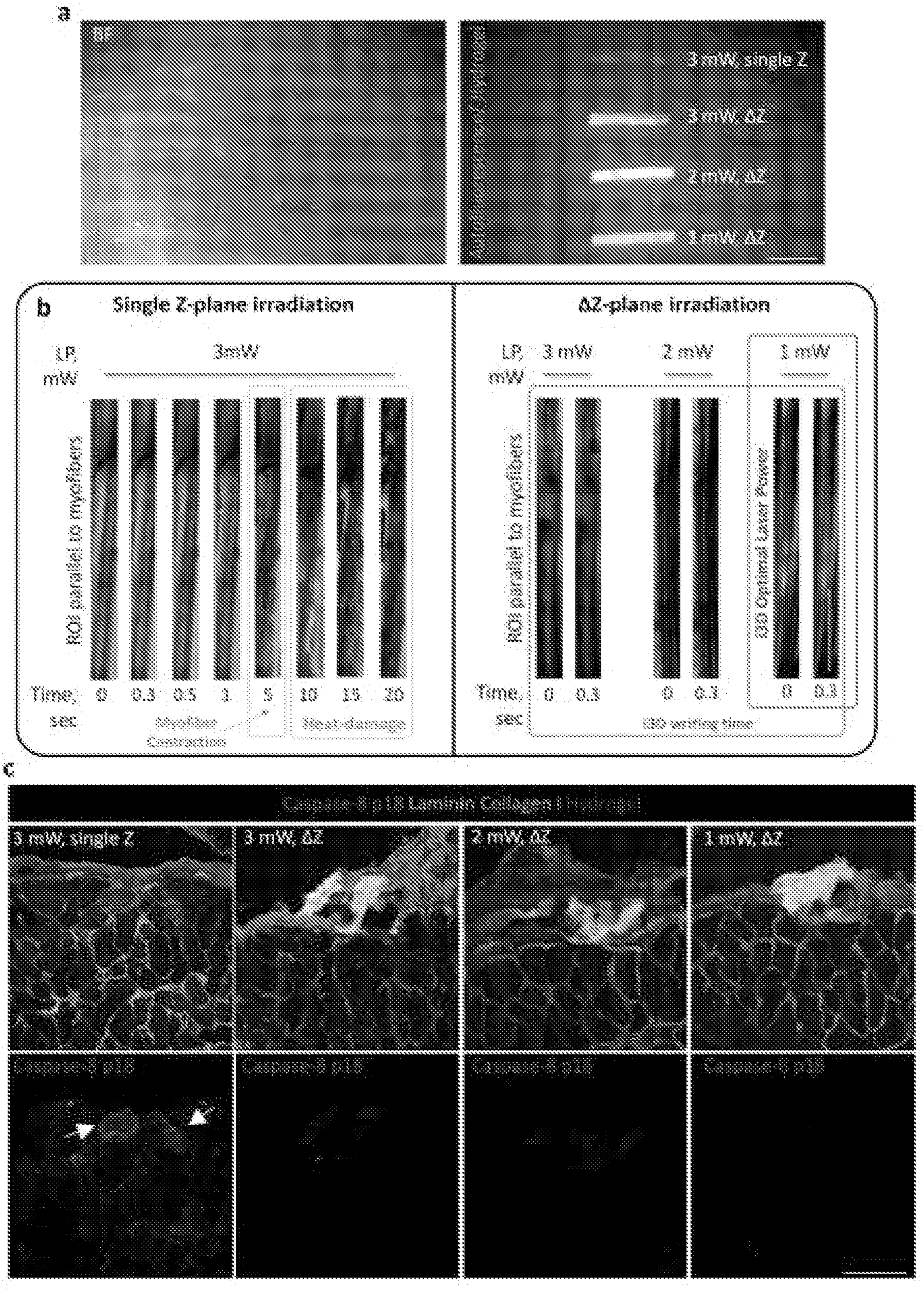
a
BF
3 mW, single Z
3 mW, ΔZ
2 mW, ΔZ
1 mW, ΔZ
b
Single Z-plane irradiation
ΔZ-plane irradiation
LP, mW
3mW
3 mW
2 mW
1 mW
ROI parallel to myofibers
IBD Optimal Laser Power
Time, sec
0
0.3
0.5
1
5
10
15
20
Myofiber Contraction
Heat-damage
IBD writing time
c
3 mW, single Z
3 mW, ΔZ
2 mW, ΔZ
1 mW, ΔZ
Caspase-8 p18

Fig. 19

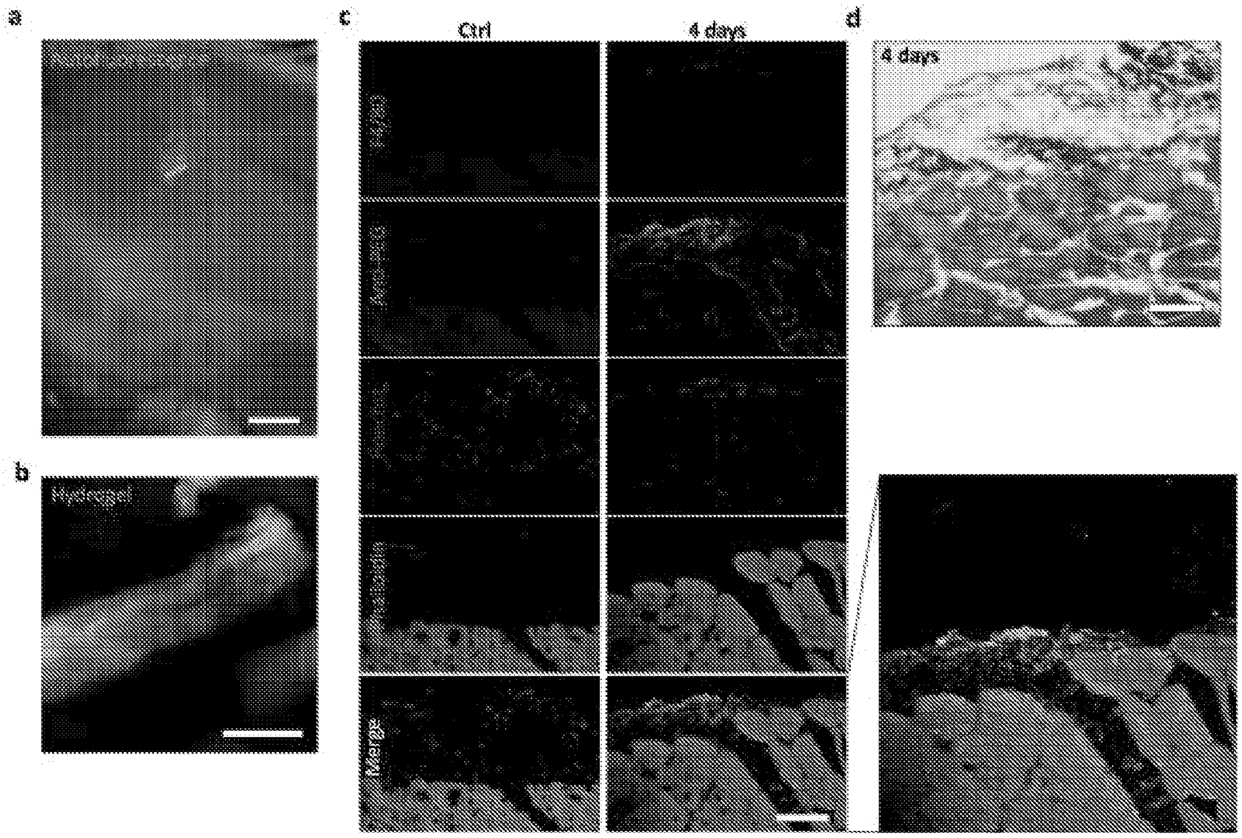


Fig. 20

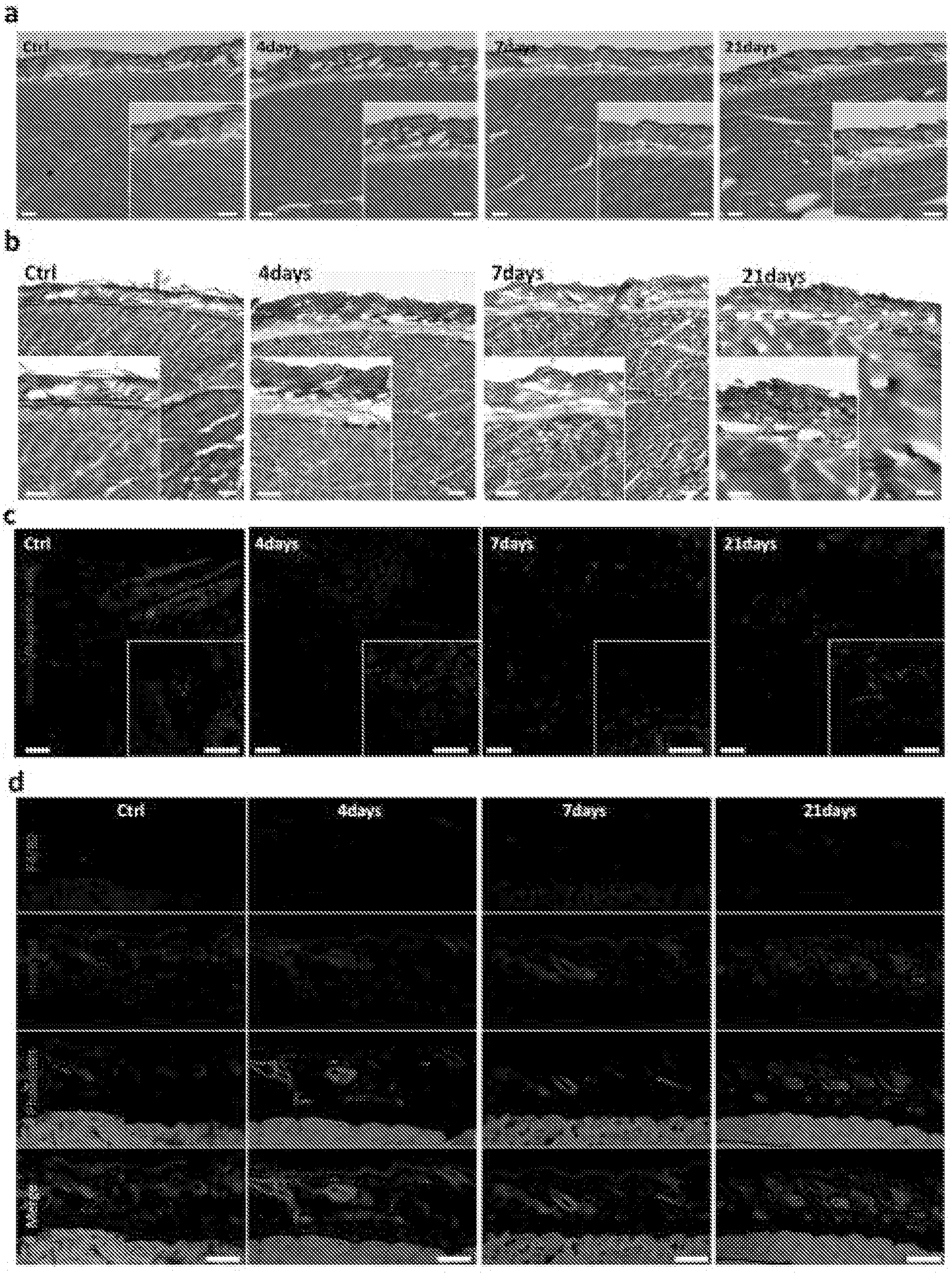
a
Ctrl
4days
7days
21days
b
Ctrl
4days
7days
21days
c
Ctrl
4days
7days
21days
d
Ctrl
4days
7days
21days

Fig 21

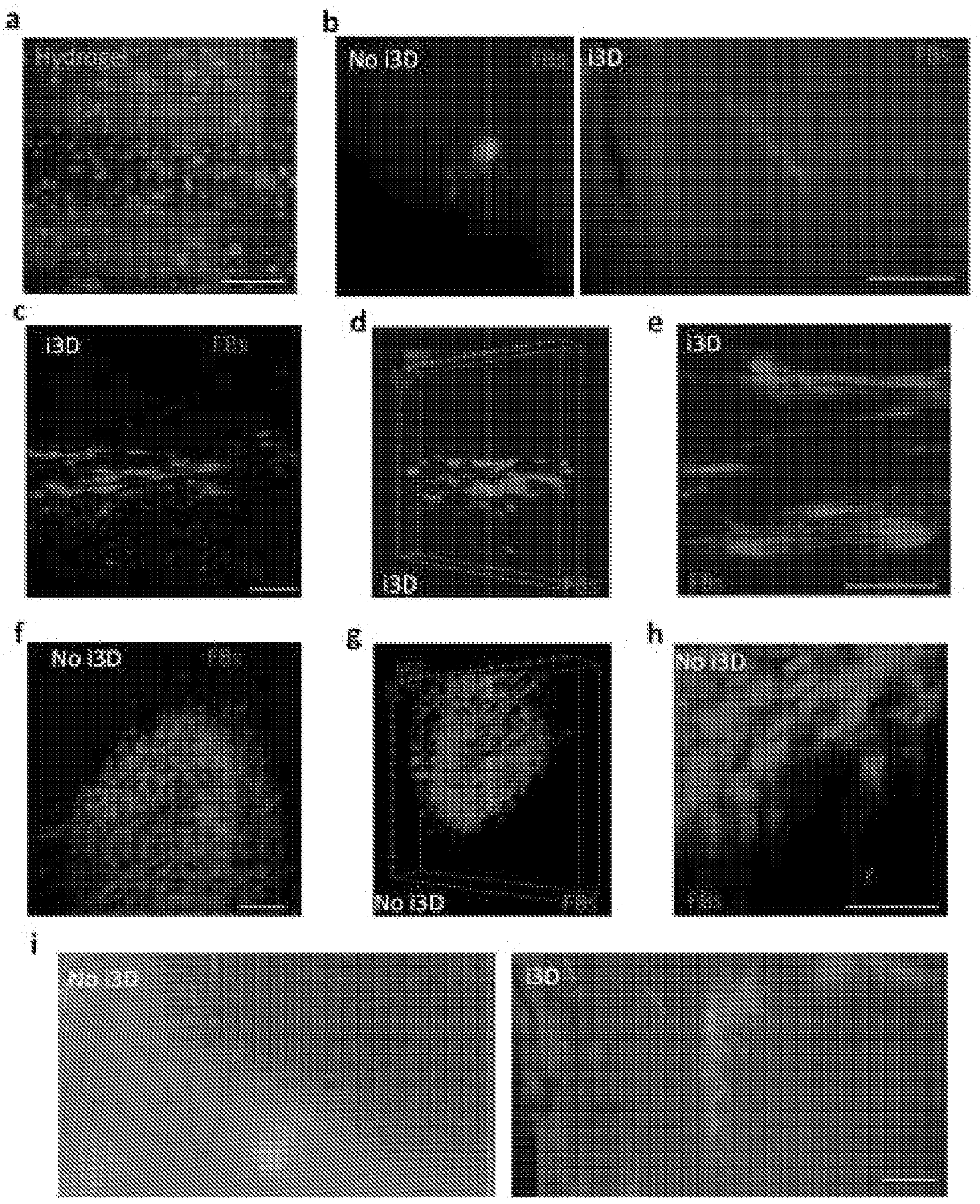
a
Hydrogel
b
No i3D
FBs
i3D
FBs
c
i3D
FBs
d
i3D
FBs
e
i3D
FBs
f
No i3D
FBs
g
No i3D
FBs
h
No i3D
FBs
i
No i3D
i3D

Fig 22

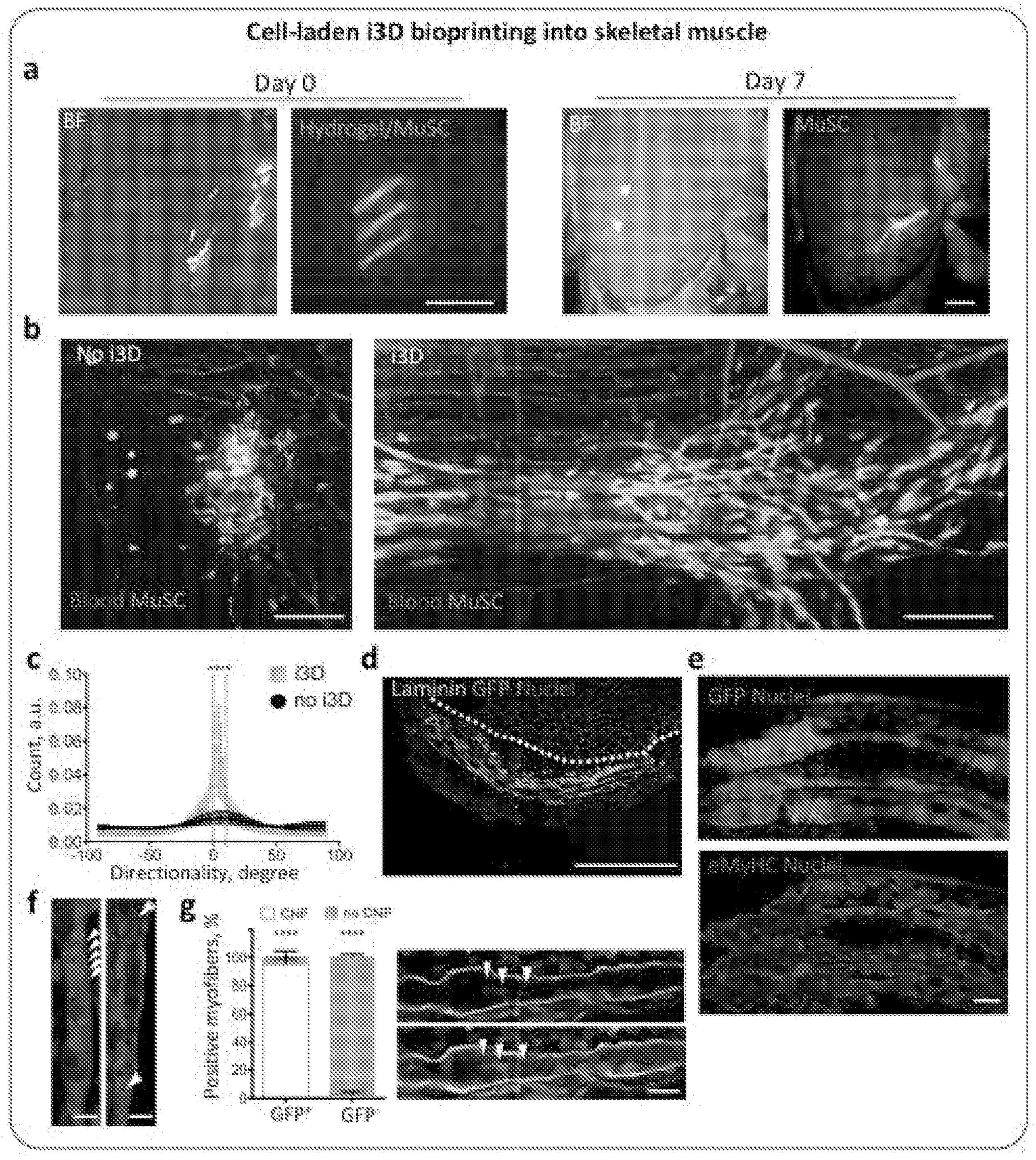
Cell-laden i3D bioprinting into skeletal muscle
a
Day 0
Day 7
BF
Hydrogel/MuSC
BF
MuSC
b
No i3D
Blood MuSC
i3D
Blood MuSC
c
i3D
no i3D
Count, a.u.
Directionality, degree
d
Laminin GFP Nuclei
e
GFP Nuclei
eMyHC Nuclei
f
g
CNF
no CNF
Positive myofibers, %
GFP+
GFP-

Fig 23

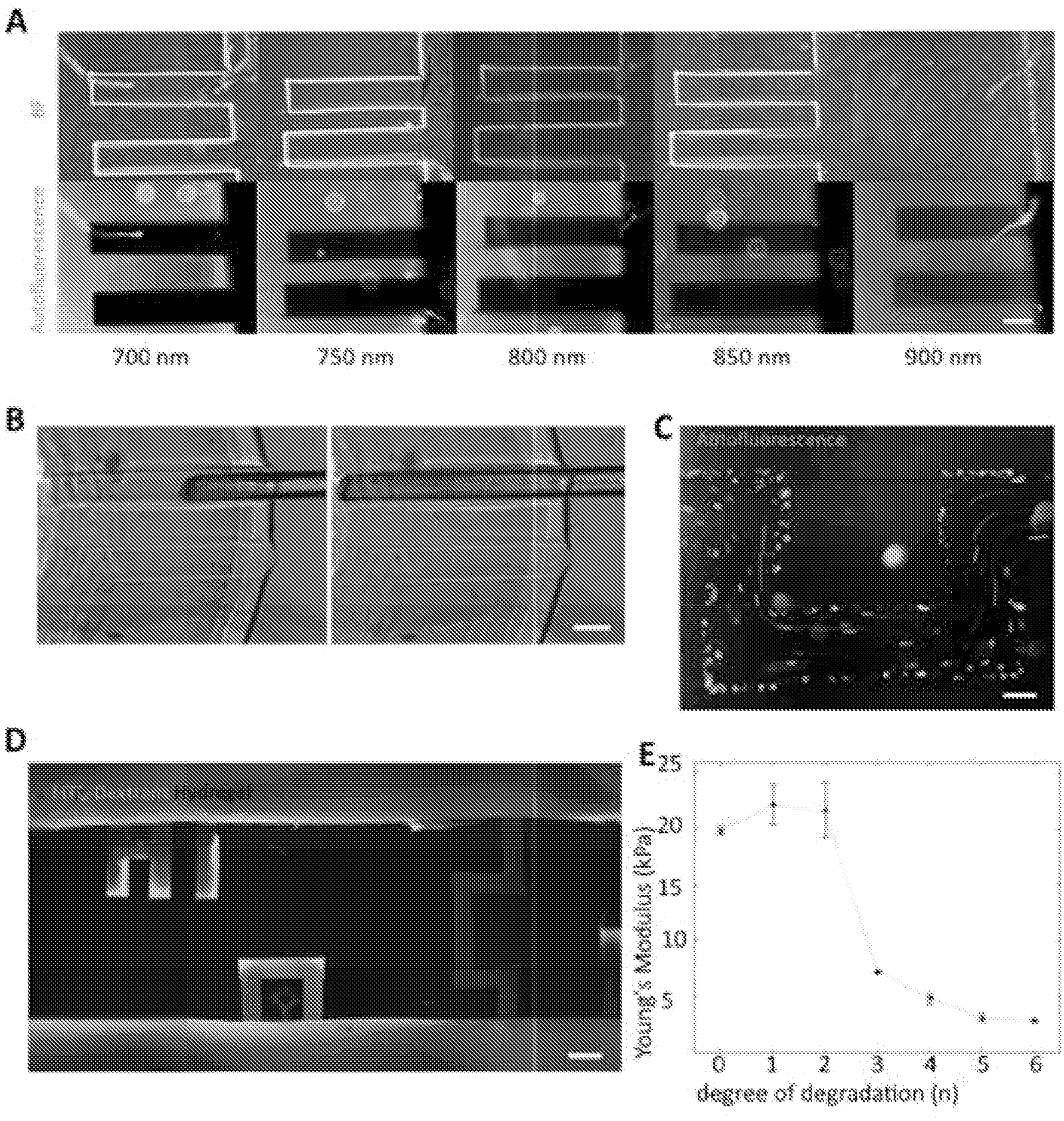
A
700 nm
750 nm
800 nm
850 nm
900 nm
B
C
Autofluorescence
D
Hydrogel
E
25
20
15
10
5
Young's Modulus (kPa)
0
1
2
3
4
5
6
degree of degradation (n)

Fig 24

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **L. MORONI et al.** Biofabrication strategies for 3D in vitro models and regenerative medicine.. *Nat. Rev. Mater.*, 2018, vol. 3, 21-37
- **S. V. MURPHY** ; **A. ATALA**. 3D bioprinting of tissues and organs.. *Nat. Biotechnol.*, 2014, vol. 32, 773-785
- **C. S. ONG et al.** 3D bioprinting using stem cells.. *Pediatr. Res.*, 2017, 1-23
- **N. HONG** ; **G.-H. YANG** ; **J. LEE** ; **G. KIM**. 3D bioprinting and its in vivo applications.. *J. Biomed. Mater. Res. Part B Appl. Biomater.*, 2017, 444-459
- **M. WANG et al.** The trend towards in vivo bioprinting.. *Int. J. Bioprinting*, 2015
- **A. SKARDAL et al.** Bioprinted Amniotic Fluid-Derived Stem Cells Accelerate Healing of Large Skin Wounds.. *Stem Cells Transl. Med.*, 2012, vol. 1, 792-802
- **K. W. BINDER et al.** In situ bioprinting of the skin for burns.. *J. Am. Coll. Surg.*, 2010, vol. 211, S76
- **V. KERIQUEL et al.** In situ printing of mesenchymal stromal cells, by laser-assisted bioprinting, for in vivo bone regeneration applications.. *Sci. Rep.*, 2017, vol. 7, 1-10
- **C. DI BELLA et al.** In situ handheld three-dimensional bioprinting for cartilage regeneration.. *J. Tissue Eng. Regen. Med.*, 2017, 611-621
- **ASHAMMAKHI et al.** *BIOMED MICRODEV.*, 2019, vol. 21 (2), 1-6
- **WANG, X et al.** Advanced Functional Biomaterials for Stem Cell Delivery in Regenerative Engineering and Medicine.. *Advanced Functional Materials*, 2019, vol. 29, 1-31
- **ZHANG, Z. et al.** 3D bioprinting of soft materials-based regenerative vascular structures and tissues.. *Compos. Part B Eng.*, 2017, vol. 123, 279-291
- **CHIN, S. Y. et al.** Additive manufacturing of hydrogel-based materials for next-generation implantable medical devices.. *Sci. Robot.*, 2017, vol. 2, eaah6451
- **MURPHY et al.** Evaluation of hydrogels for bio-printing applications.. *J. Biomed. Mater. Res. - Part A*, 2013
- **MALAR A. AZAGARSAMY et al.** *Coumarin-based Photodegradable Hydrogel: Design, Synthesis, Gelation, and Degradation Kinetics*
- **K. KÖNIG**. Multiphoton microscopy in life sciences.. *J. Microsc.*, 2000, vol. 200, 83-104
- **J. TORGERSEN et al.** Hydrogels for two-photon polymerization: A toolbox for mimicking the extracellular matrix.. *Adv. Funct. Mater.*, 2013, vol. 23, 4542-4554
- **C. G. WILLIAMS** ; **A. N. MALIK** ; **T. K. KIM** ; **P. N. MANSON** ; **J. H. ELISSEEFF**. Variable cytocompatibility of six cell lines with photoinitiators used for polymerizing hydrogels and cell encapsulation.. *Biomaterials*, 2005, vol. 26, 1211-1218